(19) Europäisches Patentamt
European Patent Office
Office européen des brevets

(11) EP 1 670 820 B1

(12) **EUROPEAN PATENT SPECIFICATION**

(45) Date of publication and mention of the grant of the patent:
**10.12.2008 Bulletin 2008/50**

(21) Application number: **04784633.2**

(22) Date of filing: **21.09.2004**

(51) Int Cl.:
***C07K 14/44*** (2006.01) ***C12N 15/30*** (2006.01)
***A61K 39/018*** (2006.01) ***G01N 33/569*** (2006.01)
***C07K 16/20*** (2006.01) ***C12N 15/10*** (2006.01)

(86) International application number:
**PCT/US2004/030831**

(87) International publication number:
**WO 2005/030802 (07.04.2005 Gazette 2005/14)**

# (54) ANTIGENS FOR AN EAST COAST FEVER VACCINE

ANTIGENE FÜR EINEN EAST COAST FEVER-IMPFSTOFF

ANTIGENES POUR VACCIN CONTRE LA THEILERIOSE (EAST COAST FEVER)

(84) Designated Contracting States:
**AT BE BG CH CY CZ DE DK EE ES FI FR GB GR HU IE IT LI LU MC NL PL PT RO SE SI SK TR**

(30) Priority: **22.09.2003 US 504428 P**
**14.07.2004 US 22605**

(43) Date of publication of application:
**21.06.2006 Bulletin 2006/25**

(73) Proprietors:
- **International Livestock Research Institute ( ILRI) 00100 Nairobi (KE)**
- **THE INSTITUTE FOR GENOMIC RESEARCH Rockville, Maryland 20850 (US)**

(72) Inventors:
- **TARACHA, Evans Lavington, Nairobi (KE)**
- **GARDNER, Malcolm, J. Potomac, MD 20854 (US)**
- **NENE, Vishvanath Potomac, MD 20854 (US)**

(74) Representative: **Harding, Charles Thomas D Young & Co 120 Holborn London EC1N 2DY (GB)**

(56) References cited:
**US-A- 5 273 744**

- **DATABASE EMBL [Online] 2 January 2003 (2003-01-02), "EST628751 TpMugugaSh01 Theileria parva cDNA clone TPFAN22, mRNA sequence." XP002318733 retrieved from EBI accession no. EM_EST:BQ545112 Database accession no. BQ545112**
- **DATABASE EMBL [Online] 2 January 2003 (2003-01-02), "EST627619 TpMugugaSh01 Theileria parva cDNA clone TPFAF71, mRNA sequence." XP002330785 retrieved from EBI accession no. EM_PRO:BQ543992 Database accession no. BQ543992**
- **DATABASE EMBL [Online] 2 January 2003 (2003-01-02), "EST627620 TpMugugaSh01 Theileria parva cDNA clone TPFAF71, mRNA sequence." XP002330786 retrieved from EBI accession no. EM_PRO:BQ543993 Database accession no. BQ543993**
- **DATABASE EMBL [Online] 2 January 2003 (2003-01-02), "EST627151 TpMugugaSh01 Theileria parva cDNA clone TPFAC45, mRNA sequence." XP002330787 retrieved from EBI accession no. EM_PRO:BQ543524 Database accession no. BQ543524**
- **DATABASE EMBL [Online] 2 January 2003 (2003-01-02), "EST627152 TpMugugaSh01 Theileria parva cDNA clone TPFAC45, mRNA sequence." XP002330788 retrieved from EBI accession no. EM_PRO:BQ543525 Database accession no. BQ543525**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 670 820 B1

- **MCKEEVER DECLAN J ET AL: "Novel vaccines against Theileria parva: Prospects for sustainability" INTERNATIONAL JOURNAL FOR PARASITOLOGY, vol. 28, no. 5, May 1998 (1998-05), pages 693-706, XP002310753 ISSN: 0020-7519**
- **MORRISON W IVAN ET AL: "Theileriosis: Progress towards vaccine development through understanding immune responses to the parasite" VETERINARY PARASITOLOGY, vol. 57, no. 1-3, 1995, pages 177-187, XP002310754 ISSN: 0304-4017 cited in the application**
- **GERHARDS JOACHIM ET AL: "Sequence and expression of a 90-kilodalton heat-shock protein family member of Theileria parva" MOLECULAR AND BIOCHEMICAL PARASITOLOGY, vol. 68, no. 2, 1994, pages 235-246, XP002310752 ISSN: 0166-6851**

**Description**

**BACKGROUND INFORMATION**

Theilerioses are a group of disease syndromes affecting cattle, sheep, goats and domestic buffalo caused by tick-borne haemo-protozoan parasites in the genus *Theileria.* The most economically important diseases include East Coast fever (ECF), Mediterranean fever, and malignant theileriosis. ECF, caused by *Theileria parva (T: parva),* affects 30 million cattle in eastern, central and southern Africa. Mediterranean fever caused by *T. annulata* occurs in North Africa, southern Europe, Near East, Middle East and many parts of Far East Asia with a population of 200 million cattle and buffalo at risk. Malignant theileriosis caused by *T. lestoquardi* affects sheep and goats in southeastern Europe, North Africa, the Near and Middle East and southern Russia and neighboring States. These parasites belong to the same api-complexan group as *Plasmodium falciparum, Toxoplasma gondii, Cytoxauzoon spp, Eimeria spp* and *Babesia spp,* with a life-cycle having the arthropod and mammalian components in which sexual and asexual stages develop, respectively. The pathogenic stages of *Theileria* parasites differ. *T. parva* causes a lymphoproliferative disorder in which schizont-infected lymphoblasts are responsible for the pathogenesis of the disease. *T. parva* is known to cause a lymphoproliferative response in infected animals that resembles a variety of T-cell lynlphomas, leukemias and other lymphocytic cancers. On the other hand, anaemic disease caused by *T. lesloquardi* and *T. sergenti* is due to piroplasm-infected erythrocytes, while both the schizont and piroplasm of *T. annulata* are pathogenic resulting in lymphoproliferative and anaemic syndromes, respectively.

Currently, theilerioses are controlled largely by tick control using acaricides, such as the chlortetracyclines (first used as a prophylactic), hydroxynaphthoquinone, menoctone and its two analogues, parvaquone and bupavaquone, and through "infection and treatment" vaccination protocols of animals at risk. Such vaccination protocols are deemed effective in that an animal vaccinated in this manner will not develop ECF disease upon subsequent exposure to infectious *T. parva.* Due to cost and problems of tick resistance and environmental pollution, control of these diseases through acaricidal destruction of ticks is not sustainable.

Vaccination, on the other hand, while effective, presents with certain shortcomings associated with the use of live vaccines. Owing to the ease of transmission of *T. annulata,* infected blood was originally used to immunise cattle by employing parasites of low virulence as the immunizing agent. However, such immunisations were still accompanied by clinically-detectable infection episodes. With the advent of *in vitro* cultivation of *T. annulata* (Sharma et al. (1998) Vet. Parasito/. 79, 135041) and the development of bulk culture techniques in the 1960s, significant progress was made in realising a more practical immunisation strategy. Currently, passage-attenuated cultures of *T. annulata* are routinely used in national vaccination programs in affected countries. By contrast, similar efforts to immunise cattle against *T. parva* have been unsuccessful. This is attributed to the failure of attenuated *T. parva* parasites to induce immunity. In addition, much higher numbers of *T. parva*-infected cells are required to infect cattle reliably, since the schizonts of *T. parva* transfer at a low frequency and donor cells get rejected before successful transfer. *T. lestoquardi* has also been cultivated *in vitro* and studies have shown that attenuated parasites can be used to immunise animals with a degree of success.

Given the unsuccessful attempts to immunise cattle witch attenuated *T. parva,* subsequent efforts have focused on the use of virulent parasites with accompanying chemotherapy. The rationale of this infection and treatment method (ITM) is to allow the infection to establish and suppress development of frank clinical disease by administering theileriacidal drugs. Animals thus immunised were found to be protected against the development of disease when exposed to the homologous parasite. This vaccination strategy has undergone successive refinements including the use of cryopreserved triturated tick stabilites containing sporozoites (the parasite stage infective for cattle lymphocytes) to standardise the immunisation-infection dose, as well as simultaneous drug administration. Further improvement of this immunisation approach has involved the identification and use of a combination of parasite stocks to broaden the immunising spectrum of the vaccine against several field *T. parva* parasite populations. The use of local parasite stocks to immunise in areas where they have been isolated is also practised. ITM immunisation against *T. parva* has been tested extensively under laboratory and field conditions and is now deployed in the affected region to control ECF.

ITM has a number of practical limitations that hinder its application as a sustainable control measure against ECF in those geographical areas most affected by the disease. Being live, it requires a cold refrigerator chain, which is impractical in Africa. ITM can also cause clinical disease if drug application is inadequate and it has the potential to introduce new parasite strains in areas under the vaccination campaign. The cost (US$10-20 per immunisation) of this current vaccine is well beyond the budget of poor farmers with cattle afflicted by ECF, due to both the cost of the drugs and the requirement for a trained veterinarian to administer the vaccine. Because of these concerns associated with the ITM vaccine protocol, a great deal of investment has been put in research work to develop a vaccine that will be sustainable and affordable.

Antigens of many parasitic protozoans that induce a protective antibody response against the development of disease have been identified. For example, the major merozoite surface protein of *Plasmodium* species has been shown to be a target of varying degrees of protective immunity against the asexual blood stages in rodent and human malaria. Vaccination of mice with purified P230, the major merozoite surface protein of the rodent malaria *Plasmodium yoelii,* has resulted in reduced parasitemias in comparison to controls upon intravenous challenge with a lethal dose of parasitized erythrocytes (Holder et al. 1981. Nature 294:361). Mice have also been protected against *P. yoelii* by passive transfer of a monoclonal antibody (Mab) specific for P230 (Majarian et al. 1984. J. Immunol. 132:3131). Mice have also been immunized against (rodent malaria) *Plasmodium chabaudi adami* challenge, by passive immunization with a monoclonal antibody specific for the homologous 250-kDa molecule of this *Plasmodium* species (Lew et al. 1989. Proc. Natl. Acad. Sci. USA 86:3768).

A 67 kDa glycoprotein (p67) from the surface of the *T. parva* sporozoite has been isolated (U.S. Patent Number 5273744) and used in a variety of immunization protocols, with little success reported so far, in the development of pratical levels of immune-mediated disease resistance. However, cattle recovering from a single infection with *T. parva* sporozoites resist infection upon homologous challenge. Such animals have weak antibody and T cell responses to p67. There is a need to identify *T. parva* antigens that can induce antigen-specific class I MHC-restricted $CD8^+$ cytotoxic T lymphocytes (CTLs).

McKeever and Morrision (1998; International Journal for Parasitology 28:693-706) disclose a first-generation subunit vaccine for East Coast fever based on the *Theileria parva* p67 sporozoite surface antigen that is entering preliminary field trials.

## SUMMARY OF THE INVENTION

In one aspect, the present invention provides an isolated polypeptide comprising a sequence represented by SEQ ID NO:1, or a variant thereof having greater than 90% identity thereto and having the same or similar biological activity as SEQ ID NO: 1, or any one of SEQ ID Nos 4 to 7.

In a further aspect, the present inventions provides a pharmaceutical composition, comprising a polypeptide of the present invention and a pharmaceutically acceptable carrier.

There is provided, in another aspect of the present invention, an immunogenic composition, comprising a polypeptide of the present invention and, optionally, an adjuvant, the composition being immunogenic.

In another aspect, the present invention provides a vaccine, comprising one or more polypeptides of the present invention and, optionally, an adjuvant; wherein said polypeptide is immunogenic.

There is provided, in a further aspect, a method for producing a polypeptide which stimulates a *T.* parva-antigen specific cytotoxic lymphocyte (CTL), comprising culturing a host cell, wherein said host cell comprises a vector comprising a recombinant construct comprising a polynucleotide encoding a polypeptide according to the present invention, operably linked to an expression control sequence, wherein said host cell is cultured under conditions effective for producing a polypeptide encoded by the polynucleotide, and harvesting the polypeptide.

Further, there is provided in another aspect of the present invention an antibody specific for the polypeptide of the present invention.

There is provided, in a further aspect of the present invention, a kit for detecting the presence of *T. parva* in a sample suspected of containing *T. parva,* or for purifying *T. parva* from a sample containing *T. parva,* comprising an antibody according to the present invention.

In another aspect, the present invention provides the use of a polypeptide according to the present invention, or a vector comprising a recombinant construct comprising a polynucleotide encoding a polypeptide according the present invention, operably linked to an expression control sequence, or a host cell comprising a vector comprising a recombinant construct comprising a polynucleotide encoding a polypeptide according to the present invention, operably linked to an expression control sequence, in the preparation of a medicament for the prevention and/or treatment of infection by *T. parva.*

The present invention provides, in another aspect, a method for preparing a polyclonal antibody, comprising immunizing an animal with one or more polypeptides of the present invention; and recovering serum from the animal.

The present invention provides, in a further aspect, a method for preparing a polyclonal antibody, comprising immunizing an animal with a host cell; wherein said host cell comprises a vector comprising a recombinant construct comprising a polynucleotide encoding a polypeptide according to the present invention, operably linked to an expression control sequence; and recovering serum from the animal.

In another aspect, the present invention provides a method for preparing a monoclonal antibody, comprising:

(a) immunizing an animal with a polypeptide according to the present invention,
(b) recovering cells from the animal which produce antibody that binds to the polypeptide,
(c) preparing a hybridoma with the cells isolated in (b), and
(d) recovering a monoclonal antibody from the hybridoma that binds to the polypeptide in (a).

In a further aspect, the present invention provides a method for preparing a monoclonal antibody, comprising:

(a) immunizing an animal with a host cell; wherein said host cell comprises a vector comprising a recombinant construct comprising a polynucleotide encoding a polypeptide according to the present invention, operably linked to an expression control sequence,
(b) recovering cells from the animal which produce antibody that binds to a polypeptide produced by the host cell,
(c) preparing hybridomas with the cells isolated in (b), and
(d) recovering a monoclonal antibody from the hybridoma that binds to the polypeptide in (b).

There is provided, in a further aspect of the present invention, the use of an antibody according to the present invention in the preparation of a composition for the diagnosis of infection by *T. parva* in a sample.

There is provided, in another aspect of the present invention, a vaccine comprising a vector comprising a recombinant construct comprising a polynucleotide encoding a polypeptide according to the present invention, operably linked to an expression control sequence.

In another aspect, the present invention provides a vector comprising a recombinant construct comprising a polynucleotide encoding a polypeptide according to the present invention, operably linked to an expression control sequence.

The description relates, *e.g.*, to methods for the identification of parasite antigens, such as *Theileria parva* antigens, that stimulate antigen-specific cytotoxic T lymphocyte (CTL) responses (*e.g.* for inducing immunoprotection against *T. parva* in bovine species); and compositions identified by this method (*e.g.* that can be used to generate a protective response in cattle, to resist the development of ECF disease, when subsequently challenged with infectious *T. parva* material). More particularly, the method includes steps wherein polynucleotide sequences encoding candidate antigens are identified by conventional nucleic sequence alignment and parsing programs that have been fine-tuned using polynucleotide sequence information employing polynucleotide sequences known to encode *T. parva* antigens that stimulate CTLs in an antigen-specific manner. Candidate antigens and epitopes, and the nucleotide sequences that encode or result in the production of these candidate antigens and/or epitopes, that were identified, can be used, *e.g.*, to stimulate CTLs and to successfully immunize cattle against infection with subsequent exposure to *T. parva* expressing such antigens.

In further refinement of the antigenic properties of the polypeptides encoded by nucleotide sequences identified in such a manner, the nucleotide sequences are transfected into cells wherein the stimulation of responding lymphocytes to cells transfected by these nucleotides encoding parasite antigen is measured in a high throughput manner, by the release of soluble factors, such as gamma interferon, using either an antibody-elispot assay or a bioassay employing endothelial cells. The use of immortalized skin fibroblast (iSF) cells from outbred animals that have recovered from exposure, as antigen presenting cells, confirms the antigen identification, especially where cloned bovine MHC class I genes are not available for co-transfection into COS-7 cells.

One aspect mentioned herein is an isolated polypeptide, comprising a sequence represented by one of SEQ ID NO:1 through SEO ID NO:7. Such isolated polypeptides are sometimes referred to herein as "polypeptides described herein." In embodiments, an isolated polypeptide described herein is in detectable amounts in isolates of *T. parva*; and/or comprises a *T. parva* antigen.

Another aspect mentioned herein is a pharmaceutical composition, which comprises a polypeptide described herein and a pharmaceutically acceptable carrier or excipicent. Another aspect mentioned herein is an immunogenic composition, which comprises one or more polypeptides described herein and, optionally, an adjuvant. The immunogenic composition may stimulate cytotoxic T cells specific to the polypeptide; and/or comprise an epitope that stimulates *T. parva*- specific cytotoxic T cells. Another aspect mentioned herein is a vaccine, which comprises one or more polypeptides described herein and, optionally, an adjuvant. In an embodiment, the vaccine protects an animal against *T. parva* infection.

Another aspect mentioned herein is an isolated polynucleotide comprising:

(a) a sequence represented by one of SEQ ID NO:25 through SEQ ID NO: 31;
(b) a sequence which is at least about 90% identical to a sequence of (a);
(c) a sequence which hybridizes under conditions of high stringency to a polynucleotide which comprises a sequence of (a);
(d) a sequence which encodes a polypeptide represented by SEQ ID NO:1 through SEQ ID NO:7; or
(e) a complement of any of (a), (b), (c) or (d). Such isolated polynucleotides are sometimes referred to herein as "polynucleotides described herein."

Other aspects described herein include a pharmaceutical composition comprising a polynucleotide described herein and a pharmaceutically acceptable carrier or excipient; a recombinant construct comprising a polynucleotide described herein, which is operably linked to an expression control sequence; and a vector comprising such a construct.

The term "operably linked" refers to a functional linkage between a nucleic acid expression control sequence (such as a promoter, or array of transcription factor binding sites) and a second nucleic acid sequence, wherein the expression control sequence directs transcription of the nucleic acid corresponding to the second sequence. A vector mentioned herein may further comprise one or more sequences encoding a selectable marker; and the vector may comprise a plasmid, a bacteriophage, a minichromosome or a eukaryotic virus vector. The selectable marker can be a nucleotide sequence that, when incorporated and expressed, provides resistance to an antibiotic such as geneticin (G418), penicillin, tetracycline, or other types of selectable markers such as methotrexate resistance, expression of a metallothionein gene or a luciferase gene. Another aspect mentioned herein is a host cell (*e.g.*, a prokaryotic cell or a eukaryotic cell) which comprises a polynucleotide or a vector described herein. Another aspect mentioned herein is a method for producing a polypeptide which stimulates a *T. parva*-antigen specific cytotoxic lymphocyte (CTL), comprising culturing a host cell mentioned herein under conditions effective for producing a polypeptide encoded by the polynucleotide, and harvesting the polypeptide

Another aspect mentioned herein is an antibody (*e.g.*, a polyclonal antibody or a monoclonal antibody) specific for a polypeptide described herein. In an embodiment, the antibody is coupled to a carrier and/or a label.

Another aspect mentioned herein is a method for preparing a polyclonal antibody, comprising immunizing a non-human animal with one or more polypeptides described herein or with cells comprising polynucleotides or vectors described herein. Another aspect mentioned herein is a method for preparing a monoclonal antibody, comprising (a) immunizing a non-human animal with a polypeptide described herein; (b) recovering cells from the non-human animal which produce antibody that binds to the polypeptide; (c) preparing a hybridoma with the cells isolated in (b), and (d) recovering a monoclonal antibody from the hybridoma that binds to the polypeptide in (a). Another aspect mentioned herein is a method for preparing a monoclonal antibody, comprising: (a) immunizing a non-human animal with a host cell comprising a polynucleotide or vector of the described herein;(b) recovering cells from the non-human animal which produce antibody that binds to a polypeptide produced by the host cell; (c) preparing hybridomas with the cells isolated in (b), and (d) recovering a monoclonal antibody from the hybridoma that binds to the polypeptide in (b).

Another aspect mentioned herein is a kit for detecting the presence of *T. parva* in a sample suspected of containing *T. parva*, or for purifying *T. parva* from a sample containing *T. parva,* comprising an antibody mentioned herein. Detection of *T. parva* in a sample may require cell lysis or cell membrane solubilization by standards methods, such as, but not limited to, the use of non-denaturing detergents or the like. The kit may further comprise means for performing an enzyme-linked or Western blot assay to detect the presence of *T. parva*; and/or means for binding the antibody to *T. parva* in the sample, and for releasing the organism from the antibody.

Another aspect mentioned herein is a method for protecting an animal against infection by *T. parva,* comprising administering to the animal a polypeptide described herein under conditions effective for the animal to generate a protective antibody against the polypeptide, or effective for the animal to generate *T. parva*-antigen-specific CTLs. Another aspect mentioned herein is a method for protecting an animal against infection by *T. parva*, comprising administering to the animal a cell comprising a polynucleotide or vector of the described herein, under conditions effective for the animal to generate a protective antibody against a polypeptide encoded by the polynucleotide (expressed from the polynucleotide), or effective for the animal to generate *T. parva*-antigen-specific CD4+ helper and CD8+ CTL responses. A "CD8+ CTL response" means that the stimulation of the CD8+CTL can be detected by measuring the release of a factor, such as a soluble factor or other response such as the lysis of antigen-displaying cell.

Another aspect mentioned herein is a method for detecting a pathogenic protozoan infection in a subject, comprising contacting peripheral blood monocytes from the subject with peptide-antigen pulsed cytotoxic T lymphocytes, wherein the cytotoxic T lymphocytes are obtained from an animal to which has been administered a polypeptides described herein, under conditions effective for the animal to generate *T. parva*-antigen-specific CTLs, or effective for the animal to generate *T. parva*-antigen-specific CD4+ helper and CD8+ cytotoxic T lymphocyte responses. Another aspect mentioned herein is a method for detecting *T. parva* in a sample suspected of containing *T. parva,* comprising detecting in the sample a polynucleotide described herein. Any of the method mentioned herein, including these detection methods, may be high throughput methods.

Another aspect mentioned herein is a method for identifying *T. parva* in a sample suspected of containing *T. parva,* comprising contacting the sample with an antibody mentioned herein, under conditions effective for the antibody to bind specifically to its cognate antigen, and detecting the presence of bound antibody. In embodiments mentioned herein, the detection is carried out by enzyme immunoassay, radioimmunoassay, fluorescence immunoassay, flocculation, particle agglutination, flow microfluorimetry, a competition assay, or *in situ* chromogenic assay; the antibody is either a monoclonal antibody or a polyclonal antibody; the assay is quantitative; or the assay is high through put.

Another aspect mentioned herein is a method for the identification of parasite antigens, selected from candidate antigens, that are targets of cytotoxic T lymphocytes or other immune responses, comprising co-culturing immortalized fibroblast cell lines transfected with pooled cDNA harvested from a pathogen, with clones of lines of cytotoxic T cells, generated in an animal that has been immunized, by infection and treatment with the pathogen and assaying the supernatant from the co-culture for the presence of a soluble factor, such as a cytokine, *e.g.* a gamma interferon; the pathogen is a protozoan organism (*e.g.* an organism in the genus *Theileria,* such as *T. parva*); the fibroblast cell line is of bovine origin; or the fibroblast cell line of bovine origin displays bovine Class I, MHC antigens.

In another embodiment, *T. parva* antigen encoding polynucleotides, a method for the identification of candidate parasite antigens, that are targets of cytotoxic T lymphocytes or other immune responses, comprising using conventional genome scanning and alignment methods such as "GlimmerM" and "phat" in which sequence information of candidate antigens that cause stimulated of CTLs is used to refine the genome scanning and alignment methods. Such sequence identity may be optimized by sequence comparison and alignment algorithms known in the art (see Gribskov and Devereus, Sequence Analysis Primer, Stockton Press, 1991 and the references cited therein) and calculating the percent difference between the nucleotides sequences by, for example, the Smith-Waterman algorithm as implemented in the BESTFIT software program using default parameters (e.g., University of Wisconsin Genetic Computing Group).

An additional embodiment mentioned herein is a method of using the nucleotide sequences, that have been shown to encode *T. parva* antigens which stimulate CTLs, to identify additional candidate CTL-stimulatory antigens from nucleotide sequence information, such as sequence contigs or whole genome sequence isolate from an organism using techniques such as hybridization under conditions of high stringency, or under other conditions that allow the identification of sequences with 95%-97% homology with known sequence.

As *T. parva* is known to cause a lymphoproliferative response in infected animals that resembles a variety of T-cell lymphomas, leukemias and other lymphocytic cancers the presently identified *T. parva* proteins, for example, could have utility in formulating an anticancer composition, e.g., antigenic proteins could have utility in prevention schemes, whereby radioactively labeled-antigens would compete for intracellular or extracellular binding sites in order to destroy cells undergoing lymphoproliferation. The *T. parva* antigens mentioned herein are also candidates for a method to prevent lymphoproliferation by assisting in the identification and manufacture of small molecules that would manipulate the signaling pathways targeted by parasite proteins.

## DESCRIPTION OF THE DRAWINGS

FIG. 1 is a graph that demonstrates IFN-gamma elispot detection of BoLA class I restricted CTL responses to schizont infected cells. Autologous TpM were pre-incubated in elispot plates with monoclonal antibodies specific for BoLA class I, BoLA class II or bovine CD21 (isotype control) before the addition of schizont specific polyclonal CTL. Cells were co-cultured for 20 hours before the plates were developed. IFN-gamma production is presented as mean number of spot forming cells (SFC)/well.

FIG.2A is a graph that indicates the sensitivity of IFN-gamma elispot to detect CTL recognition of schizont infected cells when the effects of varying the CTL input on responses to TpM titrated in COS-7 cells was addressed; FIG. 2B is a graph that indicates the ability of the elispot test to detect responses to very small numbers of TpM, using a CTL input of 10,000/well. FIG.2C shows the effect of titrating TpM in COS-7 cells, when autologous primary SF or iSF were examined and found to have no effect on the background or sensitivity of the elispot assay. IFN-gamma production is presented as mean number of spot forming cells (SFC)/well.

FIG. 3 is a graph showing the results generated when selected Tp2 transfected immortalized skin fibroblast cells (iSF) were used to stimulate BW002, BW014 CD8+ polyclonal CTL lines and D409 CTL clone #10. Recognition of selected gene #5 (also known as, Tp2) transfected iSF by BW002 (BW2), BW014 (BW14) CD8+ polyclonal CTL lines and D409 CTL clone #10 was measured by co-culture of transfected cells with CTLs. The iSF were transfected with selected genes, cultured with CTL and recognition assessed by IFN-gamma elispot. Responses are presented as mean numbers of spot forming cells (SFC)/well.

FIG. 4 is a graph indicating the lysis of Tp2 transfected autologous (BW002) and allogeneic (BV050) iSF by the schizont specific BW002 polyclonal CD8+ CTL line. Tp5 transfected autologous iSF also served as a negative control. BoLA class I restriction was assessed by pre-incubating Tp2 transfected iSF with an anti-BoLA MHC Class I mAb (class I block). Data are expressed as percent of cells that are lysed.

FIG. 5A, FIG. 5B, and FIG. 5C indicate Tp2 specific CD8+ T cell responses following challenge of immune cattle. Three ECF immune cattle, 5A (BW002), 5B (BW013), and 5C (BW014), from which schizont specific CTL lines had been generated and shown to recognize Tp2, --were challenged with a lethal dose of *T. parva* (Muguga) sporozoites. Peripheral blood was collected daily between day 7 - 13 post-challenge, and CD8+ T cells and monocytes purified. Responses to Tp2 peptides containing previously identified CTL epitopes (positive or "+ve") or control peptides were assessed by co-culturing CD8+ T cells and monocytes in the presence of 1mg/ml peptide and measuring the release of IFN-gamma by Elispot. Responses to autologous TpM were included as a positive control. Responses are presented as mean numbers of spot forming cells (SFC)/well.

FIG. 6 is a graph showing the mapping of Tp2 CTL epitopes using synthetic peptides. Eighty-four 12mer peptides overlapping by two amino acids encompassing the full length of Tp2 were synthesized and used at a final concentration of 1pg/ml for BW002 (BW2) or 1ug/ml for BW013 (BW13), BW014 (BW14) & D409, to pulse autologous iSF. Recognition of peptide pulsed iSF by CD8+ polyclonal lines from BW002 (BW2), BW013 (BW13), BW014 (BW14), and D409 CTL clone #10 was assessed by IFN-gamma-elispot. Significant responses were observed against different sets of overlapping peptides suggesting a number of unique, overlapping and shared CTL epitopes. IFN-gamma production is presented as mean number of spot forming cells (SFC)/well.

FIG. 7 is a graph that illustrates the identification of Tp2 CTL epitopes using synthetic 9mer peptides. The 6 possible 9mer peptides derived from the epitope containing SEQ ID. NO:11, FAQSLVCVLMKCRG were synthesized and used at a final concentration of 1ug/ml to pulse autologous iSF. Recognition of peptide pulsed iSF by CD8+ polyclonal lines from BW013 and BW014, and D409 CTL clone #10 assessed by IFN-gamma-elispot, indicated that the peptide #. IFN-gamma production is presented as mean number of spot forming cells (SFC)/well.

FIG. 8 is a graph of data indicating the lysis of Tp2 synthetic peptide pulsed autologous iSF by the schizont specific D409 CTL clone #10. D409 iSF were pulsed overnight with the Tp2 epitope containing 12mer peptide #77; (SEQ ID. NO:14) or a control Tp2 12mer peptide #36; (SEQ ID NO:15) at final peptide concentration of 1ug/ml. Autologous and allogeneic (4229) TpM were included as a positive and negative controls. Data are expressed at percent of peptide-pulsed cells that were lysed.

FIG. 9 is a graph that indicates the lysis of Tp2 synthetic peptide pulsed autologous iSF by the schizont specific BW014 polyclonal CD8+ CTL line. BW014 iSF were pulsed overnight with the Tp2 epitope 9mer peptide #75, (SEQ ID NO: 16) or a control Tp2 9mer peptide #76, (SEQ ID NO:17) at final peptide concentration of 1ug/ml. Autologous and allogeneic (F100) TpM were included as a positive and negative controls. Data are: expressed at percent of peptide-pulsed cells that were lysed.

FIG. 10 shows the minimal length of the CTL-stimulating epitope Tp2.1, present within the Tp2 polypeptide as established by using sets of overlapping peptides in an Elispot assay. Autologous immortalized fibroblasts (iSF) or P815 cells, stably expressing the BoLA Class I HD6 (P815-HD6) or JSP-1 (P815-JSP-1) were pulsed with overlapping 9-mer polypeptides represented by SEQ ID. NO: 4, 16, and 17 and the 8-mer SEQ ID NO: 18. Pulsed cells were tested for IFN-gamma release using the Elispot assay. Responses are presented as mean numbers of spot forming cells (SFC)/well.

FIG. 11 shows the minimal length of the CTL-stimulating epitope Tp2.2, present within the Tp2 polypeptide as established by using sets of overlapping peptides in an Elispot assay. Autologous immortalized fibroblasts (iSF) or P815 cells, stably expressing the BoLA Class I HD6 (P815-HD6) or JSP-1 (P815-JSP-1) were pulsed with overlapping 9-mer polypeptides represented by SEQ ID. NO: 5 (Tp2 Epitope 2 (Tp2.2)) and 16 (synthesized peptide #75, amino acids residues 97-105 of Tp2), and the 8-mer SEQ ID NO: 21 (amino acid residues 97-104 of Tp2). The peptides shown in the figure, from left to right, are SEQ ID NO: 20, NO: 5 and NO: 16. Pulsed cells were tested for IFN-gamma release using the Elispot assay. Responses are presented as mean numbers of spot forming cells (SFC)/well.

FIG. 12 shows the minimal length of the CTL-stimulating epitope Tp2.3, present within the Tp2 polypeptide as established by using sets of overlapping peptides in an Elispot assay. Autologous immortalized fibroblasts (iSF) or P815 cells, stably expressing the BoLA Class I HD6 (P815-HD6) or JSP-1 (P815-JSP-1) were pulsed with overlapping 10-mer polypeptides represented by SEQ ID. NO: 13 (a peptide of Tp2) and 20 (amino acid residues 49-58 of Tp2), and the 11-mer SEQ ID NO: 6 (Tp2 epitope 3 (Tp2.3)). The peptides shown in the figure, from left to right, are SEQ ID NO: 13, NO: 6 and NO: 20. Pulsed cells were tested for IFN-gamma release using the Elispot assay. Responses are presented as mean numbers of spot forming cells (SFC)/well.

FIG. 13 shows the minimal length of the CTL-stimulating epitope Tp2.4, present within the Tp2 polypeptide as established by using sets of overlapping peptides in an Elispot assay. Autologous immortalized fibroblasts (iSF) or P815 cells, stably expressing the BoLA Class I HD6 (P815-HD6) or JSP-1 (P815-JSP-1) were pulsed with overlapping 12-mer polypeptides represented by SEQ ID. NO: 8 (a peptide of Tp2), 22 (amino acid residues 97-104 of Tp2), 23 (amino acid residues 28-39 of Tp2) and the 11-mer SEQ ID NO: 7 (Tp2 epitope 4 (Tp2.4)). The peptides shown in the figure, from left to right, are SEQ ID NO: 22, NO: 8, NO: 23 and NO: 7. Pulsed cells were tested for IFN-gamma release using the Elispot assay. Responses are presented as mean numbers of spot forming cells (SFC)/well.

FIG. 14 is a graph that indicating the lysis of Tp2 peptide-pulsed autologous iSF by TpM stimulated PBMC. BW002 PBMC were collected 14 days post-challenge and co-cultured for 7 days with autologous TpM. Cells were tested for their ability to lyse Tp2 peptide pulsed autologous iSF using a $^{51}$Chromium release assay. iSF were pulsed overnight with a Tp2 12mer peptide known to contain a CTL epitope (#43; positive (+ve peptide)) or a control Tp2 12mer peptide (#40; control peptide) at a concentration of 1ug/ml. Autologous and allogeneic (F100) TpM were included as positive and negative controls. Data are expressed at percent of peptide-pulsed cells that were lysed.

FIG. 15A and FIG. 15B represents a photograph of an agarose gel showing the expression of *T. parva* Tp2 antigen protein. Recombinant protein was isolated by Ni-NTA agarose and run on 12% SDS-PAGE gels. In figure 15A the proteins that are present in the gel are stained with Coomassie blue. In figure 15B an anti-His-tag antibody is used to specifically stain the expressed Tp2-His-labelled protein (immunoblot).

FIG. 16 is a photograph of an SDS-PAGE (12.5%) analysis of over-expression of recombinant Tp3 in *E. coli.* The complete ORF of Tp3 containing 5' *Bam*HI and 3' *Bgl* II was PCR amplified and cloned into plasmid vector pQE-16 (Qiagen) from which DFHR fragment was excised using *Bam*H I and *Bgl* II. The rcombinant plasmid was transformed into E. coli JM109 and plated. Two colonies (Tp3-1 and Tp3-2) were picked for over-expression as described in the "Example" section. Tp3-1 was expressed in LB broth (Tp3-la) or in 2X YT (Tp3-1b) while Tp3-2 was expressed only in LB broth. Aliquots of midi-preps of the cultures were separated using SDS-PAGE (12.5%) gel electrophoresis. The proteins in the resultant gel were stained with Coomassie blue. An irrelevant protein of 50 kDa was used as control (TLTF). Lane MW is the size makers indicated in kiloDalton (kDa).

FIG. 17 is a photograph of an SDS-PAGE (12.5%) analysis of purified recombinant Tp6. A partial fragment (70%) of Tp6 was PCR amplified and cloned into plasmid vector pQE-16 (Qiagen). The recombinant plasmid was transformed into *E. coli* JM109. A number of colonies were identified for over-expression. The protein was purified using an anti-his-tag antibody and then analyzed using SDS-PAGE electrophoresis, followed by staining of the proteins in the polyacrylamide gel with Coomassie blue. Size markers are indicated in kiloDalton (kDa).

FIG. 18 indicates the level of CTL responses to several Tp antigens, following CP(Canary Pox mediated)/MVA (modified Vaccinia Virus Ankara-vector mediated) immunization protocols. The data indicates the production of *T. parva* antigen-specific CD8 (CTLs) in animals following immunization protocols with CanaryPox viral vectors, expressing *T. parva* antigen. Responses are presented as mean numbers of spot forming cells (SFC)/well.

FIG. 19 indicates the level of CTL responses to several Tp antigens, following DNA/MVA immunization protocols. The data indicates the production of *T. parva* antigen-specific CD8 (CTLs) in animals following immunization protocols with modified Vaccinia (Ankara) viral vectors, expressing *T. parva* antigen. Responses are presented as mean numbers of spot forming cells (SFC)/well.

FIG. 20 indicates control data for CP/MVA and DNA/MVA immunization protocols, where cattle were given phosphate buffered saline injections rather than Tp antigens. The control experimental data for measuring the development of CTLs following immunization indicates a lack of *T parva,* antigen-specific CD8+ cells in PBS immunized animals. Responses are presented as mean numbers of spot forming cells (SFC)/well.

## DETAILED DESCRIPTION

Class I MHC-restricted $CD8^+$ cytotoxic T lymphocytes (CTLs) are responsible for protecting cattle against a lethal challenge with Theileria parva (*T. parva*), sporozoites (Morrison, Taracha, and McKeever, 1995). These CTLs are directed at schizont-infected cells, which they recognize and lyse. Schizont antigens that are recognized by these $CD8^+$ CTLs are the prime candidates for inclusion into an effective sub-unit vaccine for the prevention of East Coast Fever disease (ECF).

The examples herein illustrate the identification of a variety of antigens which are suitable for use in subunit vaccines.

There is provided polynucleotides, and novel methods for their identification, which encode proteins and peptides that are targets of antigen-specific cytotoxic CD8+ T lymphocytes which have been shown to be protective against ECF infection in adoptive cell transfer experiments. Experimental immunisation of cattle with these antigens or recombinant viral vector cell systems producing these antigens have stimulated the production of antigen-specific T cell responses in the immunized animals.

The proteins identified by the novel method can also be used to as antigens to stimulate the production of Antibodies. Such antibodies are useful for the detection of the presence of a stimulating antigen, in animals tissues and fluids. Presence of such antigen would indicate infection of the animal by organisms producing or bearing, the antigen. For example, in one embodiment mentioned herein infection of an animals by *T. parva* is detected by antibodies produced as a result of using peptides encoded by polynucleotide sequences as antigens to stimulate the production of such antibodies.

The *T. parva* nuclear genome consists of 4 chromosomes and is approximately 8.2 Mb in length. The *T. parva* genome was sequenced using a whole genome shotgun strategy. *T. parva* genomic DNA (Muguga clone) was sheared and cloned into plasmid vectors. Both ends of randomly selected clones were sequenced. A total of 152,226 sequences with an average read length of 623 nt were obtained, equivalent to 9.48X coverages assuming a genome size of 10 Mb. Assembly of the sequences with "TIGR Assembler" produced a set of preliminary contigs representing 95% of the genome sequence to begin the process of identification of schizont antigens. The preliminary contigs were loaded into an annotation database and subjected to an automated process that searched the *T. parva* contigs against a nonredundant database of proteins extracted from GenBank (called "nraa"). The search results were used to produce a set of *T. parva* gene models that encoded proteins similar to those in other organisms. This set of gene models and sequence data from 164 cDNA clones were used to train the gene finding programs "GlimmerM" and "phat", which were subsequently run against all of the preliminary contigs to produce gene models for the entire preliminary genome sequence. A program called "Combiner" was then used to evaluate predicted gene models, cDNA and protein alignments to produce consensus gene models.

The proteins encoded by the predicted *T. parva* genes were subjected to a variety of analyses such as database searched ("blastp"), predictions of signal peptides and signal anchors ("SignalP 2.0"), and transmembrane domains ("TMHMM"). The results were reviewed and a set of about 55 genes encoding candidate antigens were selected for screening for immunogenicity, as tested by stimulation of cloned cytotoxic T cells lines. When these gene sequences were screened, this led to the identification of antigens referred to herein as Tp2 (SEQ ID NO: 1), Tp3 (SEQ ID NO: 2), and Tp6 (SEQ ID NO: 3). The nucleic acid sequences encoding Tp2, Tp3 and Tp6 are represented by SEQ ID NOs: 25, 26 and 27, respectively.

The candidate antigens encoded by polynucleotide sequences, cloned from expressed genes of *T. parva,* were confirmed by the use of immortalized cloned bovine skin fibroblast cell lines to stimulate the activation of cytotoxic CD8+ T lymphocytes by antigen when stimulation is measured by the release of soluble factors, more specifically gamma interferon. The description also relates to compositions isolated by methods including one or more of these particular steps and the use of these compositions: to stimulate or induce cytotoxic T cells, as diagnostic reagents for the detection of disease or an immune response, in kits or high throughput "chip" methods or assay, for the detection of or expression of, identical or homologous nucleic acids. In another preferred embodiment, the antigens identified are useful for immunization of animals for the production of CTLs that recognize *T. parva.*

The present description identifies a group of polynucleotide sequences that encode *T. parva* antigens useful for a variety of applications.

The nucleic acids described herein may comprise recombinant nucleic acid. By the term "recombinant nucleic acid" herein is meant nucleic acid, originally formed in vitro or in a cell in culture, in general, by the manipulation of nucleic acid by endonucleases and/or exonucleases and/or polymerases and/or ligases and/or recombinases, to produce a nucleic acid not normally found in nature. Thus an isolated nucleic acid, in a linear form, or an expression vector formed in vitro by ligating DNA molecules, that are not normally joined, are both considered recombinant for the purposes mentioned herein, It is understood that once a recombinant nucleic acid is made and reintroduced into a host cell or organism, it will replicate non-recombinantly, i.e., using the in vivo cellular machinery of the host cell rather than in vitro manipulations; however, such nucleic acids, once produced recombinantly, although subsequently replicated non-recombinantly, are still considered recombinant for the purposes mentioned herein.

Furthermore, as a result of the degeneracy of the genetic code, a multitude of nucleotides sequences may be produced which are based upon the sequences provided herein and corresponding peptides, polypeptides, or proteins. Some of these nucleotide sequences will bear only minimal homology to the sequences disclosed herein; however the subject described herein specifically contemplates each and every possible variation of nucleotide sequence that could be made by selecting combinations based on possible codon choices. These combinations are made in accordance with the standard triplet genetic code as applied to the nucleotide sequence of naturally occurring peptide, polypeptide, or protein, and all such variations are to be considered as being specifically disclosed herein. Recombinant nucleotide variants are alternate polynucleotides which encode a particular protein. They may be synthesized, for example, by making use of the "redundancy" in the genetic code. Various codon substitutions, such as the silent changes which produce specific restriction sites or codon usage-specific mutations, may be introduced to optimize cloning into a plasmid or viral vector or expression in a particular prokaryotic or eukaryotic host system, respectively.

It is possible to produce the polynucleotides described herein, or portions thereof, entirely by synthetic chemistry. After synthesis, the nucleic acid sequence can be used alone or joined with a preexisting sequence and inserted into one of the many available DNA vectors and their respective host cells using techniques well known in the art Moreover, synthetic chemistry may be used to introduce specific mutations into the nucleotide sequence. Alternatively, a portion of sequence in which a mutation is desired can be synthesized and recombined with a portion of an existing genomic or recombinant sequence.

Peptides and polypeptides described herein can also be produced, entirely or in part, by synthetic chemistry, using conventional procedures.

Nucleotide sequences encoding a peptide, polypeptide, or protein may be joined to a variety of other nucleotide sequences by means of well established recombinant DNA techniques (Sambrook J. et al. (1989) Molecular Cloning: A Laboratory Manual, Cold Spring Harbor Laboratory, Cold Spring Harbor, N.Y.; or Ausubel F. M. et al. (1989) Current Protocols in Molecular Biology, John Wiley & Sons, New York City). Useful sequences include an assortment of cloning vectors such as plasmids, cosmids, lambda phage derivatives, phagemids, and the like. Vectors of interest include vectors for replication, expression, probe generation, sequencing, and the like. In general, vectors of interest may contain an origin of replication functional in at least one organism, convenient restriction endonuclease sensitive sites, and selectable markers for one or more host cell systems.

Another aspect described herein is to provide for hybridization probes which are capable of hybridizing with naturally occurring antigen sequences or nucleotide sequences encoding the disclosed peptide, polypeptide, or protein. The stringency of the hybridization conditions will determine whether the probe identifies only the native nucleotide sequence or sequences of closely related molecules. If degenerate nucleotide sequences mentioned herein are used for the detection of related sequences, they should preferably contain at least 50% of the nucleotides of the sequences presented herein.

"Probes" are nucleic acid sequences of variable length, preferably between at least about 10 and as many as about 6,000 nucleotides, depending on use. They are used in the detection of identical, similar, or complementary nucleic acid sequences. Longer length probes are usually obtained from a natural or recombinant source, are highly specific and much slower to hybridize than oligomers. They may be single- or double-stranded and designed to have specificity in PCR, hybridization membrane-based, or ELISA-like technologies.

Hybridization probes mentioned herein may be derived from the polynucleotides represented by SEQ ID NO: 25-31, or from surrounding or included genomic sequences comprising untranslated regions such as promoters, enhancers and introns. Such hybridization probes may be labeled with appropriate reporter molecules. Means for producing specific hybridization probes include oligolabelling, nick translation, end-labeling or PCR amplification using a labeled nucleotide. Alternatively, the cDNA sequence may be cloned into a vector for the production of mRNA probe. Such vectors are known in the art, are commercially available, and may be used to synthesize RNA probes in vitro by addition of an appropriate RNA polymerase such as T7, T3 or SP6 and labeled nucleotides. A number of companies (such as Pharmacia Biotech, Piscataway, N.J.; Promega, Madison, Wis.; US Biochemical Corp, Cleveland, Ohio; etc.) supply commercial kits and protocols for these procedures.

The nucleotide sequences (for example, SEQ ID NO: 25-42, 45-48, 51 and 53) can be used to generate probes for mapping the native genomic sequence. The sequence may be mapped to a particular chromosome or to a specific region of the chromosome using well known techniques. These include in situ hybridization to chromosomal spreads, flow-sorted chromosomal preparations, or artificial chromosome constructions such as yeast artificial chromosomes (YACs), bacterial artificial chromosomes (BACs), bacterial P1 constructions or single chromosome cDNA libraries.

In situ hybridization of chromosomal preparations and physical mapping techniques such as linkage analysis using established chromosomal markers are invaluable in extending genetic maps in organisms, including intracellular parasites. The nucleotide sequences described herein may also be used to detect differences in the chromosomal location of nucleotide sequences due to translocation, inversion, or recombination.

Other aspects mentioned herein include use of the disclosed sequences or recombinant nucleic acids derived therefrom to produce purified peptides. The nucleotide sequences as disclosed herein maybe used to produce an amino acid sequence using well known methods of recombinant DNA technology. Goeddel (Gene Expression Technology, Methods and Enzymology [1990] Vol 185, Academic Press, San Diego, Calif) is one among many publications which teach expression of an isolated, purified nucleotide sequence. The amino acid or peptide may be expressed in a variety of host cells, either prokaryotic or eukaryotic. Some expression vectors are viral vectors such as Vaccinia-based vectors, avian-pox based vectors, and adeno-associated viral vectors, among others (Dunachie et al. (2003) J Exp Biol 206, 3771-9). Host cells may be from the same species from which the nucleotide sequence was derived, such as fibroblast cells, lymphocytes or the like, or cells or cell lines, such as P815 cells, from a different species. The disclosed sequences or recombinant nucleic acids can be introduced into the host cells by a variety of methods, known to those skilled in the art, such as methods that involve transfection. Transfection is the process whereby the nucleic acid sequences (as well as proteins and oligonucleotides) are introduced by either biochemical or physical processes. Biochemical methods include DEAE-dextran, calcium phosphate, and liposome-mediated transfection methods (such as lipofection). Physical transfection methods include the direct micro-injection of materials, biolistic particle delivery, *e.g.* a "gene gun" (both methods deliver materials by mechanically perforating the cell membrane), and electroporation. Electroporation exposes target cells to brief, defined electrical pulses to create transient pores that allow nucleic acids and proteins to cross the cell membrane.

Still further aspects mentioned herein use these purified peptides to produce antibodies or other molecules able to bind to the peptides. These antibodies or binding agents can then be used for the screening of cells in order to localize the cellular distribution of the peptides or proteins. The antibodies are also useful for the affinity purification of recombinantly produced peptides or proteins. Such antibodies are also useful as diagnostic reagents for the qualitative and quantitative detection of protozoan diseases or in antibody-mediated tests and assays.

Disclosed *T. parva* antigens can also be used as a diagnostic aid or in methods that measure the presence of *T. parva* cytotoxic lymphocytes in immunized or infected animals by co-culturing mononuclear cells harvested from an animal suspected to have an infection due to *T. parva,* with a *T. parva* antigen-specific cytotoxic T cell under standard culture conditions for mammalian cell cultures. Preferably, nononuclear cells are incubated in a culture medium containing an indicator that is released upon cell death, such as $^{51}Cr$. This is a method that is well-known in the art.

The disclosed nucleotide sequences can be used individually, or in panels, in tests or assays to detect levels of peptide, polypeptide, or protein expression. The form of such qualitative or quantitative methods may include northern analysis, dot blot or other membrane based technologies, dip stick, pin or chip technologies, PCR, ELISAs or other multiple sample format technologies.

An "oligonuelcotide" or "oligomer" is a stretch of nucleotide resides which has a sufficient number of bases to be used in a polymerase chain reaction (PCR). These short sequences are based on (or designed from) genomic or cDNA sequences and are used to amplify, confirm, or reveal the presence of an identical, similar or complementary DNA or RNA in a particular cell or tissue. Oligonucleotides or oligomers comprise portions of a DNA sequence having at least about 10 nucleotides and as many as about 50 nucleotides, preferably about 15 to 30 nucleotides. They can be chemically synthesized and may be used as probes.

A "complementary" DNA or RNA is a sequence that is 100% identical to the strand to which it is complementary.

The polynucleotides described herein can themselves be used as probes. Additional polynucleotide sequences can be added to the ends of (or internally in) the exemplified polynucleotide sequences so that polynucleotides that are longer than the exemplified polynucleotides can also be used as probes. Thus, isolated polynucleotides comprising one or more of the exemplified sequences are within the scope of the description. Polynucleotides that have fewer nucleotides than the, exemplified polynucleotides can also be used and are contemplated within the scope of the description For example, for some purposes, it might be useful to use a conserved sequence from an exemplified polynucleotide wherein the conserved sequence comprises a portion of an exemplified sequence. Thus, polynucleotides, described herein can be used to find additional, homologous (wholly or partially) genes.

Probes mentioned herein may be composed of DNA, RNA, or PNA (peptide nucleic acid). The probe will normally have at least about 10 bases, more usually at least about 17 bases, and may have up to about 100 bases or more. Longer probes can readily be utilized, and such probes can be, for example, several kilobases in length. The probe sequence is designed to be at least substantially complementary to a portion of a gene encoding a protein of interest The probe need not have perfect complementarity to the sequence to which it hybridizes. The probes may be labeled utilizing techniques that are well known to those skilled in this art.

One approach for the use as probes entails first identifying DNA segments that are homologous with the disclosed nucleotide sequences using, for example, Southern blot analysis of a gene bank. Thus, it is possible, without the aid of biological analysis, to know in advance the probable activity of many new polynucleotides, and of the individual gene products expressed by a given polynucleotide. Such an analysis provides a rapid method for identifying commercially valuable compositions.

One hybridization procedure useful according to the description typically includes the initial steps of isolating the DNA sample of interest and purifying it chemically. Either lysed cells or total fractionated nucleic acid isolated from cells can be used. Cells can be treated using known techniques to liberate their DNA (and/or RNA). The DNA sample can be cut into pieces with an appropriate restriction enzyme. The pieces can be interest can be isolated through electrophoresis in a gel, usually agarose or acrylamide. The pieces of interest can be transferred to an immobilizing.membrane.

The particular hybridization technique is not essential to the description. As improvements are made in hybridization techniques, they can be readily applied.

The probe and sample can then be combined in a hybridization buffer solution and held at an appropriate temperature until annealing occurs. Thereafter, the membrane is washed free of extraneous materials, leaving the sample and bound probe molecules typically detected and quantified by autoradiography and/or liquid scintillation counting. As is well known in the art, if the probe molecule and nucleic acid sample hybridize by forming a strong non-covalent bond between the two molecules, it can be reasonably assumed that the probe and sample are essentially identical or very similar. The probe's detectable label provides a means for determining in a known manner whether hybridization has occurred.

In the use of the nucleotide segments as probes, the particular probe is labeled with any suitable label known to those skilled in the art, including radioactive and non-radioactive labels. Typical radioactive labels include $^{32}P$, $^{35}S$, or the like. Non-radioactive labels include; for example, ligands such as biotin or thyroxine, as well as enzymes such as hydrolases or peroxidases, or the various chemiluminescers such as luciferin, or fluorescent compounds like fluorescein and its derivatives. In addition, the probes can be made inherently fluorescent as described in International Application No. WO 93/16094.

Various degrees of stringency of hybridization can be employed. The more stringent the conditions, the greater the complementarity that is required for duplex formation. Stringency can be controlled by temperature, probe concentration, probe length, ionic strength, time, and the like. Preferably, hybridization is conducted under moderate to high stringency conditions by techniques well known in the art, as described, for example, in Keller, G. H., M. M. Manak (1987) DNA Probes, Stockton Press, New York, N.Y., pp. 169-170.

As used herein "moderate to high stringency" conditions for hybridization refers to conditions that achieve the same, or about the same, degree of specificity of hybridization as the conditions described herein. Examples of moderate to high stringency conditions are provided herein. Specifically, hybridization of immobilized DNA on Southern blots with $^{32}P$-labeled gene-specific probes is performed using standard methods (Sambrook et al. (1989) Molecular Cloning: A Laboratory Manual, Cold Spring Harbor Laboratory Press, NY, Vol. 1, 2, 3). In general, hybridization and subsequent washes are carried out under moderate to high stringency conditions that allow for detection of target sequences with homology to sequences exemplified herein. Specific hybridization of a variant under stringent conditions means that the complementary strand required for duplex formation is such that a variant can be distinguished. For double-stranded DNA gene probes, hybridization can be carried out overnight at 20-25° C. below the melting temperature (Tm) of the DNA hybrid in 6 x SSPE, 5 x Denhardt's solution, 0.1% SDS, 0.1 mg/ml denatured DNA. The melting temperature is described by the following formula from Beltz et al. (1983) Methods Enzymol 100, 266-85: Tm=81.5° C.+16.6 Log[Na+] +0.41(% G+C)-0.61(% formamide)-600/length of duplex in base pairs.

Washes can typically be carried out as follows:

(1) Twice at room temperature for 15 minutes in 1.times. SSPE, 0.1% SDS (low stringency wash).
(2) Once at Tm-20° C. for 15 minutes in 0.2.times. SSPE, 0.1% SDS (moderate stringency wash).

For oligonucleotide probes, hybridization is typically carried out overnight at 10-20°C below the melting temperature (Tm) of the hybrid in 6xSSPE, 5xDenhardt's solution, 0.1% SDS, 0.1mg/ml denatured DNA. Tm for oligonucleotide probes was determined by the following formula from Suggs et al. (1981):

$$Tm\ (°C.)=2(\text{number T/A base pairs})+4(\text{number G/C base pairs})$$

In general, salt and/or temperature can be altered to change stringency. With a labeled DNA fragment of greater than about 70 or so bases in length, the following conditions can be used:

1 Low: 1 or 2X SSPE, room temperature Low: 1 or 2X SSPE, 42 °C. Moderate: 0.2X or 1X SSPE, 65° C. High: 0.1X SSPE, 65°C.

Duplex formation and stability depend on substantial complementarity between the two strands of a hybrid, and, as noted above, a certain degree of mismatch can be tolerated. Therefore, polynucleotide sequences described herein include mutations (both single and multiple), deletions, and insertions in the described sequences, and combinations thereof, wherein said mutations, insertions, and deletions permit formation of stable hybrids with a target polynucleotide of interest. Mutations, insertions, and deletions can be produced in a given polynucleotide sequence using standard methods known in the art. Other methods may become known in the future.

The mutational, insertional, and deletional variants of the polypeptide sequences described herein can be used in the same manner as the exemplified polynucleotide sequences so long as the variants have substantial sequence similarity with the original sequence. As used herein, substantial sequence similarity refers to the extent of nucleotide similarity that is sufficient to enable the variant polynucleotide to function in the same capacity as the original sequence (*e.g.*, to encode a polypeptide described herein Preferably, this similarity is greater than 50%; more preferably, this similarity is greater than 75%; and most preferably, this similarity is greater than 90%. The degree of similarity needed for the variant to function in its intended capacity will depend upon the intended use of the sequence. Mutants, variants, or fragments can include modifications to the peptide backbone, added moieties, derivatized functional groups, and fragments can have sizes in the range from the minimal length of about 11 amino acids up to the full-length of 265 amino acids. Active variants of the polypeptide sequences described herein are those variants that function in a manner similar to the original polypeptide with respect to stimulating antigen specific CTLs, in stimulating the production of or reacting With an antibody to the original polynucleotide, or in eliciting protective immunity against an antigen mentioned herein, such as a *T. parva* antigen. It is well within the skill of a person trained in this art to make mutational, insertional, and deletional mutations that are designed to improve the function of the sequence or otherwise provide a methodological advantage.

PCR technology. Polymerase Chain Reaction (PCR) is a repetitive, enzymatic, primed synthesis of a nucleic acid sequence. This procedure is well known and commonly used by those skilled in this art (see U.S. Pat. Nos. 4,683,195, 4,683,202, and 4,800,159; Saiki et al. (1985) Science 230, 1350-4). PCR is based on the enzymatic amplification of a DNA fragment of interest that is flanked by two oligonucleotide primers that hybridize to opposite strands of the target sequence. The primers are oriented with the 3' ends pointing towards each other. Repeated cycles of heat denaturation of the template, annealing of the primers to their complementary sequences, and extension of the annealed primers with a DNA polymerase result in the amplification of the segment defined by the 5' ends of the PCR primers. Since the extension product of each primer can serve as a template for the other primer, each cycle essentially doubles the amount of DNA fragment produced in the previous cycle. This results in the exponential accumulation of the specific target fragment, up to several million-fold in a few hours. By using a thermostable DNA polymerase such as Taq polymerase, which is isolated from the thermophilic bacterium *Thermus aquaticus,* the amplification process can be completely automated. Other enzymes that can be used are known to those skilled in the art.

The polynucleotide sequences described herein (and portions thereof such as conserved regions and portions that serve to distinguish these sequences from previously-known sequences) can be used as, and/or used in the design of, primers for PCR amplification. In performing PCR amplification, a certain degree of mismatch can be tolerated between primer and template. Therefore, mutations, deletions, and insertions (especially additions of nucleotides to the 5' end) of the exemplified polynucleotides can be used in this manner. Mutations, insertions and deletions can be produced in a given primer by methods known to an ordinarily skilled artisan.

Full length genes may be cloned utilizing partial nucleotide sequence and various methods known in the art. Gobinda et al. (1993; PCR Methods Applic 2:318-22) disclose "restriction-site PCR" as a direct method which uses universal primers to retrieve unknown sequence adjacent to a known locus. Inverse PCR can be used to acquire unknown sequences starting with primers based on a known region (Triglia T. et al. (1988) Nucleic Acids Res 16:8186).

CLONTECH PCR-Select™ cDNA Subtraction (Clontech Laboratories, Inc., Palo Alto, Calif.) is yet another means by which differentially expressed genes may be isolated. The procedure allows for the isolation of transcripts present in one mRNA population which is absent, or found in reduced numbers, in a second population of mRNA. Rare transcripts may be enriched 1000-fold.

Polynucleotides described herein can be defined according to several parameters. One characteristic is the biological activity of the protein products as identified herein. The proteins and genes described herein can be further defined by their amino acid and nucleotide sequences. The sequences of the molecules can be defined in terms of homology to certain exemplified sequences as well as in terms of the ability to hybridize with, or be amplified by, certain exemplified probes and primers. Additional primers and probes can readily be constructed by those skilled in the art such that alternate polynucleotide sequences encoding the same amino acid sequences can be used to identify and/or characterize additional genes. The proteins described herein can also be identified based on their immunoreactivity with certain antibodies.

The polynucleotides and proteins described herein include portions, fragments, variants, and mutants of the full-length sequences as well as fusions and chimerics, so long as the encoded protein retains a characteristic biological activity of the proteins identified herein. As used herein, the terms "variants" or "variations" of genes include nucleotide sequences that encode the same proteins or which encode equivalent proteins having equivalent biological activity. As used herein, the term "equivalent proteins" refers to proteins having the same or essentially the same biological activity as the exemplified proteins.

Variations of genes may be readily constructed using standard techniques such as site-directed mutagenesis and other methods of making point mutations and by DNA shuffling, for example. In addition, gene and protein fragments can be made using commercially available exonucleases, endonucleases, and proteases according to standard procedures. For example, enzymes such as Bal31 can be used to systematically cut off nucleotides from the ends of genes. Also, genes that encode fragments may be obtained using a variety of restriction enzymes. Proteases may be used to directly obtain active fragments of these proteins. Of course, molecular techniques for cloning polynucleotides and producing gene constructs of interest are also well known in the art. In vitro evaluation techniques, such as MAXYGENs "Molecular Breeding" can also be applied.

A "selectable marker" is a gene whose expression allows one to identify cells that have been transformed or transfected with a vector containing the marker gene.

"Reporter" molecules are chemical moieties used for labeling a nucleic or amino acid sequence. They include, but are not limited to, radionuclides, enzymes, fluorescent, chemi-huninescent, or chromogenic agents. Reporter molecules associate with, establish the presence of, and may allow quantification of a particular nucleic or amino acid sequence.

A "portion" or "fragment" of a polynucleotide or nucleic acid comprises all or any part of the nucleotide sequence having fewer nucleotides than about 6 kb, preferably fewer than about 1 kb which can be used as a probe. Such probes may be labeled with reporter molecules using nick translation, Klenow fill-in reaction, PCR or other methods well known in the art. After pretesting to optimize reaction conditions and to eliminate false positives, nucleic acid probes may be used in Southern, northern or in situ hybridizations to determine whether target DNA or RNA is present in a biological sample, cell type, tissue, organ or organism.

A "polypeptide," comprises a protein, oligopeptide or peptide fragments thereof. The terms polypeptide, peptide and protein are used interchangeably herein.

A "mutant, variant, or modified polypeptide" includes any polypeptide encoded by a nucleotide sequence that has been mutated through insertions, deletions, substitutions, or the like.

"Chimeric" molecules are polynucleotides or polypeptides which are created by combining one or more nucleotide or peptide sequences (or their parts). In the case of nucleotide sequences, such combined sequences may be introduced into an appropriate vector and expressed to give rise to a chimeric polypeptide which may be expected to be different from the native molecule in one or more of the following characteristics: cellular location, distribution, ligand-binding affinities, interchain affinities, degradation/turnover rate, signaling, etc.

"Active" is that state which is capable of being useful or of carrying out some role. It specifically refers to those forms, fragments, or domains of an amino acid sequence which display a biologic and/or immunogenic activity characteristic of the naturally occurring peptide, polypeptide, or protein. For example, the present description relates to active fragments of polypeptides (*e.g.*, represented by SEQ ID NO: 1-7). Each of these active fragments retains at least one epitope of the larger polypeptide.

"Naturally occurring" refers to a polypeptide produced by cells which have not been genetically engineered or which have been genetically engineered to produce the same sequence as that naturally produced.

"Derivative" refers to those polypeptides which have been chemically modified by such techniques as ubiquitination, labeling, pegylation (derivatization with polyethylene glycol), and chemical insertion or substitution of amino acids such as ornithine which do not normally occur in proteins.

"Recombinant polypeptide variant," refers to any polypeptide which differs from naturally occurring peptide, polypeptide, or protein by amino acid insertions, deletions and/or substitutions.

Amino acid "substitutions" are defined as one for one amino acid replacements. They are conservative in nature when the substituted amino acid has similar structural and/or chemical properties. Examples of conservative replacements are substitution of a leucine with an isoleucine or valine, an aspartate with a glutamate, or a threonine with a serine.

Amino acid "insertions" or "deletions" are changes to or within an amino acid sequence. They typically fall in the range of about 1 to 5 amino acids. The variation allowed in a particular amino acid sequence may be experimentally determined by producing the peptide synthetically or by systematically making-insertions, deletions, or substitutions of nucleotides in the sequence using recombinant DNA techniques.

An "oligopeptide" is a short stretch of amino acid residues and may be expressed from an oligonucleotide. Such sequences comprise a stretch of amino acid residues of at least about 5 amino acids and often about 17 or more amino acids, typically at least about 9 to 13 amino acids, and of sufficient length to display biologic and/or immunogenic activity.

A "standard" is a quantitative or qualitative measurement for comparison. Preferably, it is based on a statistically appropriate number of samples and is created to use as a basis of comparison when performing diagnostic assays, running clinical trials, or following patient treatment profiles. The samples of a particular standard may be normal or similarly abnormal.

An antibody or "specific binding parts" means any antibody molecule of fragment of an antibody molecule that will bind antigen or other ligands such as lectins or other molecules. "Specific binding parts" is meant to include, but not be limited to antibody fragments such as Fab fragments, Fab'(2) fragments, Fc region fragments. Complementarity determining regions (CDRs), Fv fragments, single chain Fv (scFv) fragments, and antigen binding site fragments.

An "antigen" is a macromolecule that is recognized by Antibodies or immune cells and can trigger an immune response. Usually, an antigen is a protein or a polysaccharide, but it can be any type of molecule, even small molecules if coupled to a large carrier.

An "antigen specific" cytotoxic T cell is a T lymphocyte that can recognize and kill another cell that is expressing an antigen, usually in conjunction with a type or class of molecules referred to by those familiar with the art, as Major Histocompatiblity Complex (MHC) Class I molecules. Such Cytotoxic T cells are also referred to as "$CD8^+$" (CD8 positive) or CTLs, by those familiar with the art.

An "epitope" or "antigenic determinant" is a region or section of an antigen that is approximately the minimal length of antigenic sequence that will elicit an immune response, as measured by the development of an antibody response, the development of T antigen-specific T cells, or other measurable immune cell response. For example, an epitope could be a site on an antigen recognized by antibody.

A T cell "epitope" is an antigenic determinant recognized and bound by the T-cell receptor. Epitopes recognized by the T-cell receptor are often located in the inner, unexposed side of the antigen, and become accessible to the T-cell receptors after proteolytic processing of the antigen.

An "effective" immunization protocol is one in which an animal is protected against infection, at least to a measurable degree, when exposed to the specific infectious agent for which it was immunized.

A "boost"or "booster administration", is an administration of an immunogen or antigen that is given to an animal at a time period, usually weeks or months, after a first administration of the same immunogen, in order to stimulate an antigen specific response.

An "effective" condition or state is a those situation in which a biological process may take place. Such a process could be, for example, the state in which an immune response may develop, or a protein could be expressed by a living cell.

A "pharmaceutically acceptable carrier" is an agent or carrier, often, but not limited to, a solute or liquid provided for an immunizing agent in order that it can be administered to an animal. Such a carrier may be water, saline solution, dextrose, glycerols, alcohols, oils, oil-based substances, to which an immunizing agent is added. A pharmaceutically acceptable carrier can also be a solid substrate, such as microbeads that absorb the immunizing agent or a substance that will release the immunizing agent over time, such as a biodegradable implant. A carrier can also be a combination of the above mentioned carriers,' but is not limited to such substances.

An "Elispot Assay" or "elispot assay" is a shorted name for "Enzyme-linked ImmunoSpot Assay", which refers to an antibody based method to detect secretion of soluble factors released by cells. Originally developed as a method to detect antibody-secreting B-cells, later the method was adapted to determine T-cell reaction to a specific antigen, often represented as number of activated cells per million. ,

"Effective conditions" for culturing cells or cell lines refers to those culture conditions that allow the cells to respond in a manner than mimics or is the same as the response would be if the cells were responding in vivo.

"Harvesting" cells or other materials means to separate and collect those cells from a mixture of from one source for use in another protocol.

An "adjuvant" is a substance mixed with an immunizing agent that increases that ability of an animal to respond to the immunizing agent in a manner that the immune response is of a greater degree than if the adjuvant were not present. The immune response is not to the adjuvant, but to the immunizing agent or antigen that is mixed with the adjuvant.

A "protective antibody" is an antibody that, when it is expressed in an animal, puts that animal in a state whereby it will not contract a disease caused by the agent to which the antibody binds.

An "antigen specific" cell, is a cell such as a cytotoxic T cell, that binds only, or substantially primarily, to the specific molecule or antigen for which it expresses receptors, usually as a result of an immune response.

An "immunogenic composition" is a molecule or mixture of molecules that, when administered to an animal, will cause the immune system of that animal to become activated and to produce immune molecules or cells. A composition can also be immunogenic in vitro conditions that are such that they mimic or resemble the conditions that occur in vitro in an animal.

To "stimulate" T cells or other cells of the immune system, means that the T cells or other cells of the immune system of an organism, react to a stimulus in a measurable way, such as by the secretion of a molecule or hormone, may become activated and kill or lyse other cells, or by undergoing a change of cell cycle state or the like.

A "Bovine MHC class II bioassay", means an assay that measures the presence of a T cell-derived soluble factor, such as interferon (IFN-gamma) release from T cells responding to specific stimulation through the ability of IFN-gamma to induce and up-regulate expression of class II molecules on bovine endothelial cells. Bovine endothelial cells do not constitutively express class II molecules unless triggered by external signals such as IFN-gamma.

A "vaccine," is a composition that, when administered to an animal, causes the immune system of the animal to produce antibody molecules or immune cells, such as antigen-specific T cells, that react with an antigenic substance that is in the composition and render that animal resistant to infection or other pathology that is caused by an organism bearing or producing the antigenic substance.

A "pharmaceutical composition" is a material that has a measurable effect on an animal to which it is administered. This effect is of a nature that is normally thought of as being good or desirable for the animal, such as a healing or protective effect. A pharmaceutical composition may be a therapeutic composition, or a composition used for diagnostic purposes.

Since the list of technical and scientific terms cannot be all encompassing, any undefined terms shall be construed to have the same meaning as is commonly understood by one of skill in the art. Furthermore, the singular forms "a", "an" and "the" include plural referents unless the context clearly dictates otherwise.

The description is not to be limited only to the particular sequences, variants, formulations or methods described. The sequences, variants, formulations and methodologies may vary, and the terminology used herein is for the purpose of describing particular embodiments. The terminology and definitions are not intended to be limiting.

Because of the redundancy of the genetic code, a variety of different DNA sequences can encode the amino acid sequences encoded by the polynucleotide sequences disclosed herein. It is well within the skill of a person trained in the art to create these alternative DNA sequences encoding proteins having the same, or essentially the same, amino acid sequence. These variant DNA sequences are within the scope of the description. As used herein, reference to "essentially the same" sequence refers to sequences that have amino acid substitutions, deletions, additions, or insertions that do not materially affect biological activity. Fragments retaining the characteristic biological activity are also included in this definition.

The description comprises variant or equivalent proteins (and nucleotide sequences coding for equivalent proteins) having the same or similar biological activity of proteins encoded by the exemplified polynucleotides. Equivalent proteins will have amino acid similarity with an exemplified protein (or peptide). The amino acid identity is typically greater than about 60%. Preferably, the amino acid identity will be greater than 75%. Morse preferably, the amino acid identity is greater than about 80%, and even more preferably greater than about 90%. Most preferably, amino acid identity is greater than about 95%. (Likewise, the polynucleotides that encode the subject polypeptides will also have corresponding identities in these preferred ranges.) These identities are as determined using standard alignment techniques for determining amino acid identity. The amino acid identity/similarity/homology will be highest in critical regions of the protein including those regions that account for biological activity or that are involved in the determination of three-dimensional configuration that is ultimately responsible for the biological activity. In this regard, certain amino acid substitutions are acceptable and can be expected if these substitutions are in regions which are not critical to activity or are conservative amino acid substitutions which do not affect the three-dimensional configuration of the molecule. For example, amino acids may be placed in the following classes: non-polar, uncharged polar, basic, and acidic. Conservative substitutions whereby an amino acid of one class is replaced with another amino acid of the same type fall within the scope of the description so long as the substitution does not materially alter the biological activity of the compound. Below is a list of examples of amino acids belonging to various classes:

Nonpolar - Ala, Val, Leu, Ile, Pro, Met, Phe, Trp, uncharged polar Gly, Ser, Thr, Cys, Tyr, Asn, Gln
Acidic - Asp, Glu Basic Lys, His

In some instances, non-conservative substitutions can also be made.
As used herein, reference to "isolated" polynucleotides and/or "purified" proteins refers to these molecules when they are not associated with the other molecules with which they would be found in nature. Thus, reference to "isolated" and/or "purified" signifies the involvement of the "hand of man" as described herein. Reference to "heterologous" proteins, genes, and gene constructs, also signifies the involvement of the "hand of man."
There is also provided vectors containing the nucleic acid molecules described herein. The term "vector" refers to a vehicle, preferably a nucleic acid molecule, which can transport the nucleic acid molecules. When the vector is a nucleic acid molecule, the nucleic acid molecules are covalently linked to the vector nucleic acid. With this aspect, the vector includes a plasmid, single or double stranded phage, a single or double stranded RNA or DNA viral vector, or artificial chromosomes, such as a BAC, PAC, YAC, OR MAC.
A vector can be maintained in the host cell as an extrachromosomal element where it replicates and produces additional copies of the nucleic acid molecules. Alternatively, the vector may integrate into the host cell genome and produce additional copies of the nucleic acid molecules when the host cell replicates.
There is provided vectors for the maintenance (cloning vectors) or vectors for expression (expression vectors) of the nucleic acid molecules. The vectors can function in prokaryotic or eukaryotic cells or in both (shuttle vectors).
Expression vectors contain cis-acting regulatory regions (expression control sequences) that are operably linked in the vector to the nucleic acid molecules such that transcription of the nucleic acid molecules is allowed in a host cell. The nucleic acid molecules can be introduced into the host cell with a separate nucleic acid molecule capable of affecting transcription. Thus, the second nucleic acid molecule may provide a trans-acting factor interacting with the cis-regulatory control region to allow transcription of the nucleic acid molecules from the vector. Alternatively, a trans-acting factor may be supplied by the host cell. Finally, a trans-acting factor can be produced from the vector itself. It is understood, however, that in some embodiments, transcription and/or translation of the nucleic acid molecules can occur in a cell-free system.
The regulatory sequence to which the nucleic acid molecules described herein can be operably linked include promoters for directing mRNA transcription. These include, but are not limited to, the left promoter from bacteriophage lambda., the lac, TRP, and TAC promoters from E. Coli, the early and late promoters from SV40, the CMV immediate early promoter, the adenovirus early and late promoters, and retrovirus long-terminal repeats.
In addition to control regions that promote transcription, expression vectors may also include regions that modulate transcription, such as repressor binding sites and enhances. Examples include the SV40 enhancer, the cytomegalovirus immediate early enhancer, polyoma enhancer, adenovirus enhancers, and retrovirus LTR enhancers.
In addition to containing sites for transcription initiation and control, expression vectors can also contain sequences necessary for transcription termination and, in the transcribed region a ribosome binding site for translation. Other regulatory control elements for expression include initiation and termination codons as well as polyadenylation signals. The person of ordinary skill in the art would be aware of the numerous regulatory sequences that are useful in expression vectors. Such regulatory sequences are described, for example, in Sambrook et al., Molecular Cloning: A Laboratory Manual. 2nd. ed., Cold Spring Harbor Laboratory Press, Cold Spring Harbor, N.Y., (1989).
A variety of expression vectors can be used to express a nucleic acid molecule. Such vectors include chromosomal, episomal, and virus-derived vectors, for example vectors derived from bacterial plasmids, from bacteriophage, from yeast episomes, from yeast chromosomal elements, including yeast artificial chromosomes, from viruses such as baculoviruses, papovaviruses such as SV40, Vaccinia viruses including the modified vaccinia virus Ankara strain (MVA), adenoviruses, poxviruses including fowlpox virus (FP9) and canary pox, pseudorabies viruses, and retroviruses. Vectors may also be derived from combinations of these sources such as those derived from plasmid and bacteriophage genetic elements, e.g. cosmids and phagemids. Appropriate cloning and expression vectors for prokaryotic and eukaryotic hosts are described in Sambrook et al., Molecular Cloning: A Laboratory Manual. 2nd. ed., Cold Spring Harbor Laboratory Press, Cold Spring Harbor, N.Y., (1989).
The regulatory sequence may provide constitutive expression in one or more host cells (i.e. tissue specific) or may provide for inducible expression in one or more cell types such as by temperature, nutrient additive, or exogenous factor such as a hormone or other ligand. A variety of vectors providing for constitutive and inducible expression in prokaryotic and eukaryotic hosts are well known to those of ordinary skill in the art.
The nucleic acid molecules can be inserted into the vector nucleic acid by well-known methodology. Generally, the DNA sequence that will ultimately be expressed is joined to an expression vector by cleaving the DNA sequence and the expression vector with one or more restriction enzymes and then ligating the fragments together. Procedures for restriction enzyme digestion and ligation are well known to those of ordinary skill in the art.

The vector containing the appropriate nucleic acid molecule can be introduced into an appropriate host cell for propagation or expression using well-known techniques. Bacterial cells include, but are not limited to; *E. coli, Streptomyces sp.,* and *Salmonella typhimurium.* Eukaryotic cells include, but are not limited to, yeast, insect cells such as Drosophila, animal cells such as COS and CHO cells, fibroblast, skin fibroblast, immortalized skin fibroblast and plant cells.

As described herein, it may be desirable to express the peptide as a fusion protein. Accordingly, the description provides fusion vectors that allow for the production of the peptides. Fusion vectors can increase the expression of a recombinant protein, increase the solubility of the recombinant protein, and aid in the purification of the protein by acting for example as a ligand for affinity purification. A proteolytic cleavage site may be introduced at the junction of the fusion moiety so that the desired peptide can ultimately be separated from the fusion moiety. Proteolytic enzymes include, but are not limited to, factor Xa, thrombin, and enteroenzyme. Typical fusion expression vectors include pGEX (Smith et al., Gene 67:31-40 (1988)), pMAL (New England Biolabs, Beverly, Mass.) and pRIT5 (Pharmacia, Piscataway, N.J.) which fuse glutathione S-transferase (GST), maltose E binding protein, or protein A, respectively, to the target recombinant protein. Examples of suitable inducible non-fusion *E. coli* expression vectors include pTrc (Amann et al, Gene 69:301-315 (1988)) and pET 11d (Studier et al, Gene Expression Technology: Methods in Enzymology 185:60-89 (1990)).

Recombinant protein expression can be maximized in host bacteria by providing a genetic background wherein the host cell has an impaired capacity to proteolytically cleave the recombinant protein. (Gottesman, S., Gene Expression Technology: Methods in Enzymology 185, Academic Press, San Diego, Calif. (1990) 119-128). Alternatively, the sequence of the nucleic acid molecule of interest can be altered to provide preferential codon usage for a specific host cell, for example E. coli. (Wada et al., Nucleic Acids Res. 20:2111-2118 (1992)).

The nucleic acid molecules can also be expressed by expression vectors that are operative in yeast. Examples of vectors for expression in yeast e.g., *S. cerevisiae* include pYepSecl (Baldari, et al, EMBO J. 6:229-234 (1987)), pMFa (Kujan et al, Cell 30:933-943(1982)), pJRY88 (Schultz et al, Gene 54:113-123 (1987)), and pYES2 (Invitrogen Corporation, San Diego, Calf.).

The nucleic acid molecules can also be expressed in insect cells using, for example, baculovirus expression vectors. Baculovirus vectors available for expression of proteins in cultured insect cells (e.g., Sf9 cells) include the pAc series (Smith et al, Mol. Cell Biol. 3:2156-2165 (1983)) and the pVL series (Lucklow et al. Virology 170:31-39 (1989)).

In certain embodiments, the nucleic acid molecules described herein are expressed in mammalian cells using mammalian expression vectors. Examples of mammalian expression vectors include pCDM8 (Seed, B. Nature 329:840 (1987)) and pMT2PC (Kaufman et al., EMBO J. 6:187-195 (1987)).

The expression vectors listed herein are provided by way of example only of the well-known vectors available to those of ordinary skill in the art that would be useful to express the nucleic acid molecules. The person of ordinary skill in the art would be aware of other vectors suitable for maintenance propagation or expression of the nucleic acid molecules described herein. These are found for example in Sambrook, J., Fritsh, E. F., and Maniatis, T. Molecular Cloning: A Laboratory Manual. 2nd, ed., Cold Spring Harbor Laboratory, Cold Spring Harbor Laboratory Press, Cold Spring Harbor, N.Y., 1989.

The description also encompasses vectors in which the nucleic acid sequences described herein are cloned into the vector in reverse orientation, but operably linked to a regulatory sequence that permits transcription of antisense RNA. Thus, an antisense transcript can be produced to all, or to a portion, of the nucleic acid molecule sequences described herein, including both coding and non-coding regions. Expression of this antisense RNA is subject to each of the parameters described above in relation to expression of the sense RNA (regulatory sequences, constitutive or inducible expression, tissue-specific expression).

The description also relates to recombinant host cells containing the vectors described herein. Host cells therefore include prokaryotic cells, lower eukaryotic cells such as yeast, other eukaryotic cells such as insect cells, and higher eukaryotic cells such as mammalian cells.

The recombinant host cells are prepared by introducing the vector constructs described herein into the cells by techniques readily available to the person of ordinary skill in the art. These include, but are not limited to, calcium phosphate transfection, DEAE-dextran-mediated transfection, cationic lipid-mediated transfection, electroporation, transduction, infection, lipofection, and other techniques such as those found in Sambrook, et al. (Molecular Cloning: A Laboratory Manual. 2nd, ed., Cold Spring Harbor Laboratory, Cold Spring Harbor Laboratory Press, Cold Spring Harbor, N.Y., 1989).

Host cells can contain more than one vector. Thus, different nucleotide sequences can be introduced on different vectors of the same cell. Similarly, the nucleic acid molecules can be introduced either alone or with other nucleic acid molecules that are not related to the nucleic acid molecules such as those providing trans-acting factors for expression vectors. When more than one vector is introduced into a cell, the vectors can be introduced independently, co-introduced or joined to the nucleic acid molecule vector.

In the case of bacteriophage and viral vectors, these can be introduced into cells as packaged or encapsulated virus by standard procedures for infection and transduction. Viral vectors can be replication-competent or replication-defective. In the case in which viral replication is defective, replication will occur in host cells providing functions that complement the defects.

Vectors generally include selectable markers that enable the selection of the subpopulation of cells that contain the recombinant vector constructs. The marker can be contained in the same vector that contains the nucleic acid molecules described herein or may be on a separate vector. Markers include tetracycline or ampicillin-resistance genes for prokaryotic host cells and dihydrofolate reductase or neomycin resistance for eukaryotic host cells. However, any marker that provides selection for a phenotypic trait will be effective.

While the mature proteins can be produced in bacteria, yeast, mammalian cells, and other cells under the control of the appropriate regulatory sequences, cell- free transcription and translation systems can also be used to produce these proteins using RNA derived from the DNA constructs described herein.

Where secretion of the peptide is desired, which is difficult to achieve with multi-transmembrane domain containing proteins such as MHC Class I-binding peptides, appropriate secretion signals are incorporated into the vector. The signal sequence can be endogenous to the peptides or heterologous to these peptides.

The expressed protein can be isolated from the host cell by standard disruption procedures, including freeze thaw, sonication, mechanical disruption, use of lysing agents and the like. The peptide can then be recovered and purified by well-known purification methods including ammonium sulfate precipitation, acid extraction, anion or cationic exchange chromatography, phosphocellulose chromatography, hydrophobic-interaction chromatography, affinity chromatography, hydroxylapatite chromatography, lectin chromatography, or high performance liquid chromatography.

It is also understood that depending upon the host cell in recombinant production of the peptides described herein, the peptides can have various glycosylation patterns, depending upon the cell, or maybe non-glycosylated as when produced in bacteria. In addition, the peptides may include an initial modified methionine in some cases as a result of a host-mediated process.

There are many methods for introducing a heterologous gene or polynucleotide into a host cell or cells under conditions that allow for stable maintenance and expression of the gene or polynucleotide. These methods are well known to those skilled in the art. Synthetic genes, such as, for example, those genes modified to enhance expression in a heterologous host (such as by preferred codon usage or by the use of adjoining, downstream, or upstream enhancers) that are functionally equivalent to the genes (and which encode equivalent proteins) can also be used to transfect hosts. Methods for the production of synthetic genes are known in the art. Recombinant, hosts can be used for the expression or propagation of genes and polynucleotide described herein. The genes and polynucleotides within the scope of the description can be introduced into a wide variety of microbial or plant hosts, such as bacterial cells, yeast, insect cells, plant cell cultures or plants, mammalian cells. For example, *T. parva* nucleic acids can be expressed in a recombinant baculovirus (BV) possessing an optimized promoter and translation initiation region operably linked to the *T. parva* nucleic acid. In a preferred embodiment, an optimized promoters and translation initiation region are operably linked to the *T. parva* nucleic acid. In one embodiment, insect host cells can be transformed with baculovirus expressing *T. parva* nucleic acids. In still another embodiment, Tn5 (Trichoplusiani or High Five™) host cells can be transformed with baculovirus expressing *T. parva* nucleic acids.

*T. parva* "recombinant protein", is a protein made using recombinant techniques, i.e. through the expression of a recombinant nucleic acid as depicted above. A recombinant protein is distinguished from naturally occurring protein by at least one or more characteristics. For example, the protein may be isolated or purified away from some or all of the proteins and compounds with which it is normally associated in its wild type host, and thus may be substantially pure. For example, an isolated protein is unaccompanied by at least some of the material with which it is normally associated in its natural state, preferably constituting at least about 0.5%, more preferably at least about 5% by weight of the total protein in a given sample. A substantially pure protein comprises at least about 75% by weight of the total protein, with at least about 80% being preferred, and at least about 90% being particularly preferred. The definition includes the production of a protein from one organism in a different organism or host cell. Alternatively, the protein may be made at a significantly higher concentration than is normally seen, through the use of an inducible promoter or high expression promoter, such that the protein is made at increased concentration levels. Alternatively, the protein may be in a form not normally found in nature, as in the addition of an epitope tag or amino acid substitutions, insertions and/or deletions, as discussed below.

Included in "*T. parva* antigen polypeptides" are *T. parva* polypeptide variants. These variants fall into one or more of three classes: substitutional, insertional or deletional variants. These variants ordinarily are prepared by site specific mutagenesis of nucleotides in the DNA encoding a *T. parva* antigen polypeptide, using cassette or PCR mutagenesis, scanning mutagenesis, gene shuffling or other techniques well known in the art, to produce DNA encoding the variant, and thereafter expressing the DNA in recombinant cell culture as outlined above. However, variant *T. parva* polypeptide fragments having up to about 100-150 residues may be prepared by in vitro synthesis using established techniques. Amino acid sequence variants are characterized by the predetermined nature of the variation, a feature that sets them apart from naturally occurring allelic or interspecies variation of the *T. parva* antigen polypeptide amino acid sequence.

While the site or region for introducing an amino acid sequence variation is predetermined, the mutation per se need not be predetermined. For example, in order to optimize the performance of a mutation at a given site, random mutagenesis may be conducted at the target codon or region and the expressed *T. parva* antigen polypeptide variants can be screened for the optimal combination of desired activity. Techniques for making mutations at predetermined sites in DNA having a known sequence are well known. For example, the variations can be made using oligonucleotide-mediated site-directed mutagenesis, [Carter et al., Nucl. Acids Res., 13:4331 (1986); Zoller et at., Nucl. Acids Res., 10: 6487 (1987)], cassette mutagenesis [Wells et al., Gene, 34:315 (1985)], restriction selection mutagenesis [Wells et al., Philos. Trans. R. Soc. London SerA, 317:415 (1986)] PCR mutagenesis, or other known techniques can be performed on the cloned DNA to produce *T. parva* antigen polypeptide variant DNA. Scanning amino acid analysis can also be employed to identify one or more amino acids along a contiguous sequence. Among the preferred scanning amino acids are relatively small, neutral amino acids. Such amino acids include alanine, glycine, serine, and cysteine. Alanine is typically a preferred scanning amino acid among this group because it eliminates the side-chain beyond the beta-carbon and is less likely to alter the main-chain confirmation of the variant [Cunningham and Wells, Science, 244: 1081-1085 (1989)]. Alanine is also typically preferred because it is the most common amino acid. Further, it is frequently found in both buried and exposed positions [Creighton, The Proteins, (W.H. Freeman & Co., N.Y.); Chothia, J. Mol. Biol; 150:1 (1976)]. If alanine substitution does not yield adequate amounts of variant, an isoteric amino acid can be used. Screening of the mutants or variants is done using elispot and/or bioassays of *T. parva* antigen Polypeptide activities and/or properties as described herein.

The description further provides fragments of the antigen peptides, in addition to proteins and peptides that comprise and consist of such fragments, particularly those comprising the residues identified in SEQ ID NO: 4-7. The fragments to which the invention pertains, however, are not to be construed as encompassing fragments that may be disclosed publicly prior to the present invention.

As used herein, a fragment comprises at least about 8, 10, 12, 14, 16, or more contiguous amino acid residues from an antigen peptide. Such fragments can be chosen based on the ability to retain one or more of the biological activities of the antigen peptide or could be chosen for the ability to perform a function, e.g. bind a substrate or act as an immunogen. Particularly important fragments are biologically active fragments, peptides that are, for example, about 8 or more amino acids in length. Such fragments will typically comprise a domain or motif of the antigen peptide, e.g., active site, a transmembrane domain or a substrate-binding domain. Further, possible fragments include, but are not limited to, domain or motif containing fragments, soluble peptide fragments, and fragments containing immunogenic structures. Predicted domains and functional sites are readily identifiable by computer programs well known and readily available to those of skill in the art (e.g., PROSITE analysis).

The software, "Glimmer" ver 1.0 and 2.0 (Gene Locator and Interpolated Markov Modeler), is used to find genes in DNA, using interpolated Markov models (IMMs) to identify the coding regions and distinguish them from noncoding DNA. The IMM approach, described in Salzburg et al., 1998, NAR 26:544, and Delcher et al, 1999, NAR 27:4636 use a combination of Markov models from 1st through 8th-order, weighting each model according to its predictive power. Glimmer 1.0 and 2.0 use 3-periodic nonhomogenous Markov models in their IMMs. Glimmer and other software applications that use IMM such as GlimmerM and GlimmerHMM, (Pertea, M., and Salzberg, S.L. Current Protocols in Bioinformatics, 2002.) have been used to find the genes in chromosome 2 of the malaria parasite, *P. falciparum*. GlimmerM is described in S.L. Salzberg, M. Pertea, A.L. Delcher, M.J. Gardner, and H. Tettelin, "Interpolated Markov models for eukaryotic gene finding," Genomics 59 (1999), 24-31. The Glimmer system consists of two main programs. The first of these is the training program, *build-imm* that takes an input set of sequences and builds and outputs the IMM for them. These sequences can be complete genes or just partial orfs. For a new genome, this training data can consist of those genes with strong database hits as well as very long open reading frames that are statistically almost certain to be genes. The second program is *glimmer*, which uses this IMM to identify putative genes in an entire genome. A Perl program is available that will use Glimmer's predictions as to call the BLAST program and search any locally-installed protein database.

"Combiner" (J. E. Allen, M. Pertea, S. L. Salzberg, Genome Research, 14(1), 2004. is a program that uses the output from gene finders, splice site prediction programs and sequence alignments to predict gene models. The program provides an automated way to take advantage of the many successful methods for computational gene prediction and can provide substantial improvements in accuracy over an individual gene prediction program. These software applications can incorporate information that will allow one skilled in the art to identify sequences that are candidates for encoding polypeptides of interest.

Polypeptides often contain amino acids other than the 20 amino acids commonly referred to as the 20 naturally occurring amino acids. Further, many amino acids, including the terminal amino acids, may be modified by natural processes, such as processing and other post-translational modifications, or by chemical modification techniques well known in the art. Common modifications that occur naturally in antigen peptides are described in basic texts, detailed monographs, and the research literature, and they are well known to those of skill in the art.

Known modifications include, but are not limited to, acetylation, acylation, ADP-ribosylation, amidation, covalent attachment of flavin, covalent attachment of a heme moiety, covalent attachment of a nucleotide or nucleotide derivative, covalent attachment of a lipid or lipid derivative, covalent attachment of phosphotidylinositol, cross-linking, cyclization, disulfide bond formation, demethylation, formation of covalent crosslinks, formation of cystine, formation of pyroglutamate, formylation, gamma carboxylation, glycosylation, GPI anchor formation, hydroxylation, iodination, methylation, myristoylation, oxidation, proteolytic processing, phosphorylation, prenylation, racemization, selenoylation, sulfation, transfer-RNA mediated addition of amino acids to proteins such as arginylation, and ubiquitination.

Such modifications are well known to those of skill in the art and have been described in great detail in the scientific literature. Several particularly common modifications, glycosylation, lipid attachment, sulfation, gamma-carboxylation of glutamic acid residues, hydroxylation and ADP-nbosylation, for instance, are described in most basic texts, such as Proteins-Structure and Molecular Properties, 2-sup.nd Ed., T. E. Creighton, W. H. Freeman and Company, New York (1993). Many detailed reviews are available on this subject, such as by Wold, F., Posttranslational Covalent Modification of Proteins, B. C. Johnson, Ed., Academic Press, New York 1-12 (1983); Seifter et al. (Meth. Enzymol. 182: 626-646 (1990)) and Rattan et al. (Ann. N.Y Acad. Sci. 663:48-6,2 (1992)).

Accordingly, the antigen peptides mentioned herein also encompass derivatives or analogs in which a substituted amino acid residue is not one encoded by the genetic code, in which a substituent group is included, in which the mature antigen peptide is fused with another compound, such as a compound to increase the half-life of the antigen peptide (for example, polyethylene glycol), or in which the additional amino acids are fused to the mature antigen peptide, such as a leader or secretory sequence or a sequence for purification of the mature antigen peptide or a pro-protein sequence.

Although an amino acid sequence or oligopeptide used for antibody induction does not require biological activity, it must be immunogenic. A peptide, polypeptide, or protein used to induce specific antibodies may have an amino acid sequence consisting of at least five amino acids and preferably at least 10 amino acids. Short stretches of amino acid sequence may be genetically or chemically fused with those of another protein such as keyhole limpet hemocyanin, and the chimeric peptide used for antibody production. Alternatively, the oligopeptide may be of sufficient length to contain an entire domain.

Antibodies specific for peptides, polypeptides, or proteins may be produced by inoculation of an appropriate non-human animal with an antigenic fragment of the peptide, polypeptide, or protein. Antibody production includes not only the stimulation of an immune response by injection into non-human animals, but also analogous processes such as the production of synthetic antibodies, the screening of recombinant immunoglobulin libraries for specific-binding molecules (Orlandi R. et al. [1989] PNAS 86:3833-3837, or Huse W. D. et al. [1989] Science 256:1275-1281), or the in vitro stimulation of lymphocyte populations. Current technology (Winter G. and Milstein C. [1991] Nature 349:293-299) provides for a number of highly. specific binding reagents based on the principles of antibody formation. These techniques may be adapted to produce molecules which specifically bind antigen peptides. Antibodies or other appropriate molecules generated against a specific immunogenic peptide fragment or oligopeptide can be used in Western analysis, enzyme-linked immunosorbent assays (ELISA) or similar tests to establish the presence of or to quantitate amounts of peptide, polypeptide, or protein in normal, diseased, or transformed cells, tissues, organs, or organisms as well as liquid suspensions containing said peptide, polypeptide, or protein.

The description also provides for antibodies that selectively bind to one of the peptides described herein, a proteins comprising such a peptide, as well as variants and fragments thereof. As used herein, an antibody selectively binds a target peptide when it binds the target peptide and does not significantly bind to unrelated proteins. An antibody is still considered to selectively bind a peptide even if it also binds to other proteins that are not substantially homologous with the target peptide so long as such proteins share homology with a fragment or domain of the peptide target of the antibody. In this case, it would be understood that antibody binding to the peptide is still selective despite some degree of cross-reactivity.

As used herein, an antibody is defined in terms consistent with that recognized within the art: they are multi-subunit proteins produced by a mammalian organism in response to an antigen challenge. The antibodies mentioned herein include polyclonal antibodies and monoclonal antibodies, as well as fragments of such antibodies, including, but not limited to, Fab or $F(ab')_2$, and Fv fragments.

Many methods are known for generating and/or identifying antibodies to a given target peptide. Several such methods are described by Harlow, Antibodies, Cold Spring Harbor Press, (1989).

In general, to generate antibodies, an isolated peptide is used as an immunogen and is administered to a mammalian organism, such as a rat, rabbit or mouse. The full-length protein, an antigenic peptide fragment or a fusion protein can be used. Particularly important fragments are those covering functional domains, such as the domains identified in the figures, and domain of sequence homology or divergence amongst the family, such as those that can readily be identified using protein alignment methods and as presented in the Figures.

Antibodies are preferably prepared from regions or discrete fragments of the antigen proteins. Antibodies can be prepared from any region of the peptide as described herein. However, preferred regions will include those involved in function/activity and/or antigen/binding partner interaction.

An antigenic fragment will typically comprise at least 8 contiguous amino acid residues. The antigenic peptide can comprise, however, at least 10, 12, 14, 16 or more amino acid residue. Such fragments can be selected on a physical property, such as fragments correspond to regions that are located on the surface of the protein, e.g., hydrophilic regions or can be selected based on sequence uniqueness. '

Detection on an antibody or peptide described herein can be facilitated by coupling (i.e., physically linking) the antibody or peptide to a detectable substance. Examples of detectable substances include various enzymes, prosthetic groups, fluorescent materials, luminescent materials, bioluminescent materials, and radioactive materials. Examples of suitable enzymes include horseradish peroxidase, alkaline phosphatase, beta-galactosidase, or acetylcholinesterase; examples of suitable prosthetic group complexes include streptavidin/biotin and avidin/biotin; examples of suitable fluorescent materials include umbelliferone, fluorescein, fluorescein isothiocyanate, rhodamine, dichlorotriazinylamine fluorescein, dansyl chloride or phycoerythrin, an example of a luminescent material includes luminol; examples of bioluminescent materials include luciferase, luciferin, and aequorin, and examples of suitable radioactive material include $^{125}I$,$^{131}I$,$^{35}S$ or $^{3}H$.

The antibodies can be used to isolate one of the proteins described herein by standard techniques, such as affinity chromatography or immunoprecipitation. The antibodies can facilitate the purification of the natural protein from cells and recombinantly produced protein expressed in host cells. In addition, such antibodies are useful to detect the presence of one of the proteins described herein in cells or tissues to determine the pattern of expression of the protein among various tissues in an organism and over the course of schizont development. Such antibodies can be used to detect protein in situ, in vitro, or in a cell lysate or supernatant in order to evaluate the abundance and pattern of expression. Also, such antibodies can be used to assess abnormal tissue distribution or abnormal expression during development or progression of a protozoan infection. Antibody detection of circulating fragments of the full length protein can be used to identify turnover.

Further, the antibodies can be used to assess expression in disease states such as in active stages of the disease or in lymphocytes harvested from infected animals. Experimental data has shown expression of *T. parva* antigens, using antibodies directed against such antigens, in bovine lymphocytes, infected with *T. parva.* If a disease is characterized by a specific mutation in the protein, antibodies specific for this mutant protein can be used to assay for the presence of the specific mutant protein.

The diagnostic uses can be applied, not only in genetic testing, but also in monitoring a treatment modality. Accordingly, where treatment is ultimately aimed at preventing or controlling infection; antibodies directed against the protein or relevant fragments can be used to monitor therapeutic efficacy.

The antibodies are also useful for inhibiting protein function, for example, blocking the binding of the antigenic peptide to a binding partner such as a substrate. These uses can also be applied in a therapeutic context in which treatment involves inhibiting the protein's function. An antibody can be used, for example, to block binding, thus modulating (agonizing or antagonizing) the peptides activity. Antibodies can be prepared against specific fragments containing sites required for function or against intact protein that is associated with a cell or cell membrane. See SEQ ID NO: 1-7 for structural information relating to the proteins described herein

The description also encompasses kits for using antibodies to detect the presence of a protein in a biological sample. The kit can comprise antibodies such as a labeled or labelable antibody and a compound or agent for detecting protein in a biological sample; means for determining the amount of protein in the sample; means for comparing the amount of protein in the sample with a standard; and instructions for use. Such a kit can be supplied to detect a single protein or epitope or can be configured to detect one of a multitude of epitopes, such as in an antibody detection array. Arrays are described in detail below for nucleic acid arrays and similar methods have been developed for antibody arrays.

The proteins described herein can be used in assays related to the functional information provided in the Figures; for example, to stimulate CD8+ cytotoxic T cell lines, to raise antibodies or to elicit another immune response; as a reagent (including the labeled reagent) in assays designed to quantitatively determine levels of the protein (or its binding partner or ligand) in biological fluids; and as markers for tissues in which the corresponding protein is preferentially expressed (either constitutively or at a particular stage of tissue differentiation or development or in a disease state). Where the protein binds or potentially binds to another protein or ligand (such as, for example, in an enzyme-effector protein interaction or enzyme-ligand interaction), the protein can be used to identify the binding partner/ligand so as to develop a system to identify inhibitors of the binding interaction. Any or all of these uses are capable of being developed into reagent grade or kit format for commercialization as commercial products.

Methods for performing the uses listed above are well known to those skilled in the art. References disclosing such methods include "Molecular Cloning: A Laboratory Manual", 2d ed., Cold Spring Harbor Laboratory Press, Sambrook, J., E. F. Fritsch and T. Maniatis eds., 1989, and "Methods in Enzymology. Guide to Molecular Cloning Techniques", Academic Press, Berger, S. L. and A. R. Kimmel eds., 1997.

The potential uses of the peptides described herein are based primarily on the source of the protein as well as the class/action of the protein. For example, antigens isolated from *T. parva* and their protozoan orthologs serve as targets for identifying agents for use in mammalian therapeutic applications, e.g. an animal drug, particularly in modulating a biological or pathological response in a cell, tissue, or protozoan that expresses an immunogenic antigen. Experimental data has shown that the antigens described herein are expressed in infected host lymphocytes, as indicated by flow cytometric analysis. Such uses can readily be determined using the information provided herein, that which is known in the art, and routine experimentation.

Lymphoblastosis is a condition that describes the multiplication or the proliferation of lymphocytes. Often lymphoblastosis occurs when certain lineages of lymphocytes, such as those in the T-cell mediated immunological pathways, are stimulated by an antigen. At other times, lymphoblastosis can be the result of pathological processes such as viral or other pathogenic infection or as a result of tumorgenesis.

To perform assays mentioned herein, such as assays to detect a pathogen, *e.g.*, by detecting the presence and/or expression of a polypeptide described herein in the pathogen, it is sometimes desirable to immobilize either the antigen protein, or fragment, or its target molecule to facilitate separation of complexes from uncomplexed forms of one or both of the proteins, as well as to accommodate automation of the assay.

Techniques for immobilizing proteins on matrices can be used in assays mentioned herein. In one embodiment, a fusion protein can be provided which adds a domain that allows the protein to be bound to a matrix. For example, glutathione-S-transferase fusion proteins can be adsorbed onto glutathione sepharose beads (Sigma Chemical, St. Louis, Mo.) or glutathione derivatized microtitre plates, which are then combined with the cell lysates (e.g., $^{35}S$-labeled) and the candidate compound, and the mixture incubated under conditions conducive to complex formation (e.g., at physiological conditions for salt and pH). Following incubation, the beads are washed to remove any unbound label, and the matrix immobilized and radiolabel determined directly, or in the supernatant after the complexes are dissociated. Alternatively, the complexes can be dissociated from the matrix, separated by SDS-PAGE, and the level of antigen-binding protein found in the bead fraction quantitated from the gel using standard electrophoretic techniques. For example, either the polypeptide or its target molecule can be immobilized utilizing conjugation of biotin and streptavidin using techniques well known in the art. Alternatively, antibodies reactive with the protein but which do not interfere with binding of the protein to its target molecule can be derivatized to the wells of the plate, and the protein trapped in the wells by antibody conjugation. Preparations of an antigen-binding protein and a candidate compound are incubated in the antigen protein-presenting wells and the amount of complex trapped in the well can be quantitated. Methods for detecting such complexes, in addition to those described above for the GST-immobilized complexes, include immunodetection, of complexes using antibodies reactive with the antigen protein target molecule, or which are reactive with antigen protein and compete with the target molecule, as well as enzyme-linked assays which rely on detecting an enzymatic activity associated with the presence of the target molecule.

The antigen proteins mentioned herein are also useful to provide a target for diagnosing a disease or a disease mediated by the peptide. Accordingly, the description provides methods for detecting the presence, or levels of, the protein (or encoding mRNA) in a cell, tissue, or organism. Experimental data has shown expression in *T. parva*-infected bovine lymphocytes. The method involves contacting a biological sample with a compound capable of interacting with the antigen protein such that the interaction can be detected. Such an assay can be provided in a single detection format or a multi-detection format such as an antibody chip array.

One agent for detecting a protein in a sample is an antibody capable of selectively binding to protein. A biological sample includes tissues, cells and biological fluids isolated from a subject, as well as tissue, cells and fluids present within a subject.

Another aspect mentioned herein is a kit for purifying *T. parva* from a sample containing *T. parva,* comprising an antibody mentioned herein. The kit may further comprise means for performing an enzyme-linked or Western blot assay to detect the presence of *T. parva*; and/or means for binding the antibody to *T. parva* in the sample, and for releasing the organism from the antibody.

The peptides described herein also provide targets for diagnosing active parasite activity or disease, in an animal having a variant peptide, particularly activities and conditions that are known for other members of the family of proteins to which the present one belongs. Thus, the peptide can be isolated from a biological sample and assayed for the presence of a genetic mutation that results in aberrant peptide. This includes amino acid substitution, deletion, insertion, rearrangement, (as the result of aberrant splicing events), and inappropriate post-translational modification. Analytic methods include altered electrophoretic mobility, altered tryptic peptide digest, altered enzyme activity in cell-based or cell-free assay, alteration in substrate or antibody-binding pattern, altered isoelectric point, direct amino acid sequencing, and any other of the known assay techniques useful for detecting mutations in a protein. Such an assay can be provided in a single detection format or a multi-detection format such as an antibody chip array.

In vitro techniques for detection of peptide include enzyme linked immunosorbent assays (ELISAs), Western blots, immunoprecipitations and immunofluorescence using a detection reagent, such as an antibody or protein binding agent, as well as the methods that represent embodiments described herein . Alternatively, the peptide can be detected in vivo in a subject by introducing into the subject a labeled anti-peptide antibody or other types of detection agent. For example, the antibody can be labeled with a radioactive marker whose presence and location in a subject can be detected by standard imaging techniques. Such methods can be used to detect the allelic variant of a peptide expressed in an infected subject and methods which detect fragments of a peptide in a sample.

The diagnostic uses can be applied, not only in genetic testing, but also in monitoring a treatment modality. Accordingly, where treatment is ultimately aimed at preventing or controlling infection, antibodies directed against the protein or relevant fragments can be used to monitor therapeutic efficacy.

The description also encompasses kits for using antibodies to detect the presence of a protein in a biological sample. The kit can comprise antibodies such as a labeled or labelable antibody and a compound or agent for detecting protein in a biological sample; means for determining the amount of protein in the sample; means for comparing the amount of protein in the sample with a standard; and instructions for use. Such a kit can be supplied to detect a single protein or epitope or can be configured to detect one of a multitude of epitopes, such as in an antibody detection array. Arrays are described in detail below for nucleic acid arrays and similar methods have been developed for antibody arrays.

The present description further provides isolated nucleic acid molecules that encode a *T. parva* antigen peptide for protein described herein (cDNA, transcript and genomic sequence). Such nucleic acid molecules will consist of, consist essentially of, or comprise a nucleotide sequence that encodes one of the enzyme peptides described herein, an allelic variant thereof, or an ortholog or paralog thereof.

As used herein, an "isolated" nucleic acid molecule is one that is separated from other nucleic acid present in the natural source of the nucleic acid. Preferably, an "isolated" nucleic acid is free of sequences which naturally flank the nucleic acid (i.e., sequences located at the 5' and 3' ends of the nucleic acid) in the genomic DNA of the organism from which the nucleic acid is derived. However, there can be some flanking nucleotide sequences, for example up to about 5KB, 4KB, 3KB, 2KB, or 1KB or less, particularly contiguous peptide encoding sequences and peptide encoding sequences within the same gene but separated by introns in the genomic sequence. The important point is that the nucleic acid is isolated from remote and unimportant flanking sequences such that it can be subjected to the specific manipulations described herein such as recombinant expression, preparation of probes and primers, and other uses specific to the nucleic acid sequences.

Moreover, an "isolated" nucleic acid molecule, such as a transcript/cDNA molecule, can be substantially free of other cellular material, or culture medium when produced by recombinant techniques, or chemical precursors or other chemicals when chemically synthesized. However, the nucleic acid molecule can be fused to other coding or regulatory sequences and still be considered isolated.

For example, recombinant DNA molecules contained in a vector are considered isolated. Further examples of isolated DNA molecules include recombinant DNA molecules maintained in heterologous host cells or purified (partially or substantially) DNA molecules in solution. Isolated RNA molecules include in vivo or in vitro RNA transcripts of the isolated DNA molecules described herein. Isolated nucleic acid molecules described herein further include such molecules produced synthetically.

The nucleic acid may be double stranded, single stranded, or contain portions of both double stranded or single stranded sequence. As will be appreciated by those in the art, the depiction of a single strand ("Watson") also defines the sequence of the other strand ("Crick"); thus the sequences depicted in the figures also include the complement of the sequence.

As disclosed herein, 100% sequence identity between the RNA and the target gene is not required to practice the present invention. Thus the invention has the advantage of being able to tolerate sequence variations that might be expected due to genetic mutation, strain polymorphism, or evolutionary divergence. RNAi molecules mentioned herein are not limited to those that are targeted to the full-length polynucleotide or gene. Gene product can be inhibited with an RNAi molecule that is targeted to a portion or fragment of the exemplified polynucleotides; high homology (90-95%) or greater identity is also preferred, but not necessarily essential, for such applications.

Accordingly, the description provides nucleic acid molecules that consist of, consist essentially of, or comprise, the nucleotide sequence shown in SEQ ID NO: 25-31 or any nucleic acid molecule that encodes a protein encoded by SEQ ID NO: 25-31.

The term "consisting essentially of," when used in the context of biopolymers, refers to a sequence which is intermediate between the number of residues (amino acids or, in the present case, nucleotides) encompassed by the term "consisting of" and the longer length encompassed by the term "comprising." Residues in addition to the residues encompassed by "consisting of" language do not affect the basic and novel characteristics (*e.g.*, in the case of a polynucleotide, the ability to encode a functional peptide, or to bind specifically to a nucleic acid of interest) of the molecule encompassed by the "consisting of" language.

The description further provides nucleic acid molecules that encode fragments of the peptides described herein as well as nucleic acid molecules that encode obvious variants of the enzyme proteins mentioned herein that are described above. Such nucleic acid molecules may be naturally occurring, such as allelic variants (same locus), paralogs (different locus), and orthologs (different organism), or may be constructed by recombinant DNA methods or by chemical synthesis. Such non-naturally occurring variants may be made by mutagenesis techniques, including those applied to nucleic acid molecules, cells, or organisms. Accordingly, as discussed above, the variants can contain nucleotide substitutions, deletions, inversions and insertions. Variation can occur in either or both the coding and non-coding regions. The variations can produce conservative and non-conservative amino acid substitutions.

The description further provides epitope fragments of the antigen proteins and the nucleic acid molecules encoding the antigens, provided in the description of epitope-mapping experiments described below. An epitope coding region comprises a contiguous nucleotide sequence greater than 12 or more nucleotides. Further, a fragment could at least 30, 40, 50, 100, 250 or 500 nucleotides in length. The length of the fragment will be based on its intended use. For example, the fragment can encode epitope bearing regions of the peptide, or can be useful as DNA probes and primers. Such fragments can be isolated using the known nucleotide sequence to synthesize an oligonucleotide probe. A labeled probe can then be used to screen a cDNA library, genomic DNA library, or mRNA to isolate nucleic acid corresponding to the coding region. Further, primers can be used in PCR reactions to clone specific regions of gene.

A probe/primer typically comprises substantially a purified oligonucleotide or oligonucleotide pair. The oligonucleotide typically comprises a region of nucleotide sequence that hybridizes under stringent conditions to at least about 12, 20, 25, 40, 50 or more consecutive nucleotides.

Orthologs, homologs, and allelic variants can be identified using methods well known in the art. As described in the Peptide Section, these variants comprise a nucleotide sequence encoding a peptide that is typically 60-70%, 70-80%, 80-90%, and more typically at least about 90-95% or more homologous to the nucleotide sequence shown in the Figure sheets or a fragment of this sequence. Such nucleic acid molecules can readily be identified as being able to hybridize under moderate to stringent conditions, to the nucleotide sequence shown in the Figure sheets or a fragment of the sequence. Allelic variants can readily be determined by genetic locus of the encoding gene. The gene encoding the novel enzyme described herein is located on a genome component that has been mapped to the *T. parva* chromosome1 and 2 which is supported by multiple lines of evidence, such as STS and BAC map data.

The nucleic acid molecules described herein are useful as probes, primers, chemical intermediates, and in biological assays. The nucleic acid molecules are useful as a hybridization probe for messenger RNA, transcript/cDNA and genomic DNA to isolate full-length cDNA and genomic clones encoding the peptide described in SEQ ID NO:4-7 and to isolate cDNA and genomic clones that correspond to variants (alleles, orthologs, etc.) producing the same or related peptides shown in SEQ ID NO:1-7.

The probe can correspond to any sequence along the entire length of the nucleic acid molecules provided in the SEQ ID information. Accordingly, it could be derived from 5' noncoding regions, the coding region, and 3' noncoding regions. However, as discussed, fragments are not to be construed as encompassing fragments disclosed prior to the present invention.

The nucleic acid molecules are also useful as primers for PCR to amplify any given region of a nucleic acid molecule and are useful to synthesize antisense molecules of desired length and sequence.

The nucleic acid molecules are also useful for constructing recombinant vectors. Such vectors include expression vectors that express a portion of, or all of, the peptide sequences. Vectors also include insertion vectors, used to integrate into another nucleic acid molecule sequence, such as into the cellular genome, to alter in situ expression of a gene and/or gene product. For example, an endogenous coding sequence can be replaced via homologous recombination with all or part of the coding region containing one or more specifically introduced mutations.

The nucleic acid molecules are also useful for expressing antigenic portions of the proteins.

The nucleic acid molecules are also useful as probes for determining the chromosomal positions of the nucleic acid molecules by means of in situ hybridization methods. The genes encoding the novel antigens described herein are located on a genome component that has been mapped to *T. parva* chromosomes 1 (Tp2).

The nucleic acid molecules are also useful in making vectors containing the gene regulatory regions of the nucleic acid molecules described herein

The nucleic acid molecules are also useful for designing antigens corresponding to all, or a part, of the mRNA produced from the nucleic acid molecules described herein.

The nucleic acid molecules are also useful for making vectors that express part, or all, of the peptides.

The nucleic acid molecules are also useful for constructing host cells expressing a part, or all, of the nucleic acid molecules and peptides.

The nucleic acid molecules are also useful as hybridization probes for determining the presence, level, form and distribution of nucleic acid expression. Such probes could be used to detect the presence of, or to determine levels of, a specific nucleic acid molecule in cells, tissues, and in organisms. The nucleic acid whose level is determined can be DNA or RNA. Accordingly, probes corresponding to the peptides described herein can be used to assess expression and/or gene copy number in a given cell, tissue, or organism. These uses are relevant for diagnosis of disorders involving an increase or decrease in antigen protein expression relative to normal results.

In vitro techniques for detection of mRNA include Northern hybridizations and in situ hybridizations. In vitro techniques for detecting DNA include Southern hybridizations and in situ hybridization.

Probes can be used as a part of a diagnostic test kit for identifying cells or tissues that express an *T. parva* antigen protein, such as by measuring a level of an antigen-encoding nucleic acid in a sample of cells from a subject e.g., mRNA or genomic DNA, or determining if an antigen gene has been mutated.

The description encompasses kits for detecting the presence of an antigen nucleic acid in a biological sample.

For example, the kit can comprise reagents such as a labeled or labelable nucleic acid or agent capable of detecting antigen nucleic acid in a biological sample; means for determining the amount of antigen nucleic acid in the sample; and means for comparing the amount of antigen nucleic acid in the sample with a standard. The compound or agent can be packaged in a suitable container. The kit can further comprise instructions for using the kit to detect antigen protein mRNA or DNA.

The description further provides nucleic acid detection kits, such as arrays or microarrays of nucleic acid molecules that are based on the sequence information provided in SEQ ID NO: 25-31.

As used herein "Arrays" or "Microarrays" refers to an array of distinct polynucleotides or oligonucleotides synthesized on a substrate, such as paper, nylon or other type of membrane, filter, chip, glass slide, or any other suitable solid support. In one embodiment, the microarray is prepared and used according to the methods described in U.S. Pat. No. 5,837,832, Chee et al., PCT application W095/11995 (Chee et al.), Lockhart, D. J. et al. (1996; Nat. Biotech. 14: 1675-1680) and Schena, M. et al. (1996; Proc. Natl. Acad. Sci. 93: 10614-10619). In other embodiments, such arrays are produced by the methods described by Brown et al., U.S. Pat. No. 5,807,522.

The microarray or detection kit is preferably composed of a large number of unique, single-stranded nucleic acid sequences, usually either synthetic antisense oligonucleotides or fragments of cDNAs, fixed to a solid support. The oligonucleotides are preferably about 6-60 nucleotides in length, more preferably 15-30 nucleotides in length, and most preferably about 20-25 nucleotides in length. For a certain type of microarray or detection kit, it may be preferable to use oligonucleotides that are only 7-20 nucleotides in length. The microarray or detection kit may contain oligonucleotides that cover the known 5', or 3', sequence, sequential oligonucleotides which cover the full length sequence; or unique oligonucleotides selected from particular areas, along the length of the sequence. Polynucleotides used in the microarray or detection kit may be oligonucleotides that are specific to a gene or genes of interest.

In order to produce oligonucleotides to a known sequence for a microarray or detection kit, the gene(s) of interest (or an ORF identified in the description) is typically examined using a computer algorithm which starts at the 5' or at the 3' end of the nucleotide sequence. Typical algorithms will then identify oligomers of defined length that are unique to the gene, have a GC content within a range suitable for hybridization, and lack predicted secondary structure that may interfere with hybridization. In certain situations it may be appropriate to use pairs of oligonucleotides on a microarray or detection kit. The "pairs" will be identical, except for one nucleotide that preferably is located in the center of the sequence. The second oligonucleotide in the pair (mismatched by one) serves as a control. The number of oligonucleotide pairs may range from two to one million. The oligomers are synthesized at designated areas on a substrate using a light-directed chemical process. The substrate may be paper, nylon or other type of membrane, filter, chip, glass slide or any other suitable solid support.

In another aspect, an oligonucleotide may be synthesized on the surface of the substrate by using a chemical coupling procedure and an ink jet application apparatus, as described in PCT application W095/251116 (Baldeschweiler et al.). In another aspect, a "gridded" array analogous to a dot (or slot) blot may be used to arrange and link cDNA fragments or oligonucleotides to the surface of a substrate using a vacuum system, thermal, UV, mechanical or chemical bonding procedures. An array, such as those described above, may be produced by hand or by using available devices (slot blot or dot blot apparatus), materials (any suitable solid support), and machines (including robotic instruments), and may contain 8, 24, 96, 384, 1536, 6144 or more oligonucleotides, or any other number between two and one million which lends itself to the efficient use of commercially available instrumentation.

In order to conduct sample analysis using a microarray or detection kit, the RNA or DNA from a biological sample is made into hybridization probes. The mRNA is isolated, and cDNA is produced and used as a template to make antisense RNA (aRNA). The aRNA is amplified in the presence of fluorescent nucleotides, and labeled probes are incubated with the microarray or detection kit so that the probe sequences hybridize to complementary oligonucleotides of the microarray or detection kit. Incubation conditions are adjusted so that hybridization occurs with precise complementary matches or with various degrees of less complementarity. After removal of nonhybridized probes, a scanner is used to determine the levels and patterns of fluorescence. The scanned images are examined to determine degree of complementarity and the relative abundance of each oligonucleotide sequence on the microarray or detection kit. The biological samples may be obtained from any bodily fluids (such as blood, urine, saliva, phlegm, gastric juices, etc.), cultured cells, biopsies, or other tissue preparations. A detection system may be used to measure the absence, presence, and amount of hybridization for all of the distinct sequences simultaneously. This data may be used for large-scale correlation studies on the sequences, expression patterns, mutations, variants, or polymorphisms among samples.

Using such arrays, the description provides methods to identify the presence or expression of the antigen proteins/peptides described herein . In detail, such methods comprise incubating a test sample with one or more nucleic acid molecules and assaying for binding of the nucleic acid molecule with components within the test sample. Such assays will typically involve arrays comprising many genes, at least one of which is a gene described herein and or alleles of the enzyme gene described herein. The figures and associated information below provide information on epitope sequence and micro-variation in strains of *T. parva* that have been found in the gene encoding the antigen described herein.

Conditions for incubating a nucleic acid molecule with a test sample vary. Incubation conditions depend on the format employed in the assay, the detection methods employed, and the type and nature of the nucleic acid molecule used in the assay. One skilled in the art will recognize that any one of the commonly available hybridization, amplification or array assay formats can readily be adapted to employ the novel fragments of the *T. parva* genome disclosed herein. Examples of such assays can be found in Chard, T, An Introduction to Radioimmunoassay and Related Techniques, Elsevier Science Publishers, Amsterdam, The Netherlands (1986); Bullock, G. R. et al., Techniques in Immunocytochemistry, Academic Press, Orlando, Fla. Vol. 1 (1982), Vol. 2 (1983), Vol. 3 (1985); Tijssen, P., Practice and Theory of Enzyme Immunoassays: Laboratory Techniques in Biochemistry and Molecular Biology, Elsevier Science Publishers, Amsterdam, The Netherlands (1985).

The test samples described herein include cells, protein or membrane extracts of cells. The test sample used in the above-described method will vary based on the assay format, nature of the detection method and the tissues, cells or extracts used as the sample to be assayed. Methods for preparing nucleic acid extracts or of cells are well known in the art and can be readily be adapted in order to obtain a sample that is compatible with the system utilized.

In another embodiment, kits are provided which contain the necessary reagents to carry out the assays described herein.

Specifically, the description provides a compartmentalized kit to receive, in close confinement, one or more containers which comprises: (a) a first container comprising one of the nucleic acid molecules that can bind to a fragment of the *T. parva* genome disclosed herein; and (b) one or more other containers comprising one or more of the following: wash reagents, reagents capable of detecting presence of a bound nucleic acid.

In detail, a compartmentalized kit includes any kit in which reagents are contained in separate containers. Such containers include small glass containers, plastic containers, strips of plastic, glass or paper, or arraying material such as silica. Such containers allows one to efficiently transfer reagents from one compartment to another compartment such that the samples and reagents are not cross-contaminated, and the agents or solutions of each container can be added in a quantitative fashion from one compartment to another. Such containers will include a container which will accept the test sample, a container which contains the nucleic acid probe, containers which contain wash reagents (such as phosphate buffered saline, Tris-buffers, etc.), and containers which contain the reagents used to detect the bound probe. One skilled in the art will readily recognize that the previously unidentified enzyme gene described herein can be routinely identified using the sequence information disclosed herein can be readily incorporated into one of the established kit formats which are well known in the art, particularly expression arrays.

The *T. parva* antigens mentioned herein can induce, *in vivo*, antigen-specific CD8+ cytotoxic T cells for prophylactic immunization of cattle for the prevention of East Coast Fever disease. In addition, CTLs specific to the following metazoan parasites may also be induced by compositions identified in accordance with the methods mentioned herein : *Plasmodium falciparum* (which causes, malaria), *Schistosoma mansoni* (which causes schistosomiasis), and *Trypanosoma cruzi* (which causes Chagas disease), *Giardia lamblia*, *Entoemeba histolytica*, *Cryptospiridium* spp., *Leishmania* spp., *Brugia* spp., *Wuchereria* spp., *Onchocerca* spp., *Strongyloides* spp., *Coccidia*, *Haemanchus* spp., *Ostertagia* spp., *Trichomonas* spp., *Dirofilaria* spp., *Toxocara* spp., *Naegleria* spp., *Pneumocystis carinii*, *Ascaris* spp., other *Trypanosoma* spp., other *Schistosome* spp., other *Plasmodium* spp., *Babesia* spp., *Theileria* spp., including but not limited to *T. parva. T. lawrencei*, *T. annulata*, *T. hirci*, *T. ovis*, *T. lastoguardi*, *T. orientalis*, *T. buffeli* and *T. taurotragi;, Babesia spp*., including, but not limited to *B. bigemina*, *B. divergens*, *B. major*, *B. bovis*, *B. motasi, B. ovis*, *B. cabelli, B. equii*, *B. traumani. B. canis. B. gibsoni, B. felis, and B. microfti; Adelina app*., including, but not limited to *A. delina, A. castana, A. picei, A. palori, and A, triboli, Anisakis and Isospora beli.*

Following are examples which illustrate procedures for practicing the invention. These examples should not be construed as limiting. All percentages are by weight and all solvent mixture proportions are by volume unless otherwise noted. As used herein and further defined below, "nucleic acid" may refer to either DNA or RNA, or molecules which contain both deoxy- and ribonucleotides. The nucleic acids include genomic DNA, cDNA, and oligonucleotides including sense and anti-sense nucleic acids. Such nucleic acids may also contain modifications in the ribose-phosphate backbone to increase stability and half life of such molecules in physiological environments.

## EXAMPLES

### Genome sequencing, preliminary annotation, selection of genes encoding candidate antigens

The Theileria parva nuclear genome consists of 4 chromosomes and is approximately 8.2 Mb in length. Tp2, Tp3, and Tp6 loci are located on Chromosome 1. The *T. parva* genome was sequenced using a conventional whole genome shotgun strategy. *T. parva* genomic DNA (Muguga clone) was sheared and cloned into plasmid vectors. Both ends of randomly selected clones were sequenced. A total of 152,226 sequences with an average read length of 623 nt were obtained, equivalent to 9.48X coverages assuming a genome size of 10 Mb. Assembly of the sequences with "TIGR Assembler" produced a set of preliminary contigs represent 95% of the genome sequence.

The preliminary contigs were loaded into an annotation database and subjected to an automated process that searched the *T. parva* contigs against a nonredundant database of proteins extracted from GenBank (called nraa). The search results were used to produce a set of *T. parva* gene models that encoded proteins similar to those in other organisms. This set of gene models was used to train the gene finding programs "GlimmerM" and "phat", which were subsequently run against all of the preliminary contigs to produce gene models for the entire preliminary genome sequence.

The proteins encoded by the predicted *T. parva* genes were subjected to a variety of analyses such as database searched ("blastp"), predictions of signal peptides and signal anchors ("SignalP 2.0"), and transmembrane domains ("TMHMM"). The results were reviewed and a set of ~55 genes encoding candidate antigens were selected for screening for immunogenicity. These gene sequences were screened and this led to the identification of antigens known as Tp2, Tp3, and Tp6.

**Cloning of selected genes for screening for candidate antigens**

**1.1 RNA Extraction:**

One million of *T. parva* (Muguga strain)-infected cells were washed with 1 ml of Phosphate Buffered Saline (PBS) and were centrifuged at 15,000rpm for 10 seconds. After removing the supernatant, the cells were resuspended in 1ml of RNAzol® by pipetting. Then 100μl of chloroform were added and mixed briefly by vortex. The tube was spun at, 15,000rpm for 15min at 4°C and 400μl of the upper aqueous phase were transferred to a new Eppendorf™ tube. 0.7 volume of Iso-propanol was added, vortex mixed and microfuged again at 15,000rpm for 10min at 4°C. After removing the supernatant, the pellet was washed with 300μl of 70% ethanol. After centrifugation at 15,000rpm for 5min at 4°C, 70% ethanol was aspirated and the pellet was resolved in 60μl of RNase free water. The concentration of the RNA was determined by a spectrophotometer.

**1.2 One-step RT-PCR:**

A Qiagen® One-step RT-PCR kit was used to amplify selected genes using RNA obtained above. Primers were designed to amplify the coding region of the selected schizont genes. Forward primers (FW) are listed in Table 1 and reverse primers (RV) are listed in Table 2, and Internal forward primers are listed in Table 3. The components mixed for a 50μl reaction for each set of primers. A master mix (5x RT-PCR buffer 10μl; dNTP mix 2μl; Enzyme mix 2μl; RNase inhibitor 1μl; RNA template 1μl; Water 32μl) was made less the primers and dispensed in labeled tubes, and then 1μl each of the corresponding FW1 and RV1 primers were added. The mixture was overlaid with mineral oil and placed in an MJ Research minicycler and the following programs were applied:

50°C 30 minutes; 95°C 15 minutes; 94°C 30 seconds; 55°C 30 seconds; 72°C 1minute; 35 times from 94°C cycle and finally 72°C 10minutes, or
50°C 30 minutes; 95°C 15 minutes; 94°C minute; 55°C 1 minute; 72°C 1 minute; 35 times from 94°C cycle and finally 72°C 10minutes, or
45°C 30 minutes; 95°C 15 minutes; 94°C 10 seconds; 55°C 1 minute; 68°C 3 minutes; 3.5 times from 94°C cycle and finally 68°C 10minutes.

Table 1.

| Gene (Antigen) | Forward Primer |
|---|---|
| SEQ IN NO:1; Tp2 | SEQ IN NO:34: GCCGCCACCATGAAATTGGCCGCCAGATTAATTAGCC |
| SEQ IN NO:2; Tp3 | SEQ IN NO:35: GCCGCCACCATGAAATTAAATACTATCGCAATAGCCTTT |
| SEQ IN NO:3; Tp6 | SEQ IN NO:36: GCCGCCACCATGGCTCAGATTCCTGTTGATAAATTCG |

Table 2:

| Gene (Antigen) | Reverse Primers for gene cloning |
|---|---|
| SEQ IN NO:1; Tp2 | SEQ IN NO:37: CTATGAAGTGCCGGAGGCTTCTCC |
| SEQ IN NO:2; Tp3 | SEQ IN NO:38: TTAGGATTTTTATTATCGTCTGGACTC |
| SEQ IN NO:3; Tp6 | SEQ IN NO:39: TTATTTATCAGTTGAGAGTAAGAGAGTATTA |

Table 3:

| Gene (Antigen) | Internal Forward Primers for gene cloning |
|---|---|
| SEQ IN NO: 1; Tp2 | SEQ IN NO:40: TCCATGTAAATGGAAAGAAGATTATC |
| SEQ IN NO:2; Tp3 | SEQ IN NO:41: GGAACTCAGGCAGGTTGAATCTCTTC |
| SEQ IN NO:3; Tp6 | SEQ IN NO:42: CCGCTAAGGAAGTGGCTAACATTC |

**1.3 Electrophoresis, Isolation and Purification of PCR products:**

To the completed PCR reaction, 10μl of 6x loading dye was added and loaded onto 0.7% agarose gel containing 0.5ug/ml of Ethidium bromide. Electrophoresis was done at 150 volts for 30minutes. The DNA was visualized over an Ultraviolet light illuminator and expected sizes of fragments were excised from the gel and transferred to appropriately labeled tubes. Fragment DNA was extracted from gel by QIAquick Gel Extraction Kit from Qiagen. To the excised gel piece, buffer QG (three volume of the gel weight) was added and incubated at 50°C with intermittent shaking until dissolved. The dissolved gel was transferred to a QIAquick spin column in a provided 2ml collection tube, then 750μl at a time applied and spun for 1 minute to bind the column until all the gel solution were applied. The flow-through was discarded and the column replaced to the emptied collection tube: The column was washed with 750ul of wash buffer PE and spun for 1minute. The flow-through was discarded and spun again for 1 minute to dry. The column was transferred to a 1.5ml EppendorfTM tube containing 5ul of 3M Sodium Acetate and then 50μl of buffer EB was pipetted into the column and left to stand for 1 minute before being spun for another minute. The eluted DNA was precipitated by addition of 150ul of ethanol, mixed well and incubated at -20°C for 20minutes. The tube was then spun in a microfuge at 15,000rpm for 15min at 4°C. The supernatant was aspirated then the residual pellet was washed with 300ul of 70% ethanol. The tube was spun again in a cooled microfuge at 15,000rpm for 10min at 4°C. The supernatant was aspirated again and the pellet dried. The DNA was resuspended in 8ul of water and 1μl run on a gel for quality and quantity assessment.

**1.4 Ligation:**

After determination the concentration of the fragments, an adjustment was made such that a ligation mix was set up at a molar ratio of 1:10 (vector: insert); -- (pTargeT vector (Promega, 20ng/ul) 1ul; fragment 1-7ul (depending upon the concentration of the fragments); 10x ligation buffer 1ul; T4 DNA ligase 1ul; water to make up final vol. to 10ul). After incubation at 4°C overnight, ligase was inactivated by incubation of the tube at 70°C for 30 minutes and then ethanol precipitated. Pellets were reconstituted in 10μl of water.

**1.5 Transformation:**

JM109 strain of *E.coli* electro-competent cells were used for transformation with the ligated samples. Preparation for electroporation was done by cooling the BIO-RAD® 0.2cm *E.coli* Pulser® Cuvette on ice, thawing the competent cells on ice, setting the electroporator equipment at 2.5KV, 2000mega and 25μF. Five μl of the ligation mixture was added to 50μl of the competent cells, and then quickly transferred into cold cuvettes on ice, tapping firmly onto tissue paper to ensure that all cells went to the bottom without leaving any spaces. The cuvette was wiped dry and pulsed till the PLS signal and an alarm signified successful pulsing. Pulsed cells were resuspended in 1ml of 2xYT medium immediately and then transferred into a Falcon 2059 tube and incubated for 1 hour at 37°C with shaking. When the incubation finished, the cells were plated on 2xYT plates containing 60ug/ml Xgal, 0.1mM IPTG, and 50ug/ml Ampicillin at a various dilutions. The plates were incubated at 37oC for overnight.

### 1.6 Colony screening by PCR:

V-bottom 96 microtiter plates were used for screening of the colonies. The PCR master mix was prepared such that each well has a total of 25μl of [1x PCR buffer mix 23.75μl; gene specific-forward primer 0.5μl; vector specific-reverse primer 0.5μl; Taq polymerase 0.25μl]. To screen the selected gene-containing plasmids, gene-specific primers (IF1, Table3) were used. The vector-specific reverse primer was SEQ ID NO: 44, (5'GAGCGGATAACATCACACAGG3'). Using a sterile tip, a colony was touched, then touched the master plate with the same tip at a place that is numbered (for identification purpose) and finally placed in the corresponding well, leaving it there until all the wells were completed. Using a pipette, the well contents were mixed then the tip was discarded and then followed with a drop of mineral oil in each well. The plate was placed in a MJ Research minicycler and run using the following cycles. 95°C 5 minutes; 94°C20 seconds; 55°C 20 seconds; 72°C 30 seconds; then to step two 34 more times; 72oC 5 minutes; and finally at 10°C for a holding time. At the end of the PCR, the plate was removed and 5ul of 6X loading dye was added to each well. The products were run on a 2% agarose gel. The positive clones on the master plates were identified and a sample taken for a 25ml overnight culture.

### 1.7 Midi-prep:

After the overnight growth of the 25ml culture, cells were spun down in a falcon tube at 3,000rpm for 10min at 4°C and processed as per QIAGEN® HiSpeed™ plasmid purification kit protocol. The pellet was resuspended in 4ml of buffer P1, added 4ml buffer P2, mixed well by inverting six times and left at room temperature for 5min. QIAfilter cartridges were prepared by screwing the cap onto the outlet nozzle and placing in a convenient tube. 4ml of chilled buffer P3 was added into the lysate and mixed immediately but gently by inverting 6 times. The neutralized lysate was poured into the barrel of the cartridge and incubated for 10mm. Meanwhile, a Speed midi tip was equilibrated by adding 4ml of buffer QBT and allowing the column to empty, by gravity flow. The cap was removed from the outlet nozzle and the plunger gently inserted to filter through into the equilibrated HiSpeed tip. The cleared lysate was allowed to enter the resin by gravity flow then washed with 20ml buffer QC. DNA was eluted with 5ml buffer QF into a clean Falcon tube, followed by 3.5ml of 100% Iso-propanol and mixed well. This was then spun at 3500rpm for 30minutes at 4°C, the supernatant discarded, the residual pellet resuspended in 1.5ml 70% ethanol and then transferred into a labeled 2ml EppendorfTM tube. The tube was spun at 15000rpm for 10minutes at 4°C, aspirated and dried then reconstituted in 50μl water. A 1μl sample was run on an agarose gel for quantification and another 10μl were submitted for sequencing and *in vitro* assay (Elispot and bioassay).

### 1.8 Sequencing:

Nucleotide sequence of pTargeT plasmids were determined by ABI PRISM® 3700 DNA Analyzer (Applied Biosystems) using a vector-specific forward primer SEQ ID NO:43; 5'ACGCCAGGATTTTCCCAGTCACG3' and a vector-specific reverse primer SEQ ID.NO:44; 5'GAGCGGATAACATCACACAGG3'.

### Immunization & details of cattle

The 11 cattle used in this example were pure bred *Bos indicus* (Boran) pure bred *Bos taurus* (Friesian) or crossbreeds. Details of the animals are shown in Table 4. Seronegative cattle, as measured by the presence of serum Antibodies to *T. parva*, were immunized by infection and treatment' against the Muguga.stock of *T. parva* by simultaneous inoculation of sporozoites and long-acting oxytetracycline at 20mg/kg BW (Radley et al (1975) Vet Rec 96, 525-7). Cryopreserved sporozoites (Stabilate # 4133) were thawed and diluted 1/20 as previously described. Animals were given a subcutaneous injection of 1ml of diluted sporozoites 2cm above the right parotid lymph node. Animals were monitored daily for changes in rectal temperature and from day 5 post challenge lymph node biopsies were taken using a 21 G needle. Giemsa stained biopsy smears were examined for the presence of schizont infected cells and scored on a scale of 1-3. Animals suffering from moderate reactions were treated with Buparvaquone (Butalex, Mallinckrodt Veterinary Ltd, UK).

Table 4.

| Animal # | Breed | Date of birth | Sire# | Dam# |
|---|---|---|---|---|
| BV050 | Boran | 09/12/99 | 291 | 2772 |
| BV057 | Boran/Jersey | 03/07/00 | NK | AT230 |
| BV115 | Friesian | NK | NK | NK |

(continued)

| Animal # | Breed | Date of birth | Sire# | Dam# |
|---|---|---|---|---|
| BW002 | Jersey | NK | NK | NK |
| BW012 | Boran/Friesian | 18/08/00 | Mystig | 1507 |
| BW013 | Guernsey/Ayshire | 08/09/00 | D14 | D646 |
| BW014 | Guemsey/Ayshire | 15/09/00 | D14 | D638 |
| BX063 | Boran | 11/04/01 | 282 | BN59 |
| BX065 | Boran | 03/04/01 | 282 | BN59 |
| D409 | Guernsey | 06/12/85 | 1422 | D23 |
| F100 | Boran | 17/03/88 | 103 | 1130 |

**Establishment of TpM**

Prior to immunization, venous blood was collected from the eleven animals, , listed in Table 3 and peripheral blood monocytes , (PBMC), were prepared as described previously (Godeeris & Morrison, 1986. Purified PBMC were adjusted to $4x10^6$/ml in CTL medium (RPMI-1640 without HEPES supplemented with 10% fetal bovine serum (FBS); tested for BVDV and mycoplasma spp.). 2mML-glutamine, $5x10^{-5}$ M 2-mercaptoethanol and antibiotics were added to the culture medium and 1ml/well added to 24-well plates (Costar, Corning, NY). and infected *in vitro* with *T. parva* (Muguga) sporozoites). Infected cell lines were maintained in TpM medium (RPMI-1640 supplemented with 10% Fetaclone II (Hyclone, UK; tested for BVDV& *Mycoplamsa spp.*), 100iU/ml Penicillin, 100mg/ml Streptomycin, 50mg/ml Gentamycin, $5x10^{-5}$ M 2-mercaptoethanol and 2mM L-Glutamine) and passaged1/5 three times a week.

**Generation of *T. parva* specific Bulk CTL Cultures**

Venous blood from immunized animals was collected from .4 weeks post-immunization. PBMC were prepared as described previously (Goddeeris & Morrison, 1988). PBMC were adjusted to 4x10 $^6$/ml in CTL medium (RPMI-1640 without HEPES supplemented with 10% FBS (HyClone; tested for BVDV & mycoplamsa spp.), L-glutamine, 2-mercaptoethanol and antibiotics as described above) and 1ml/well added to 24 well plates (Costar, Corning, NY, USA). PBMC were co-cultured with irradiated (50Gy) autologous *T. parva* infected cells (TpM) at 2 x $10^5$ /well and incubated for 7 days at 37°C in a 5% $CO_2$ humidified atmosphere. Cells were harvested by aspiration and dead cells removed by centrifugation over Ficoll-Paque Plus (Amersham Pharmacia Biotech, Uppsala, Sweden). After washing in CTL medium, cells were added to 24 well plates (3x10 $^6$/well) and co-cultured with irradiated PBMC (filler cells) at 1 $x10^6$/well and TpM at 2 $x10^5$/well for 7 days as before. Viable cells were harvested as described above, adjusted to 2 $x10^6$/well and stimulated with 4 x $10^5$/well irradiated TpM and 2 $x10^6$/well irradiated autologous PBMC as filler cells.

**$^{51}$Chromium Release Assay:**

Autologous and allogeneic TpM in log phase of growth were resuspended at $2x10^7$/ml cytotoxicity medium (RPMI-1640 medium with 5% Fetaclone II). 100μl of the target cells were mixed with 100μl (100μCi) of $^{51}$Cr-sodium chromate and incubated for 1 hour at 37°C. Cells were washed 3 times in 7ml of cytotoxicity medium by centrifugation at 1500 rpm for 7 min at RT and resuspended at 1X10 $^6$/ml. Viable cells were harvested from TpM stimulated lines 7 days post-stimulation (effector cells) and resuspended in cytotoxicity medium at $2x10^7$/ml. Two-fold doubling dilutions of effector cells were distributed in duplicate (100μl/well) to 96-well half area (A/2) flat-bottom culture plates (Costar, Corning, NY, USA) resulting in a range of effector cell concentrations of $4x10^6$ to $2.5x10^5$/well. Target cells were added to each well containing effector cells (50μl/well) resulting in target cell ratios ranging from 80:1 to 5:1. In separate triplicate wells target cells were added to 100μl cytotoxicity medium or 1% Tween20 to measure spontaneous and maximal release of the label respectively. Plates were incubated for 4hours at 37°C in 5% $CO_2$ humidified atmosphere. Cells were resuspended in wells by repeated pipetting and pelleted by centrifugation at 180X*g* at room temperature. 75μl of supernatant was transferred from each well into sample vials (Milian, Geneva, Switzerland) and gamma emissions counted in a gamma counter (Micromedic MEplus, TiterTek, Huntsville, AL, USA). Results were calculated and expressed as percent cytotoxicity (= 100 x (test release - spontaneous release) / (maximum release - spontaneous release).

Evidence for MHC class I restricted lysis was assessed by the capacity of monoclonal antibodies recognizing bovine MHC class I to inhibit lysis. Cells were prepared for the cytotoxicity assay as described above except that target cells were resuspended at double the density ($2x10^6$/ml) and 25$\mu$l added first to the plate. 25$\mu$l of either cytotoxicity medium or monoclonal antibody (mAb; IL-A88 diluted 1/15 in cytotoxicity medium was added to target cells and incubated for 30min at room temperature. Serial dilutions of effector cells were then added as described above.

**Generation of *T. parva* specific CD8+ CTL Polyclonal Lines and Clones**

Viable cells from TpM stimulated cultures were harvested day 7 post-infection and $CD8^+$ T cells were isolated either by positive selection using flow cytometry (FACStar Plus, BD Biosciences, San Jose, CA, USA) or negative selection using Dynabeads (Dynal Biotech, Bromborough, UK).

Positive selection: Cells were adjusted to $2x10^7$/ml in sterile monoclonal antibody IL-A105 (specific for bovine CD8) diluted 1/100 and incubated for 30 minutes at 4°C. Cells were washed twice in cold culture medium and resuspended at $2x10^7$/ml in sterile goat anti-mouse polyvalent immunoglobulins conjugated to FITC (Sigma). Cells were washed twice in cold medium before being run through a FACStar plus cell-sorter and $CD8^+FITC^+$ cells collected.

Negative selection: Cells were adjusted to $2x10^7$/ml in sterile mAbs IL-A12 (specific for bovine CD4) and GB21A (specific for bovine gamma-delta-TCR) diluted 1/100 and incubated for 30 minutes at 4°C. Cells were washed twice in cold PBS and resuspended $1.4x10^7$/ml in sterile PBS containing washed sheep anti-mouse IgG Dynabeads according to the manufacturers instructions Cells and beads were rotated for 30 min at 4°C and Dynabead rosetted cells were collected by placing the sample tube in a Dynal Magnetic Particle Concentrator (MPC) for 5 min, the supernatant was removed, transferred to another sample tube and residual Dynabead rosetted cells removed by another incubation in a Dynal MHC. The supernatant was removed and cells washed twice in complete medium.

Cloning: CD8+ T cells enriched by either of the methods described above were adjusted to 30, 10, and 3 cell/ml and distributed into 96-well, round bottom culture plates containing $2x10^4$ irradiated autologous TpM, $5x10^3$ irradiated autologous PBMC and 5U/ml recombinant human IL-2 (HuIL-2; Sigma, Poole, UK) in a final volume of 200$\mu$l/well. Plates were incubated at 37°C in humidified incubator containing 5% $CO_2$ in air. Wells showing significant cell growth were selected for analysis of lysis of autologous TpM in an ($^{111}$In) Indium oxine release assay. The $^{111}$In release assay was performed as described above for $^{51}$Cr release assay except that targets cells were labeled by addition of 5$\mu$Ci $^{111}$In/1 x $10^6$ cells and incubation for 15 minutes at 37°C. Cells were washed five times with cytotoxicity medium and resuspended at $1x10^5$/ml and added 50$\mu$l/well to 96-well V-bottom 96 well plates. 100$\mu$l cells from wells showing significant growth were transferred to 96-well V-bottom culture plates (Greiner) and centrifuged (180xg) for 5 min. Cells were resuspended 100$\mu$l/well in cytotoxicity medium and transferred to wells containing labeled target cells. Plates were centrifuged as described above to pellet cells before being incubated for 4 hours at 37°C in 5% $CO_2$ humidified atmosphere.

CTL populations that exhibited lytic activity on autologous infected cells and originated from cell dilutions that gave rise to cell growth in less than 30% of the wells, as they have high probability of being clones, were selected for expansion. The remaining cells were harvested from each well and resuspended them in culture medium (without HEPES) at a concentration estimated to be between 500 and 5000 cells/ml. 100$\mu$l of cell suspension was distributed into 96 well, round bottom culture plates. 100$\mu$l of autologous irradiated TpM at 5 x$10^4$ autologous irradiated TpM in medium containing 5U/ml HuIL-2. Between day 14 and 21. post-stimulation clones and polyclonal CTL lines were subcultured in 96 well, round bottom culture plates by co-culturing 5000 CTL/well with 25,000 autologous irradiated TpM and 5U/ml HuIL-2 in a final volume of 200$\mu$l/well.

**Establishment and maintenance of bovine skin fibroblasts**

A skin biopsy was taken aseptically from the ears of cattle and placed in a 50ml falcon tube containing Elsevier's solution. In the laboratory laminar flow hood, the biopsy was placed into sterile petri dishes (Sterilin) containing 1ml of 0.25% Trypsin-EDTA. Using a sterile scalpel blade, the sample was cut into small pieces and placed into a 50 ml falcon tube containing Trypsin-EDTA. The preparation was placed in a shaking incubator for 1-1.5 hr at 37°C with gentle continuous shaking. This facilitates detachment of cells. This digestion was stopped by addition of 1ml heat-inactivated FBS. The cell suspension was centrifuged for 10 min at $200\times g$ and the cell pellet re-suspended in 6 ml of Dulbecco's minimum essential medium, DMEM (Gibco-BRL, Paisley, UK) supplemented with 10% heat-inactivated fetal bovine serum (FBS) (Hyclone, Logan, UT), 400 IU/ml penicillin, 300 $\mu$g/ml streptomycin and 2 mM L-glutamine. This cell suspension was seeded in 5ml amounts into 25-$cm^2$ flasks and incubated at 37°C in a 5% $CO_2$ humidified atmosphere. Cultures were examined microscopically after every 4 days for growth and half the medium was replaced with fresh one until growing colonies were evident. Once positive colonies were identified they were rinsed with $Ca^{2+}$- and $Mg^{2+}$-free PBS/EDTA (0.02% EDTA) and detached by 2 min incubation at 37°C with Trypsin-EDTA solution containing 2.5 mg/ml Trypsin and 0.2 mg/ml EDTA in HBSS (Sigma). Following a wash in complete DMEM containing 10% FBS the skin fibroblasts (SF) were passaged into 25-$cm^2$ tissue culture flasks (Costar). Cells were maintained in complete DMEM containing 10% FBS, 200 IU/ml penicillin, 100 $\mu$g/ml streptomycin and 2 mM L-glutamine and passaged every 3 days at a ratio of 1:3. Cells were expanded in 75-$cm^2$ tissue culture flasks (Costar) until confluent yielding $3\text{-}4\times10^6$ cells/flask.

For consistency a liquid nitrogen cell bank was prepared with 2×10[6] cells/freezing vial in 10% DMSO in FBS.

**Establishment and maintenance of bovine testicular endothelial cells**

Bovine testicular vein or pulmonary artery EC lines were established as described by Byrom and Yunker (1990) Cytotechnology 4, 285-90) with the modifications of Mwangi et al (1998) Vet Parasitol. 79, 1-17). Briefly, the vein was placed in wash buffer consisting of $Ca^{2+}$- and $Mg^{2+}$-free PBS supplemented with 400 IU/ml penicillin, 400 μg/ml streptomycin and 5μg/ml fungizone. The vessel was then slit longitudinally, washed twice before being cut into 1-$cm^2$ pieces and then placed lumen side down on a drop of collagenase (1 mg/ml) and incubated for 1 h at 37°C. The cell suspension was centrifuged for 5 min at 200×*g* and resuspended in 24 ml 2× complete DMEM (Gibco-BRL, Paisley, UK) supplemented with 20% heat-inactivated fetal bovine serum (FBS) (Hyclone, Logan, UT), 400 IU/ml penicillin, 300 μg/ml streptomycin, 5 mg/ml fungizone, 300 μg/ml endothelial growth supplement (Sigma, St Louis, MO) and 2 mM L-glutamine. One ml of the cell suspension was seeded in each well of a 24-well tissue culture plate (Costar, Cambridge, MA, USA). Once confluent, monolayers in each well were rinsed with $Ca^{2+}$- and $Mg^{2+}$-free PBS/EDTA (0.02% EDTA) and detached by 2 min incubation at 37°C with 0.25% bypsin/EQTA solution containing 2.5mg/ml trypsin and 0.2 mg/ml EDTA in HBSS (Sigma). Following a wash in DMEM containing 10% FBS the cells were passaged into 25-$cm^2$ tissue culture flasks (Costar). Cells were maintained in complete DMEM containing 10% FBS, 200 IU/ml penicillin, 100 μg/ml streptomycin, 2.5 mg/ml fungizone, 2 mM L-glutamine and passaged every 3 days at a ratio of 1:3. Cells were expanded in 75-$cm^2$ tissue culture flasks (Costar) until confluent yielding between $3\times10^6$ and $4\times10^6$ cells per flask. For consistency a liquid nitrogen cell bank was prepared with 2x $10^6$ cells per freezing vial. Cells were raised from nitrogen into 75-$cm^2$ flasks and used between the fourth and tenth passages.

**Immortalization of bovine skin fibroblasts and endothelial cells**

Cells were immortalized with the SV40 early region gene by transfection of an expression plasmid psvNeo (ATCC code 37150). The product is supplied as a freeze dried to which 300ul of 2XYT with ampicillin was added and fully resuspended. The 300ul suspension was sub-cultured into 6mls and divided into 3mls each. The cultures were incubated overnight at 37°C and then subcultured again into 50mls of 2XYT with ampicillin overnight at 37°C. Maxiprep of psvNeo plasmid were made for use in immortalizations. Plasmid DNA for transfection was standardized to 2mg/ml.

The DNA mix was prepared by mixing 5μl DNA with 495μl of DMEM with antibiotics but no serum. A working dilution of Fugene 6 transfection reagent (Roche) was prepared by mixing 15μl of Fugene with 485μl of DMEM. Mixing was done using the 2ml sterile non-pyrogenic, DNAse and RNase free cryopreservation tubes. The diluted Fugene was added to the diluted DNA drop wise with constant tapping at the end of the tube. The Fugene-DNA complex was allowed to form at room temperature for 30 min.

Cells were cultured in 6-well plates (Costar) at a density of 2 x $10^5$ cells per well and grown to confluence overnight at 37°C, 5% $CO_2$ in a humidified incubator. Culture medium was removed completely from the monolayers to be transfected. The Fugene-DNA complex was then added gently onto the monolayer. Plates were incubated at 37°C, 5% $CO_2$ in a humidified atmosphere for 3-4 hours. The transfection complex was removed; fresh DMEM medium containing 10% fetal calf serum was added and then cultured for a further 72h. Cells from each well were rinsed with PBS/ EDTA, detached with Trypsin/EDTA, washed and re-suspended in complete DMEM and sub-cultured into one T-25 flask (Costar). After incubation for 2-3h, normal DMEM medium was removed and replaced with a selection DMEM medium containing 10% FCS and 0.5μg/ml of G418 (2.5μg/ml G418 for endothelial cells) and incubated further. Upon observation of high death rate of cells, half the medium was replaced with fresh selection medium until growing colonies were evident. This process took 3-4 weeks depending on the cell lines. Positive colonies were the sub-cultured into 24 well plates and then to T-25 flasks. Immortalization was confirmed by checking for expression of large T-antigen using an anti-SV40 antibody conjugated to HRP. Further expansion and maintenance of the cells was carried out using complete DMEM.

**Transfection of COS-7 cells and immortalized skin fibroblasts with schizont cDNA library**

COS-7 cells and iSF were maintained in T75 and T150 TC flasks with DMEM supplemented with 10% FCS, 2mM L-glutamine and antibiotics (TC medium) as described above. Cells are split 1:4 every 3 days. The day prior to transfection, cells were harvested by the removal of medium, washing in PBS and incubation in 0.25% Trypsin-EDTA for 5 min at 37°C. Once cells had detached TC medium was added and cells removed. Cells were washed by centrifugation at 1200 rpm for 10min and resuspended in TC medium. A viable cell count taken, density adjusted to 2.0x$10^5$/ml, cells dispensed, 100μl/well, into 96 well flat-bottom TC plates and incubated for overnight at 37°C in a $CO_2$ (5%) humidified incubator.

DNA was prepared for either single transfections of SF or double transfections (co-transfection of schizont cDNA and BoLA class I cDNA) of COS cells. 6μl of schizont cDNA and 6μl of BoLA class I cDNA (for co-transfection)

at 50ng/μl in $dH_2O$ were added to 150μl unsupplemented DMEM in wells of a 96-well round-bottom plate. FuGENE 6 transfection reagent was pre-warmed to 37°C. 0.9μl or 0.45μl FuGENE 6 was added to each well for double and single transfections respectively. The well contents mixed by shaking on a Dynatech Varishaker for 1 min and incubated at RT for 20 min. The medium from the 96 well plates containing adherent COS cells and SF was removed and each transfection complex added to triplicate wells (50μl/well). The cells were then incubated for 4 hours at 37°C in a $CO_2$ (5%) humidified incubator. The transfection complex was removed and replaced with 200μl/well DMEM supplemented as described above and incubate for 24 or 48 hours at 37°C in a $CO_2$ (5%) humidified incubator.

**Detection of CTL recognition of transfected SF and COS-7 cells by IFN-gamma**γ Elispot

24 hours post-transfection, medium was removed from wells containing transfected cells, cells were washed cells with PBS (200μl/well) and detached by the addition of 100μl/well Trypsin-EDTA as described above. Once the cells had detached the contents of each well were transferred to a 96 well round-bottom plates containing 100μl/well cold RPMI with no HEPES supplemented with 10% FCS (CTL medium). The cells were centrifuged at 1200 rpm for 3 min; supernatant removed and resuspended 50μl/well in CTL medium Schizont specific CTL, generated and maintained as described above, were harvested 7-14 days post-stimulation, transferred to polycarbonate tubes, pelleted at 1200 rpm for 10min and resuspended at $2x10^5$/ml in CTL medium supplemented with 5U/ml HuIL-2 (Sigma). Elispot plates (Millipore Corporation, Bedford, MA, USA) were coated 50μl/well with 2μg/ml of murine anti-bovine IFN-gamma mAb (CC302; Serotec, UK) and incubated overnight at 4°C. Wells were washed twice with unsupplemented RPMI-1640 and blocked 200μl/well with RPMI-1640 supplemented with 10% FBS by incubating at 37°C for 2 hours. The blocking medium was removed and replaced with 50μl/well CTL and 100μl/well transfected cells. As a positive control, irradiated TpM are serially diluted in COS cells or SF with each at a density of $4x10^5$/ml. and populations containing 32, 16, 8, 4, 2, 1% TpM are added 50μl/well to wells containing CTL. Plates were incubated in a humidified incubator at 37°C for 20 hours. After incubation, the contents of wells were removed and wells washed four times with sterile distilled water supplemented with 0.05% Tween 20 per well and the plate shaken on a shaker for 30 seconds between washes. The process was repeated an additional four times, using PBS supplemented with Tween 20 (PBS-T). Wells were then incubated with 100μl/well rabbit anti-bovine IFN-gamma antisera diluted 1/1500 in PBS-T supplemented with 0.2% BSA (PBS/BSA) for 1 hour at room temperature. Wells were washed 4 times with PBS-T before being incubated for 1 hour at room temperature with 100μl/well murine monoclonal anti-rabbit IgG conjugated to alkaline phosphatase (Sigma) diluted 1/2000 in PBS-T/BSA. Sigma Fast BCIP/NBT buffered substrate (Sigma) was by dissolving 1 tablet/10ml $dH_2O$ and passing it through a 0.2μm filter. Plates were washed six times as described above with PBS-T, 100μl/well BCIP/NBT substrate added and plates incubated for 10minutes at room temperature in the dark. The substrate was then removed, wells washed with copious amounts of $H_2O$ and plates air-dried at room temperature in the dark. Plates were finally read on an automated elispot reader (AID Diagnostica, Strasberg, Germany).

**Bioassay for CTL activation based on rapid induction of class II MHC expression by constitutively negative bovine endothelial cells**

Endothelial cells were detached from confluent 75-$cm^2$ tissue culture flasks by treatment with trypsin/EDTA as described. $2.5\times10^4$ cells were seeded into each well of a 48-well plate in 1 ml of complete DMEM and incubated overnight. Cell-free test supernatants derived from either co-culture of *T. parva*-specific $CD8^+$ T cell lines and SF transfected with test genes or recombinant bovine IFN-gammaγ (rBoIFN-gammaγ, the kind gift of Ciba-Geigy, Basel, Switzerland) were dispensed in duplicate wells in a final volume of 160 μl per well. Following 48 h at 37oC, culture supernatants were discarded and the monolayers were washed 3X in FACS medium (RPMI 1640 supplemented with 2%-globulin-free horse serum and 0.1% sodium azide). Endothelial cells ere labeled on ice for 30 mini with 100 ul of an antibody cocktail comprising equal volumes of bovine class II MHC-specific monoclonal antibodies (mAbs) J11, R1 and IL-A21 (each at a 1/500 dilution of ascitic fluid). After 3 washed in FACS medium, 100 ul of FITC-conjugated goat anti-mouse Ig (Sigma) diluted 1/200 in FACS medium were added. After incubation at 4°C for 30 min and a further three washes in FACS medium, cells were detached as described previously and cell surface class II MHC expression determined by flow cytometry, using a FACScan (Becton-Dickinson, Sunnyvale, CA, USA). The percentage of class II MHC-expressing cells was determined by comparison with unstimulated EC labeled in an identical manner.

*T. parva*-specific $CD8^+$ CTL lines were generated and maintained using methods initially described. Test supernatants were collected from 96-well flat-bottomed microtitre plates (Costar) 48 hours after restimulation of resting T cell lines ($2\times10^4$ per well) with $4\times10^5$ COS-7 cells co-transfected with the KN104 gene and test gene(s) or confluent autologous SF transfected with the test gene(s) in a final volume of 200 μl for 24 h. These tests were set-up at least in duplicates. Where indicated, class I MHC was blocked using a specific antibody (IL-A88) to check for MHC class I restriction of the CTL lines. Additional negative control supernatants were derived from co-culture of CTL with untransfected immortalized SF or COS-7 cells. Positive control supernatants were obtained from co-culture of CTL with varying proportions of irradiated autologous TpM.

**Detection of CTL lysis of transfected iSF and COS-7 cells**

Autologous iSF or COS-7 cells were seeded in 6-well plates (Costar) at a density of $2.5x10^5$ /well and incubated for 2 hours at 37°C to allow cells to adhere. For single transfections of iSF, 2μg of test or control cDNA was added to 1ml unsupplemented DMEM containing 3μl FuGENE 6 transfection reagent and incubated for 40min. For COS-7 cells, 2μg of test or control cDNA was added with 2μg of BoLA class I cDNA to 1ml DMEM containing 6μul FuGENE. 1ml of DNA/Fugene complex was added per well of adherent COS-7 or iSF. Plates were incubated for 4 hours at 37°C, the transfection complex was removed and replaced with 2ml/well of complete DMEM and the plates incubated for a further 20 hours at 37°C. Transfected cells were harvested by removal of medium, washing in PBS, detachment by Trypsin-EDTA and washing in complete DMEM. Transfected cells and TpM were labeled with $^{51}$Chromium and the ability of schizont-specific CTL lines (day 6-8 post-stimulation) to lyse these targets was assessed as described above.

**Optimization of IFN-gamma Elispot for the recognition of target antigens by schizont specific CTL**

The ability of the IFN-gamma elispot to detect the recognition of TpM by CTL was first assessed using a CD8+ polyclonal CTL line from animal F100. Fourteen days post-stimulation, CTL were added (5000/well) to coated/blocked elispot wells containing 25,000 irradiated autologous schizont and the formation of IFN-gamma spots assessed after a 20-hour incubation (FIG. 1). Pre-incubating the TpM for 30 min with a mAb against BoLA class I completely inhibited the IFN-gamma response whilst mAbs against MHC class II or the irrelevant CD21 antigen had no effect (FIG. 1). Significantly, there was almost no spontaneous release of IFN-gamma from CTL cultured without TpM. This TpM line did not constitutively express TpM and no IFN-gamma spots could be attributed to the TpM (Data not shown).

In an attempt to replicate the transient transfection situation, where CTL would be co-cultured with COS-7 or iSF of which only a small proportion of cells would be expressing the target antigen, TpM were titrated in COS-7 or iSF and co-cultured with CTL in IFN-gamma elispot plates (FIG. 2A, FIG 2B and FIG. 2C). The stimulator population was fixed at an input of 40,000/well, with only the proportions of TpM and COS-7/SF varying. This cell input was adopted since it was thought to mirror the numbers of APC that would be co-cultured with CTL after transient transfection. Initially different CTL inputs were tried against TpM titrations with the aim of identifying the minimum CTL input required to detect significant responses to 1-3% TpM (FIG 2A). A CTL input of 10,000/well was determined to be optimal since it could elicited significant responses to less than 1% TpM and it was practically feasible.

to raise such CTL numbers for screening experiments. Further titration experiments were performed with CTL clones from F100 to confirm that with these CTL and APC inputs the IFN-gamma elispot was meeting or exceeding the desired sensitivity level (FIG. 2 B). With all the clones tested the IFN-gamma elispot could still detect recognition of target cells when they constituted only 0.1% of the total cell population. The Elispot assay worked well with little background noise and met the sensitivity requirements when TpM were titrated in COS-7 cells but it was important to determine that the assay performed as well when the TpM were titrated in autologous iSF. FIG 2C shows the results of co-culturing F100 CD8+ polyclonal CTL line with TpM diluted in COS-7 cells, autologous primary SF and iSF. Neither primary nor iSF significantly affected the background levels or the sensitivity of the elispot assay.

The conclusions that iSF were appropriate APC and that the elispot had been sufficiently optimized using TpM were rapidly validated by the identification of CTL target schizont antigens initially with Tp1 and COS-7 cells and then Tp2 with iSF. The effect of reduced DNA input upon subsequent recognition of target antigen transfected COS-7 cells and iSF were compared using Tp1 and Tp2 and the results showed recognition of target antigen transfected COS-7 or BW014 when only 1ng/well if target antigen DNA is used to transfect. The response increased with the concentration of target DNA peaking at peaking at 50ng/well. This result validated the use of test genes or cDNA pools at 100ng/well for transfection of both COS-7 and iSF.

**Identification of Tp2 following the screening of selected genes with BW002, BW013, and BW014 CD8+ CTL and D409 CTL clone #10.**

Tp2 was identified through the screening of selected gene transfected autologous iSF with CTL from BW002 (BW2), BW014 (BW14) and D409 (Figure 3). Selected gene #5 was specifically recognized by all CTL and was named Tp2. Tp2 transfected iSF were also specifically lysed by CTL as assessed by $^{51}$Chromium release assays (Figure 4). MHC restriction of Tp2 recognition was assessed by the introduction of blocking anti-BoLA class I mAbs. Anti-class I mAb resulted in the inhibition of lysis. The introduction of a mAb (IL-A13) that specifically binds to the BoLA class I molecule w7 also inhibited the lysis of Tp2 transfected targets by a CTL clone from D409, suggesting that the recognition was restricted by w7.

## Identification of CTL target *T. parva* schizont antigens

The identification of CTL target schizont antigen Tp2 was performed using the assay that employs immortalized skin fibroblast (iSF) cell lines. The effect of reduced DNA input upon subsequent recognition of target antigen transfected COS-7 cells and iSF were compared using Tp1 and Tp2. The results, show recognition of target antigen transfected COS-7 or BW014 when only 1ng/well if target antigen DNA is used to transfect. The response increased with the concentration of target DNA peaking at peaking at 50ng/well. This result validated the use of test genes or cDNA pools at 100ng/well for transfection of both COS-7 and iSF. Testing of Tp2-transfected autologous BW002 and Tp2-transfected allogeneic, BV050 iSF, suing the schizont-specific BW002 polyclonal CD8+ CTL line, indicated lysis of the Tp2-transfected BW002 line . In addition, when the Tp2 transfected target cells were pre-incubated with an anti-BoLA, Class I monoclonal antibody (mAb), no lysis occurred; indicating the MHC Class I restriction of CTL-mediated killing of the transfected iSF (Fig. 4).

## Detection of Tp2 Specific Ex Vivo CD8+ T Cell Responses from Immune Cattle after Challenging with *T. parva* Sporozoites

Cattle, BW002, BW013 and BW014, whose schizont specific CTL lines had been shown to recognize Tp2, were challenged with a lethal dose of *T. parva* (Muguga) sporozoites. Cry preserved sporozoites (Stabilate # 4133) were thawed and diluted 1/20 as previously described. Animals were challenged by subcutaneous injection of 1ml of diluted sporozoites 2cm above the right parotid lymph node. Animals were monitored daily for changes in rectal temperature and from day 5 post challenge lymph node biopsies were taken using a 21G needle. Giemsa stained biopsy smears were examined for the presence of schizont infected cells and scored on a scale of 1-3. Animals were bled on day 2 and daily from day 6 to 13 and PBMC were isolated as described above. CD8+ T cells and CD14+ monocytes were purified from PBMC by MACS magnetic cell sorting according to the manufacturer's instructions (Miltenyi Biotec, Gergisch Gladbach, Germany). CD8+ T cells were sorted indirectly using a monoclonal antibody specific for bovine CD8 (IL-A105) followed by incubation with goat anti-mouse IgG microbeads (Miltenyi Biotec). CD14 monocytes were sorted directly by incubation with CD14 microbeads (Miltenyi Biotec). PBMC and CD8+ T cells were added to wells (2.5x105/well) of coated/blocked Elispot plates and stimulated with autologous TpM (2.5x104/well) or Tp2 peptides (1mg/ml final concentration). Purified monocytes were additionally added (2.5x104/well) to wells containing peptide and CD8+ T cells. Elispot plates were incubated and developed as described above. In order to recall Tp2 peptide specific CTL responses, PBMC were stimulated with autologous TpM 14 days post-challenge as described above. Viable cells were harvested 7 days post-stimulation and lytic activity against TpM and Tp2 peptide pulsed autologous iSF assessed as described above. FIG. 5A, FIG. 5B, and FIG. 5C illustrate Tp2 specific $CD8^+$ T cell responses following challenge of immune cattle. Three ECF immune cattle (BW002, BW013, BW014), from which schizont specific CTL lines had been generated and shown to recognize Tp2, were challenged with a lethal dose of *T. parva* (Muguga) sporozoites. Peripheral blood was collected daily between day 7 - 13 post-challenge, and CD8+ T cells and monocytes purified. Responses to Tp2 peptides containing previously identified CTL epitopes or control peptides were assessed by co-culturing CD8+ T cells and monocytes in the presence of 1□g/ml peptide and measuring the release of IFN-gamma elispot. Responses to autologous TpM were included as a positive control.

## Mapping of the Tp2 CTL epitopes

A cleaved PepSet library of 84 peptides was synthesized to cover the full length of the Tp2 protein (Mimotopes). Recognition of Tp2 peptides was assessed by IFN-gamma elispot using autologous iSF as antigen-presenting cells. FIG. 6 shows the results of screening the Tp2 PepSet with BW002, BW013, BW014 CD8+ polyclonal CTL lines and D409 CTL clone#10. The BW002 polyclonal line responded to a single peptide #43; (SEQ ID NO:8 CSHEELKKLGML) when the peptides were used at a concentration of 1pg/ml. BW0013 CTL responded significantly to two pairs of overlapping peptides; peptides #53 and 54 (SEQ ID NO: 9 (FKSSHGMGKVGKRY) and peptides #78 and 79 (SEQ ID NO: 11 FAQSLVCVLMKCRG). BW014 CTL also responded to peptides #78 and 79 suggesting a common epitope. This was not perhaps surprising since BW013 and BW014 shared a sire. The w7 restricted D409 CTL clone responded to peptides #77 and 78, SEQ ID NO: 12 (KCFAQSLVCVLMKC), suggesting an overlapping epitope. In order to define the minimal length epitope for the shared BW013 and BW014 CTL epitope, all possible 9, 10 and 11mers covering SEQ ID NO: 9 (FKSSHGMGKVGKRY) and SEQ ID NO: 11 (FAQSLVCVLMKCRG) were synthesized and screened by IFN-gamma elispot. BW013 responded to the 11mer SEQ ID NO: 6 (KSSHGMGKVGK) and not to any shorter sequence suggesting this was the epitope. The results of screening the six possible 9mers of SEQ ID NO: 11 (FAQSLVCVLMKCRG) are shown in FIG 7. The results revealed that both BW013 and BW014 CTL responded to only the 9mer SEQ ID NO: 4 (QSLVCVLMK) suggesting that this was the minimal length epitope. The peptides were also screened with D409 clone #10 and found that only the 9mer peptide SEQ ID NO:5 (FAQSLVCVL) was recognized. This epitope was named Tp2 epitope 2 (Tp2.2). 51Chromium-release assays were performed with peptide pulsed autologous iSF. FIG. 8 and FIG. 9 illustrate that both BW014 and D409 CTL lysed Tp2 peptide pulsed iSF with levels of lysis comparable to TpM. The lysis was very specific with almost 100% lysis of iSF pulsed with Tp2 peptide 75B SEQ ID NO: 4 (QSLVCVLMK) and background levels of lysis of iSF pulsed with Tp2 peptide 76B SEQ ID NO: 17 (SLVCVLMKC). SEQ ID NO: 4 (QSLVCVLMK) was named Tp2 epitope 1 (Tp2.1). Subsequent screening using similar experiments with sets of peptides also identified Tp2 epitope 4 (Tp2.4), SEQ ID NO: 7 (SHEELKKLGML) and Tp2 epitope 3 (Tp2.3), SEQ ID NO: 6, (KSSHGMGKVGK). Peptide-pulsed iSF and BoLA class I P815 transfectants were prepared as targets for 51Chromium release assays by incubating 2x106 iSF or P815 cells overnight in T25 tissue culture flasks (Costar) with Tp2 peptides diluted to 1mg/ml in complete DMEM. Cells were harvested, labeled and assayed as described above.

**Identification of Minimal Tp2 CTL Epitopes with Synthetic Peptide Libraries**

Peptide libraries (Cleaved PepSets; Mimotopes, Clayton, Australia) were generated for the Tp2- HD6 restricted CTL epitope. The PepSet libraries contained every 12mer, 11mer, 10mer and 9mer offset by 2 amino acids from the protein sequences. However, the peptides were prepared by truncations of the 12mers at the N-teminus and were supplied lyophilized with each tube containing a nominal 12mer and the 9, 10, 11 mer truncations with the same C terminus. Peptides were dissolved in 400ml 50% (v/v) DNA synthesis grade acetonitrile/water (Applied Biosystems, Warrington, UK). To aid the dissolution, tubes were held in a sonicator water bath for 2 x 10 min. Peptides were aliquoted into labeled cryopreservation tubes (Greiner) and stored at —20oC. For screening with CTL, peptides were prepared at 10mg/ml in complete RPMI-1640 and 10ml added to triplicate wells of an Elispot plate, coated, washed and blocked as described above. Autologous iSF or P815 cells stably expressing the BoLA class I HD6 (P815-HD6) or-JSP-1 (P815-JSP-1) were adjusted to a density of 4x105/ml and 50ml added to wells containing peptides. The plates were incubated at 37oC for 1 hour before CTL, prepared as described above for screening transfectants, were added 50ml/well. Plates were incubated for 20 hours at 37oC and then developed as described above. Based on the results of the screening with the Tp2 PepSets, individual 9, 10 and 11mer peptides were synthesized in order to define the CTL epitopes of Tp2, figures 10, 11, 12, and 13. Peptides were prepared and screened using the IFN-gamma elispot as described above. Table 5 summarizes that minimal length epitopes of Tp2, both in terms of amino acid sequence information and corresponding nucleotide sequences.

Table 5.

| *T. parva* antigenic polypeptides | CTL (antigen)epitopes | |
|---|---|---|
| | aa sequence | DNA sequence |
| Tp2 \(SEQ ID NO: 1) | | |
| (Tp2.1) | SEQ. ID. NO:4 QSLVCVLMK | SEQ. ID. NO:28 CAAAGCCTAGTGTGCGTATTAATGAAA |
| (Tp2.2) | SEQ ID. NO:5 FAQSLVCVL | SEQ. ID. NO:29 TTTGCACAAAGCCTAGTGTGCGTATTA |
| (Tp2.3) | SEQ. ID. NO:6 KSSHGMGKVGK | SEQ. ID. NO:30 AAATCATCACATGGTATGGGAAAGGTAGGAAAA |
| (Tp2.4) | SEQ. ID. NO:7 SHEELKKLGML | SEQ. ID. NO:31 AGTCATGAAGAACTAAAAAAATTGGGAATGCTA |

**Kinetics of Tp2 specific CD8$^+$ T cell responses from immune cattle following challenge with *T. parva* sporozoites**

Cattle BW002, BW013 and BW014, whose CTL lines had recognized Tp2, were challenged with a lethal dose of *T. parva* (Muguga) sporozoites and the responses of PBMC and purified CD8+ T cells to TpM and Tp2 peptides were measured longitudinally. All animals were solidly resistant to challenge with none experiencing fever or detectable parasitosis (data not shown). FIG 5A, FIG. 5B, and FIG. 5C, show the TpM and Tp2 specific CD8+ T cell responses of BW002, BW014 and BW013. From day 9 post-challenge, CD8+ T cells responded specifically to the Tp2 peptides containing the previously identified CTL epitopes. The responses increased rapidly exceeding the response to TpM and were sustained over the period of observation (day 13 post-infection). The frequency of Tp2 epitope responding CD8+ cells peaked around 1:500 for both BW002 (FIG. 5A) and BW014 (FIG. 5B), the Tp2 specific response of BW013 was significantly weaker (FIG. 5C). The kinetics of this response is comparable to that previously described for schizont specific CTL in efferent lymph following challenge of immune cattle with *T. parva* sporozoites.

Attempts were made to detect Tp2 specific lytic responses directly in peripheral blood post-challenge but these failed (Data not shown). An experiment was instigated to first expand schizont specific CTL numbers by a single in vitro stimulation with TpM and then to assess Tp2 specific lysis. Stimulated cells exhibited extremely high cytotoxic activity against both TpM and fibroblasts pulsed with a Tp2 peptide known to contain a CTL epitope (FIG. 14). These results provide the first evidence direct from immune animals that Tp2 may be involved in protection against ECF.

**Optimization of INF-gammaγ Elispot for the recognition of target antigens by schizont specific CTL**

The ability of the IFN-gamma elispot to detect the recognition of TpM by CTL was first assessed using a $CD8^+$ polyclonal CTL line from animal F100. Fourteen days post-stimulation, CTL were added (5000/well) to coated/blocked elispot wells containing 25,000 irradiated autologous schizont and the formation of IFN-gamma elispot assessed after a 20-hour incubation. CO-culture of CTL with TpM resulted in significant release of IFN-gamma. Pre-incubating the CTL for 30 min with a mAb against BoLA class I completely inhibited the IFN-gamma response whilst mAbs against MHC class II or the irrelevant CD21 antigen had no effect. Significantly, there was almost no spontaneous release of IFN-gamma from CTL cultured without TpM. This TpM line did not constitutively express TpM and no IFN-gammaγ spots could be attributed to the TpM.

In an attempt to replicate the transient transfection situation, where CTL would be co-cultured with COS-7 or iSF of which only a small proportion of cells would be expressing the target antigen, TpM were titrated in COS-7 or iSF and co-cultured with CTL in IFN-gamma elispot plates. The stimulator population was fixed at an input of 40,000/well, with only the proportions of TpM and COS-7/SF varying. This cell input was adopted since it was thought to mirror the numbers of APC that would be co-cultured with CTL after transient transfection. Initially different CTL inputs were tried against TpM titrations with the aim of identifying the minimum CTL input required to detect significant responses to 1-3% TpM. A CTL input of 10,000/well was determined to be optimal since it could elicit significant responses to less than 1% TpM and it was practically feasible to raise such CTL numbers for screening experiments. Further titration experiments were performed with CTL clones from F100 to confirm that with these CTL and APC inputs the IFN-gamma elispot was meeting or exceeding the desired sensitivity level. With all the clones tested the IFN-gamma elispot could still detect recognition of target cells when they constituted only 0.1 % of the total cell population. The elispot assay worked well with little background noise and met the sensitivity requirements when TpM were titrated in COS-7 cells but it was important to determine that the assay performed as well when the TpM were titrated in autologous SF. Neither primary nor iSF significantly affected the background levels or the sensitivity of the elispot assay.

In advance of the initiation of screening for CTL target antigens by the transient transfection of COS-7 cells and iSF, the efficiencies of COS-7 and iSF transfection in 96 well TC plates was assessed using GFP as a reporter gene. Whilst there was considerable variation in transfection efficiencies between cell lines and between experiments, COS-7 consistently transfected better than iSF with efficiencies varying from 5-50% whereas for iSF transfection efficiency ranged between 0.5 - 20%. The transfection efficiency of iSF was assessed to be good enough to allow the presentation and identification of transfected schizont cDNA.

**Evaluation of IFN-gamma Elispot Bioassay as a complementary read-out system for CTL recognition of target antigens**

Bovine vascular EC were stained for surface class II MHC expression following culture for 48 h in the presence of media containing recombinant IFN-gamma The sensitivity to rBoIFN-gammaγwas determined and found to be between 100 and 10 pg/ml. Comparisons were made between IFN-gamma bioassay and elispot by co-culturing *T.parva*-specific CTL with fixed number of autologous iSF containing varying proportions of target autologous TpM and skin fibroblasts. There was a good correlation between the two assays. Both assays detected production of IFN-gamma in co-cultures containing as low as 1% TpM.

**Nucleotide and deduced amino acid sequences and putative identity of the 5 candidate antigens**

Following confirmation of the candidates as CTL target antigens, the individual cDNA were sequenced and confirmed to be of *T. parva* origin by interrogating the *T. parva* genome sequence database. BLAST searches (Altschul *et al* 1990) of DNA and protein database were performed and homologues of some of the antigens identified. SignalP (Nielsen et al (1997) Int J Neural Syst. 8, 581-99) and Tmpred (http://www.ch.embnet.org/software/TMPRED_form.html) analyses were conducted to predict the presence of a signal peptide and transmembrane domain. SEQ ID NO: 25-31 indicate the DNA and SEQ ID NO: 1-7, the deduced protein sequences of the five candidate antigens.

**Expression of Tp2, Tp3, and Tp6 protein from recombinant DNA plasmids**

The reading frames of *T. parva* candidate antigen genes were amplified by PCR using Taq polymerase (Promega, Madison, WI USA 53711) from the original full-length genes in their respective plasmids in pcDNA3. Both the forward and reverse primers contained restriction enzyme sites (Table 6). The product of the amplification of Tp2 was .8 Kb in size, as measured by PAGE electrohoresis. The PCR products were digested with the respective restriction enzymes, and ligated into bacterial His-tag expression vectors, pQE30 (Qiagen, 28159 Avenue Stanford, Valencia, CA91355) or PET28 (Novagen, Madison, WI 53719 USA). Primer sequences for the cloning are given in Table 6 (primers for cloning Tp gene sequences), Table 7 (primers for adding "Tag"-expression sequences), and Table 8 (primers for amplifying Tp3tag sequences and Tp6tag sequences).

**Table 6.**

| Gene (Plasmid) | Forward Primer | Reverse Primer |
|---|---|---|
| Tp2 (pTargeT) | SEQ ID NO: 32: GGTAATTGTAGTCATGAAGAAC | SEQ ID NO: 33: TTTACTAATACCACCAAGACCGTG |
| Tp3 (pTargeT) | SEQ ID NO: 45: CGCGGATCCGCCACCATGAAATTAAATACTATC | SEQ ID NO: 46: CGCCTCGAGGGATTTTTTATTATCGTCTGGAC |
| Tp6 (pTargeT) | SEQ ID NO: 47: CGCGCTAGCGCCGCCACCATGGCTCAGATTCCT | SEQ ID NO: 48: GCGCTCGAGTTTATCAGTTGAGAGTAAGAGAG |

**Table 7.**

| To Add Tag | Forward Primer | Reverse Primer |
|---|---|---|
| | SEQ ID NO: 49: SEQ ID NO: 50. CGCCTCGAGTCCTACATACCATCTGCCGAAAAG CGCAAGCTTTCAGTCTAGTCCTAGCAAAGGGTTAG | |

**Table 8.**

| Gene Amplifica tion | Forward Primer | Reverse Primer |
|---|---|---|
| Tp3 | SEQ ID NO: 51: CGCGGATCCGCCACCATGAAATTAAATACTATC | |
| Tp6 | SEQ ID NO: 53: CGCGCTAGCGCCGCCACCATGGCTCAGATTCCT | |
| pTargeT | | SEQ ID NO: 52: CGCAAGCTTTCAGTCTAGTCCTAGCAAAGGGTTAG |

The plasmids were transformed into *E. coli* strain DH5α (Life Technologies, Carlsbad, CA 92008, USA). All the plasmids were sequenced to ensure that they harbored no substitutions compared to the original genes. Purified plasmids were then used to transform competent BL21 DE3 bacterial cells.

Single BL21 DE3 bacterial colonies bearing the recombinant plasmids were isolated and cultured in 2XYT (formula) at 37°C to an $OD_{600}$ of 0.6, and protein expression induced by addition of IPTG to a final concentration of 1mM, and further cultured for 4 h. The cells were harvested by centrifugation at 4000 g for 20 min. Recombinant proteins were isolated by either the native or denaturing nickel-nitrilotriacetic (Ni-NTA) agarose according to the manufacturer's protocol (Qiagen, 28159 Avenue Stanford, Valencia, CA91355), dialyzed against PBS and stored at -20 °C. Purified proteins were checked by checked on 12% SDS-PAGE gels (Laemmli, 1970) and western blot. Protein concentration was determined by the BCA Protein Assay reagent (PIERCE, Rockford, IL 61105, USA). Bacterial cells or purified proteins were applied to a 12% SDS-PAGE gel under denaturing conditions (Laemmli, 1970). Proteins were electroblotted onto nitrocellulose sheets (Schleicher and Schull, Dassel, Germany). Mouse His-tag antibody (SIGMA) was used as the primary antibody, while anti mouse horse-radish peroxidase conjugate (SIGMA) was used as the secondary antibody followed detection with 3, 3'-Diamnobenzidine and hydrogen peroxide. The reading frame of the segments of Tp2 amplified and cloned into the bacterial expression vector harbored no substitutions compared to the original gene sequences (not shown). The recombinant protein Tp2 containing His-tag was produced from the construct, and can be determined by immunoblotting using His-tag antibody. Figure 15A indicates total protein from an aliquot of lysate and Figure 15B shows the presence of the Tp2-His-tag product as stained by the His-tag-labeled antibody. Expression of Tp3 (16) and Tp6 (FIG 17) has been demonstrated using SDS-PAGE gel electrophoresis of protein from cultures oftransfected *E. coli*.

**Vaccination strategy**

Cattle trials were performed to assess the vaccine potential of identified CTL target antigens utilizing a recombinant canary pox virus (patented by Merial Ltd) and modified Vaccinia Virus Ankara (MVA) as an antigen delivery method. FIG. 18 indicates the level of CTL responses to several Tp antigens, following CP (Canary Pox mediated)/MVA (modified Vaccinia Virus Ankara-vector mediated) immunization protocols. The data indicates the production of *T. parva* antigen-specific CD8 (CTLs) in animals following immunization protocols with Canary Pox viral vectors, expressing *T. parva* antigen. Note that at day 7, the response to Tp2 shows an increase in the number of spot forming cells (SFC)/ well, over that shown in FIG. 20 (representing the phosphate buffered saline (PBS) control), for Day 7.

In FIG. 19 the level of CTL responses to Tp2 antigen, following DNA/MVA immunization protocols is plotted. The data indicates the production of *T. parva* antigen-specific CD8 (CTLs) in animals following immunization protocols with modified Vaccinia (Ankara) viral vectors, expressing *T. parva* antigen. Note that at day 7 and at DAY 28, after a second, booster administration, the response to Tp2 shows an increase in the number of spot forming cells (SFC)/well, over that shown in FIG. 20 (representing the phosphate buffered saline (PBS) control), for Day 7 and Day 28.

The data in FIG. 20 represent control data for CP/MVA (FIG. 18) and DNA/MVA (FIG. 19) immunization protocols, where cattle were given phosphate buffered saline injections rather than Tp antigens. The control experimental data for measuring the development of CTLs following immunization indicates a lack of *T parva*, antigen-specific CD8+ cells in PBS immunized animals. These trials tested the Tp2 antigen and involved use of 16 cattle in order to evaluate antigen-specific CTL responses and relate these to the outcome to challenge. Animals were inoculated intramuscularly with 1 ml of vaccine ($1x10^8$ pfu of virus) and boosted similarly after 4 weeks.

Additional immunization trials to test cattle Following a further 4 weeks, cattle are subjected to an $LD_{100}$ challenge with *T. parva* sporozoites by administering subcutaneously 1 ml of diluted stabilated infective material four weeks after immunization with the T. parva antigens, using Canary Pox and MVA vectors, and the DNA/MVA system. Animals are monitored parasitologically and clinically over a period of 2-3 weeks to determine whether the vaccine has protected. It is expected that the vaccines are protective. Additional immunological assays are performed following immunization and challenge.

Various modifications and variations of the described method and system of the invention will be apparent to those skilled in the art without departing from the scope and spirit of the invention. Although the invention has been described in connection with specific preferred embodiments, it should be understood that the invention as claimed should not be unduly limited to such specific embodiments. Indeed, various modifications of the above-described modes for carrying out the invention which are obvious to those skilled in the field of molecular biology or related fields are intended to be within the scope of the following claims.

## Claims

1. An isolated polypeptide comprising a sequence represented by SEQ ID NO:1, or a variant thereof having greater than 90% identity thereto and having the same or similar biological activity as SEQ ID NO: 1, or any one of SEQ ID Nos 4 to 7.

2. A pharmaceutical composition, comprising a polypeptide of claim 1 and a pharmaceutically acceptable carrier.

3. An immunogenic composition, comprising a polypeptide of claim 1 and, optionally, an adjuvant, the composition being immunogenic.

4. The immunogenic composition of claim 3, which stimulates cytotoxic T cells specific to the polypeptide.

5. The immunogenic composition of claim 3, which comprises an epitope that stimulates *Theileria parva* (*T. parva*-) specific cytotoxic T cells.

6. A vaccine, comprising one or more polypeptides of claim 1 and, optionally, an adjuvant; wherein said polypeptide is immunogenic.

7. The vaccine of claim 6, which protects an animal against *T. parva* infection.

8. The polypeptide of claim 1, which is present in detectable amounts in isolates of *T. parva.*

9. The polypeptide of claim 1, comprising a *T. parva* antigen.

10. A method for producing a polypeptide which stimulates a *T. parva*-antigen specific cytotoxic lymphocyte (CTL), comprising culturing a host cell, wherein said host cell comprises a vector comprising a recombinant construct comprising a polynucleotide encoding a polypeptide according to claim 1, operably linked to an expression control sequence, wherein said host cell is cultured under conditions effective for producing a polypeptide encoded by the polynucleotide, and harvesting the polypeptide.

11. An antibody specific for the polypeptide of claim 1.

12. The antibody of claim 11, which is a polyclonal antibody.

13. The antibody of claim 11, which is coupled to a carrier and/or a label.

14. A kit for detecting the presence of *T. parva* in a sample suspected of containing *T. parva,* or for purifying *T. parva* from a sample containing *T. parva,* comprising an antibody according to any one of claims 11 to 13.

15. The kit of claim 14, which further comprises means for performing an enzyme-linked or Western blot assay to detect the presence of *T. parva.*

16. The kit of claim 14 or 15, which further comprises means for binding the antibody to *T. parva* in the sample, and for releasing the organism from the antibody.

17. Use of a polypeptide according to claim 1, or a vector comprising a recombinant construct comprising a polynucleotide encoding a polypeptide according to claim 1, operably linked to an expression control sequence, or a host cell comprising a vector comprising a recombinant construct comprising a polynucleotide encoding a polypeptide according to claim 1, operably linked to an expression control sequence, in the preparation of a medicament for the prevention and/or treatment of infection by *T. parva.*

18. A method for preparing a polyclonal antibody, comprising immunizing a non-human animal with one or more polypeptides of claim 1.

19. A method for preparing a polyclonal antibody, comprising immunizing a non-human animal with a host cell; wherein said host cell comprises a vector comprising a recombinant construct comprising a polynucleotide encoding a polypeptide according to claim 1, operably linked to an expression control sequence.

20. A method for preparing a monoclonal antibody, comprising:

(a) immunizing a non-human animal with a polypeptide of claim 1,
(b) recovering cells from the non-human animal which produce antibody that binds to the polypeptide,
(c) preparing a hybridoma with the cells isolated in (b), and
(d) recovering a monoclonal antibody from the hybridoma that binds to the polypeptide in (a).

21. A method for preparing a monoclonal antibody, comprising:

(a) immunizing a non-human animal with a host cell; wherein said host cell comprises a vector comprising a recombinant construct comprising a polynucleotide encoding a polypeptide according to claim 1, operably linked to an expression control sequence,
(b) recovering cells from the non-human animal which produce antibody that binds to a polypeptide produced by the host cell,
(c) preparing hybridomas with the cells isolated in (b), and
(d) recovering a monoclonal antibody from the hybridoma that binds to the polypeptide in (b).

22. Use of an antibody according to any one of claims 11 to 13 in the preparation of a composition for the diagnosis of infection by *T. parva* in a sample.

23. A vaccine comprising a vector comprising a recombinant construct comprising a polynucleotide encoding a polypeptide according to claim 1, operably linked to an expression control sequence.

**24.** A vector comprising a recombinant construct comprising a polynucleotide encoding a polypeptide according to claim 1, operably linked to an expression control sequence.

**25.** The vector according to claim 24 wherein said vector is a bacterial plasmid, a bacteriophage, a yeast episome, a yeast chromosomal element, a yeast artificial chromosome, a viral vector, a baculovirus, a papovavirus, SV40, a vaccinia virus, modified vaccinia virus Ankara strain (MVA), an adenovirus, a poxvirus, a fowlpox virus (FP9), a canary pox, a pseudorabies virus, or a retrovirus.

## Patentansprüche

**1.** Isoliertes Polypeptid, umfassend eine Sequenz, die in SEQ ID NO:1 dargestellt ist, oder eine Variante davon, die mehr als 90% Identität dazu aufweist und die gleiche oder eine ähnliche biologische Aktivität wie SEQ ID NO:1 aufweist, oder eine der SEQ ID NOs: 4 bis 7.

**2.** Arzneimittel, umfassend ein Polypeptid nach Anspruch 1 und einen pharmazeutisch verträglichen Träger.

**3.** Immunogene Zusammensetzung, umfassend ein Polypeptid nach Anspruch 1 und gegebenenfalls ein Adjuvans, wobei die Zusammensetzung immunogen ist.

**4.** Immunogene Zusammensetzung nach Anspruch 3, die Polypetid-spezifische cytotoxische T-Zellen stimuliert.

**5.** Immunogene Zusammensetzung nach Anspruch 3, die ein Epitop umfasst, das *Theileria parva* (*T. parva*-)-spezifische cytotoxische T-Zellen stimuliert.

**6.** Impfstoff, umfassend ein oder mehrere Polypeptide nach Anspruch 1 und gegebenenfalls ein Adjuvans, wobei das Polypeptid immunogen ist.

**7.** Impfstoff nach Anspruch 6, der ein Tier vor einer *T. parva*-Infektion schützt.

**8.** Polypeptid nach Anspruch 1, das in nachweisbaren Mengen in Isolaten aus *T. parva* vorliegt.

**9.** Polypetid nach Anspruch 1, umfassend ein *T. parva*-Antigen.

**10.** Verfahren zur Herstellung eines Polypeptids, das einen für *T. parva*-Antigenspezifischen cytotoxischen Lymphocyten (CTL) stimuliert, umfassend das Züchten einer Wirtszelle, wobei die Wirtszelle einen Vektor umfasst, der ein rekombinantes Konstrukt umfasst, das ein Polynucleotid umfasst, das ein Polypeptid nach Anspruch 1 codiert, funktionell verknüpft mit einer Expressionskontrollsequenz, wobei die Wirtszelle unter Bedingungen gezüchtet wird, die für die Herstellung eines durch das Polynucleotid codierten Polypeptids günstig sind, und das Gewinnen des Polypeptids.

**11.** Antikörper, der für das Polypeptid nach Anspruch 1 spezifisch ist.

**12.** Antikörper nach Anspruch 11, der ein polyclonaler Antikörper ist.

**13.** Antikörper nach Anspruch 11, der an einen Träger und/oder eine Markierung gekoppelt ist.

**14.** Kit zum Nachweis der Anwesenheit von *T. parva* in einer Probe, von der angenommen wird, dass sie *T. parva* enthält, oder zur Aufreinigung von *T. parva* aus einer *T. parva* enthaltenden Probe, umfassend einen Antikörper nach einem der Ansprüche 11 bis 13.

**15.** Kit nach Anspruch 14, der ferner Mittel zur Durchführung eines enzymgebundenen oder Western-Blot-Assays zum Nachweis der Anwesenheit von *T. parva* umfasst.

**16.** Kit nach Anspruch 14 oder 15, der ferner Mittel zur Bindung des Antikörpers an *T. parva* in der Probe und zur Freisetzung des Organismus vom Antikörper.

**17.** Verwendung eines Polypeptids nach Anspruch 1 oder eines Vektors, der ein rekombinantes Konstrukt umfasst, das ein Polynucleotid umfasst, das ein Polypeptid nach Anspruch 1 codiert, funktionell verknüpft mit einer Expressionskontrollsequenz, oder einer Wirtszelle, die einen Vektor umfasst, der ein rekombinantes Konstrukt umfasst, das ein Polynucleotid umfasst, das ein Polypeptid nach Anspruch 1 codiert, funktionell verknüpft mit einer Expressionskontrollsequenz, für die Herstellung eines Medikaments zur Prävention und/oder Behandlung von *T. parva*-Infektion.

**18.** Verfahren zur Herstellung eines polyclonalen Antikörpers, umfassend das Immunisieren eines nicht-menschlichen Tieres mit einem oder mehreren Polypeptiden nach Anspruch 1.

**19.** Verfahren zur Herstellung eines polyclonalen Antikörpers, umfassend das Immunisieren eines nicht-menschlichen Tieres mit einer Wirtszelle; wobei die Wirtszelle einen Vektor umfasst, der ein rekombinantes Konstrukt umfasst, das ein Polynucleotid umfasst, das ein Polypeptid nach Anspruch 1 codiert, funktionell verknüpft mit einer Expressionskontrollsequenz.

**20.** Verfahren zur Herstellung eines monoclonalen Antikörpers, umfassend:

(a) Immunisieren eines nicht-menschlichen Tieres mit einem Polypeptid nach Anspruch 1,
(b) Gewinnen von Zellen aus dem nicht-menschlichen Tier, die einen Antikörper produzieren, der an das Polypeptid bindet,
(c) Herstellen eines Hybridoms mit den in (b) isolierten Zellen, und
(d) Gewinnen eines monoclonalen Antikörpers aus dem Hybridom, der an das Polypeptid in (a) bindet.

**21.** Verfahren zur Herstellung eines monoclonalen Antikörpers, umfassend:

(a) Immunisieren eines nicht-menschlichen Tieres mit einer Wirtszelle; wobei die Wirtszelle einen Vektor umfasst, der ein rekombinantes Konstrukt umfasst, das ein Polynucleotid umfasst, das ein Polypeptid nach Anspruch 1 codiert, funktionell verknüpft mit einer Expressionskontrollsequenz,
(b) Gewinnen von Zellen aus dem nicht-menschlichen Tier, die einen Antikörper produzieren, der an das von der Wirtszelle produzierte Polypeptid bindet,
(c) Herstellen von Hybridomen mit den in (b) isolierten Zellen, und
(d) Gewinnen eines monoclonalen Antikörpers aus dem Hybridom, der an das Polypeptid in (b) bindet.

**22.** Verwendung eines Antikörpers nach einem der Ansprüche 11 bis 13 bei der Herstellung einer Zusammensetzung für die Diagnose einer Infektion mit *T. parva* in einer Probe.

**23.** Impfstoff, umfassend eine Vektor, der ein rekombinantes Konstrukt umfasst, das ein Polynucleotid umfasst, das ein Polypeptid nach Anspruch 1 codiert, funktionell verknüpft mit einer Expressionskontrollsequenz.

**24.** Vektor, der ein rekombinantes Konstrukt umfasst, das ein Polynucleotid umfasst, das ein Polypeptid nach Anspruch 1 codiert, funktionell verknüpft mit einer Expressionskontrollsequenz.

**25.** Vektor nach Anspruch 24, wobei der Vektor ein bakterielles Plasmid, ein Bakteriophage, ein Hefeepisom, ein chromosomales Hefeelement, ein künstliches Hefechromosom, ein viraler Vektor, ein Baculovirus, ein Papovavirus, SV40, ein Vaccinia-Virus, ein modifizierter Vaccinia-Virus des Ankara-Stamms (MVA), ein Adenovirus, ein Pockenvirus, ein Geflügelpockenvirus (FP9), ein Kanarienpockenvirus, ein Pseudorabies-Virus oder ein Retrovirus ist.

## Revendications

**1.** Polypeptide isolé comprenant une séquence représentée par SEQ ID NO:1, ou sa variante présentant plus de 90 % d'identité par rapport à celle-ci et présentant la même activité biologique ou une activité biologique similaire à celle de SEQ ID NO:1, ou l'une quelconque des SEQ ID n°4 à 7.

**2.** Composition pharmaceutique, comprenant un polypeptide selon la revendication 1 et un vecteur pharmaceutiquement acceptable.

**3.** Composition immunogène, comprenant un polypeptide selon la revendication 1 et, en option, un adjuvant, la composition étant immunogène.

**4.** Composition immunogène selon la revendication 3, qui stimule les cellules T cytotoxiques spécifiques au polypeptide.

**5.** Composition immunogène selon la revendication 3, qui comprend un déterminant antigénique qui stimule les cellules T cytotoxiques spécifiques au *Theileria parva (T. parva*).

**6.** Vaccin, comprenant un ou plusieurs polypeptides selon la revendication 1 et, en option, un adjuvant ; dans lequel ledit polypeptide est immunogène.

**7.** Vaccin selon la revendication 6, qui protège un animal contre l'infection *T. parva.*

**8.** Polypeptide selon la revendication 1, qui est présent en quantités détectables dans des isolats de *T. parva.*

**9.** Polypeptide selon la revendication 1, comprenant un antigène de *T. parva.*

**10.** Procédé de production d'un polypeptide qui stimule un lymphocyte cytotoxique (LC) spécifique à l'antigène T. parva, comprenant l'étape consistant à faire une culture d'une cellule hôte, dans laquelle ladite cellule hôte comprend un vecteur comprenant une construction recombinante comprenant un polynucléotide encodant un polypeptide selon la revendication 1, lié de manière opérationnelle à une séquence de contrôle d'expression, dans lequel ladite cellule hôte est cultivée dans des conditions efficaces pour produire un polypeptide encodé par le polynucléotide, et récolter le polypeptide.

**11.** Anticorps spécifique pour le polypeptide selon la revendication 1.

**12.** Anticorps selon la revendication 11, qui est un anticorps polyclonal.

**13.** Anticorps selon la revendication 11, qui est couplé à un vecteur et/ou un traceur.

**14.** Kit de détection de la présence de *T. parva* dans un échantillon suspecté de contenir *T. parva,* ou de purification de *T. parva* à partir d'un échantillon contenant *T. parva*, comprenant un anticorps selon l'une quelconque des revendications 11 à 13.

**15.** Kit selon la revendication 14, qui comprend en outre des moyens destinés à mettre en oeuvre une méthode de dosage d'immunosorption liée à enzyme ou une analyse par buvardage de western pour détecter la présence de *T. parva.*

**16.** Kit selon la revendication 14 ou 15, qui comprend en outre des moyens destinés à lier l'anticorps à *T. parva* dans l'échantillon, et à libérer l'organisme de l'anticorps.

**17.** Utilisation d'un polypeptide selon la revendication 1, ou d'un vecteur comprenant une construction recombinante comprenant un polynucléotide encodant un polypeptide selon la revendication 1, lié de manière opérationnelle à une séquence de contrôle d'expression, ou une cellule hôte comprenant un vecteur comprenant une construction recombinante comprenant un polynucléotide encodant un polypeptide selon la revendication 1, lié de manière opérationnelle à une séquence de contrôle d'expression, dans la préparation d'un médicament destiné à prévenir et/ou traiter une infection par *T. parva.*

**18.** Procédé de préparation d'un anticorps polyclonal, comprenant l'étape consistant à immuniser un animal non humain avec un ou plusieurs polypeptides de la revendication 1.

**19.** Procédé de préparation d'un anticorps polyclonal, comprenant l'étape consistant à immuniser un animal non humain avec une cellule hôte ; dans lequel ladite cellule hôte comprend un vecteur comprenant une construction recombinante comprenant un polynucléotide encodant un polypeptide selon la revendication 1, lié de manière opérationnelle à une séquence de contrôle d'expression.

**20.** Procédé de préparation d'un anticorps monoclonal, comprenant les étapes consistant à :

(a) immuniser un animal non humain avec un polypeptide de la revendication 1,
(b) récupérer les cellules de l'animal non humain qui produisent l'anticorps qui se lie au polypeptide ;
(c) préparer un hybridome avec les cellules isolées au cours de l'étape (b), et

(d) récupérer un anticorps monoclonal à partir de l'hybridome qui se lie au polypeptide au cours de l'étape (a).

**21.** Procédé de préparation d'un anticorps monoclonal, comprenant les étapes consistant à :

(a) immuniser un animal non humain avec une cellule hôte ; dans lequel ladite cellule hôte comprend un vecteur comprenant une construction recombinante comprenant un polynucléotide encodant un polypeptide selon la revendication 1, lié de manière opérationnelle à une séquence de contrôle d'expression,
(b) récupérer les cellules de l'animal non humain qui produisent l'anticorps qui se lie à un polypeptide produit par la cellule hôte,
(c) préparer des hybridomes avec les cellules isolées au cours de l'étape (b), et
(d) récupérer un anticorps monoclonal à partir de l'hybridome qui se lie au polypeptide au cours de l'étape (b).

**22.** Utilisation d'un anticorps selon l'une quelconque des revendications 11 à 13 dans la préparation d'une composition pour le diagnostic de l'infection par *T. parva* dans un échantillon.

**23.** Vaccin comprenant un vecteur comprenant une construction recombinante comprenant un polynucléotide encodant un polypeptide selon la revendication 1, lié de manière opérationnelle à une séquence de contrôle d'expression.

**24.** Vecteur comprenant une construction recombinante comprenant un polynucléotide encodant un polypeptide selon la revendication 1, lié de manière opérationnelle à une séquence de contrôle d'expression.

**25.** Vecteur selon la revendication 24 dans lequel ledit vecteur est un plasmide bactérien, un bactériophage, un épisome de levure, un élément chromosomique de levure, un chromosome artificiel de levure, un vecteur viral, un baculovirus, un virus du groupe Papova, SV40, un virus de la vaccine, la souche du virus de la vaccine modifié d'Ankara (MVA), un adénovirus, un poxvirus, un virus de la variole aviaire (FP9), une variole du canari, un virus de la pseudo-rage, ou un rétrovirus.

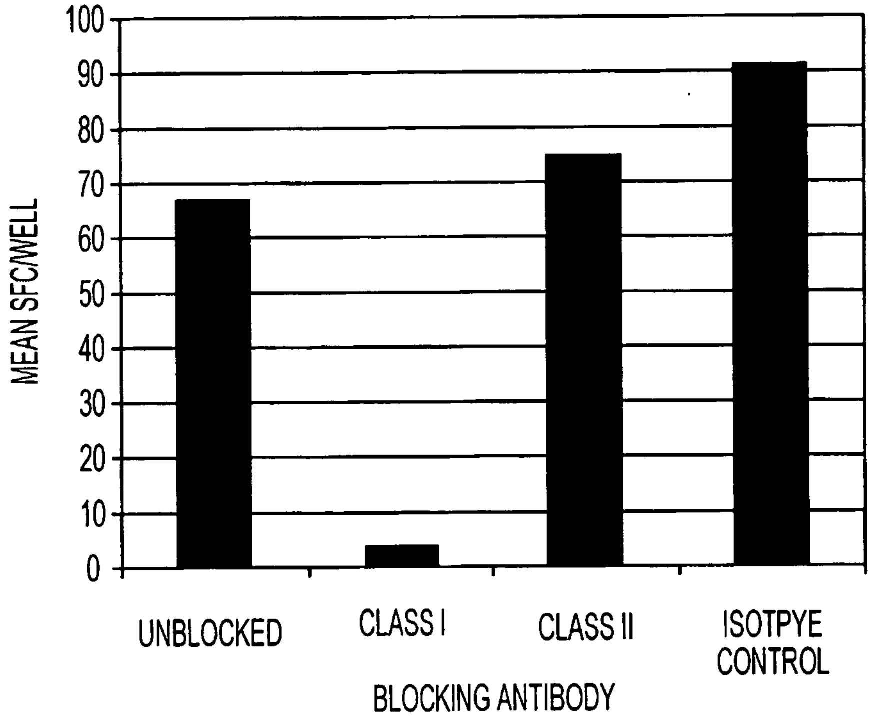
100
90
80
70
60
50
40
30
20
10
0
MEAN SFC/WELL
UNBLOCKED
CLASS I
CLASS II
ISOTPYE CONTROL
BLOCKING ANTIBODY

FIG. 1

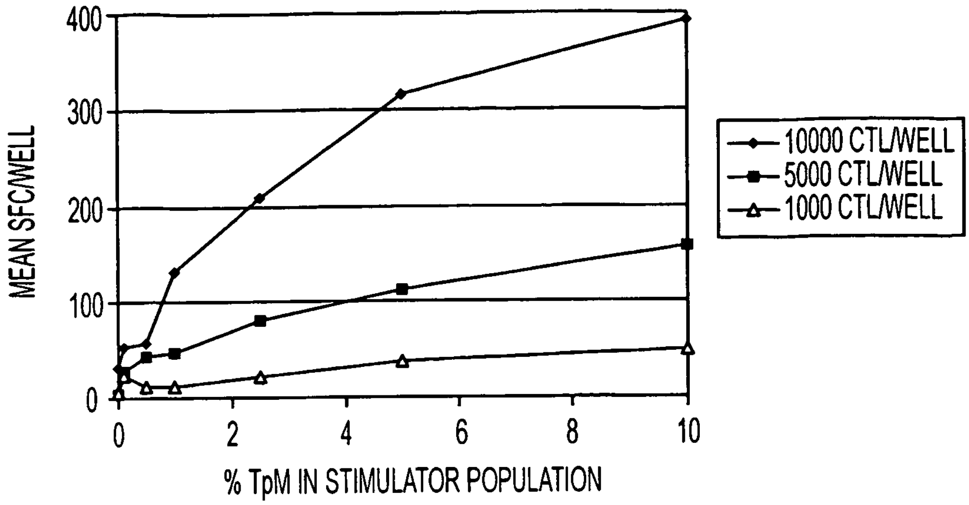


FIG. 2A

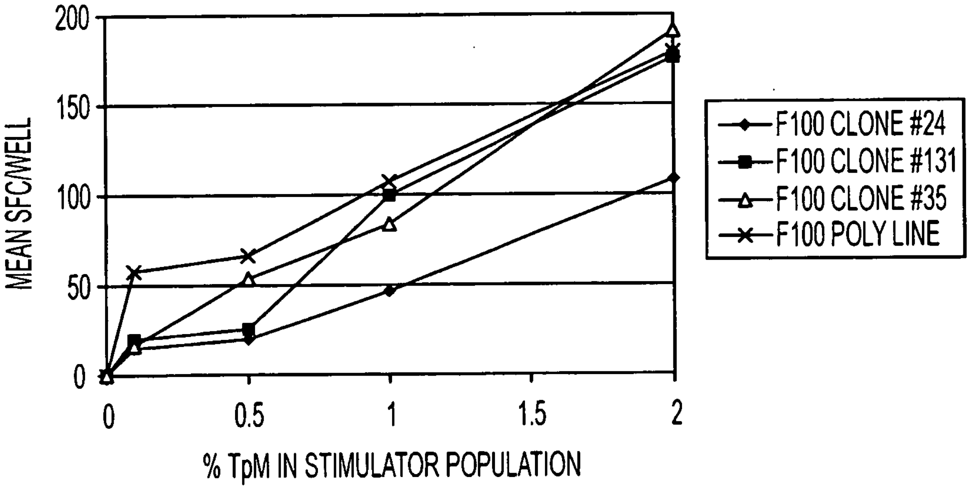


FIG. 2B

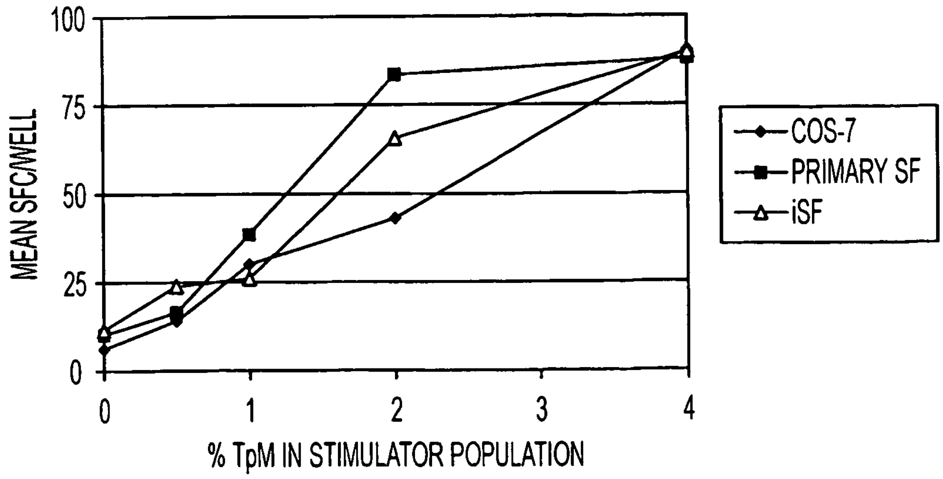


FIG. 2C

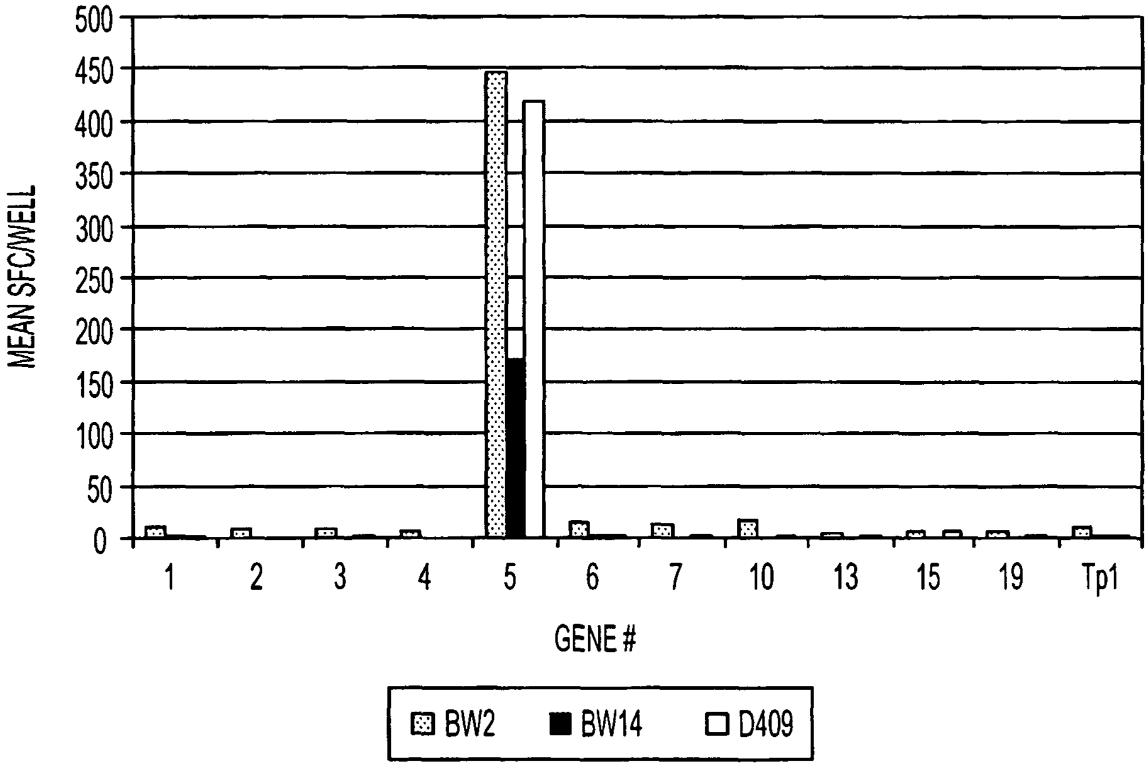
500
450
400
350
300
250
200
150
100
50
0
MEAN SFC/WELL
1
2
3
4
5
6
7
10
13
15
19
Tp1
GENE #
BW2
BW14
D409

FIG. 3

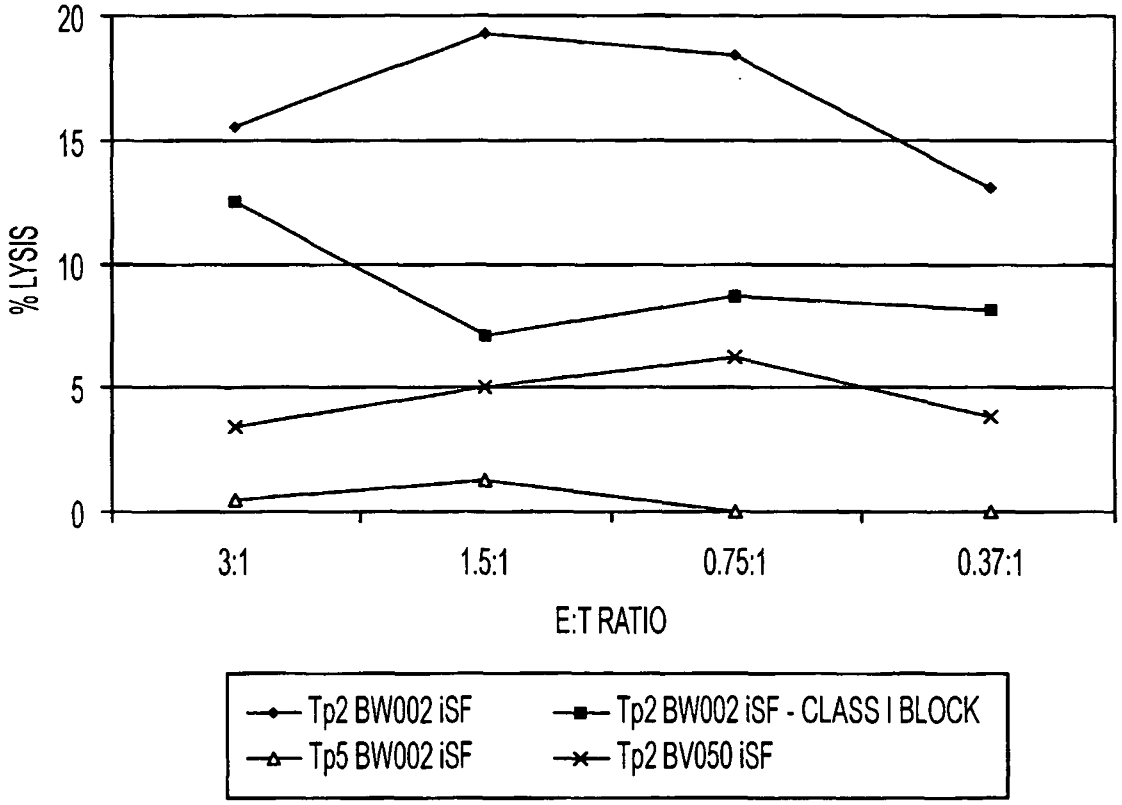
20
15
10
5
0
% LYSIS
3:1
1.5:1
0.75:1
0.37:1
E:T RATIO
Tp2 BW002 iSF
Tp2 BW002 iSF - CLASS I BLOCK
Tp5 BW002 iSF
Tp2 BV050 iSF

FIG. 4

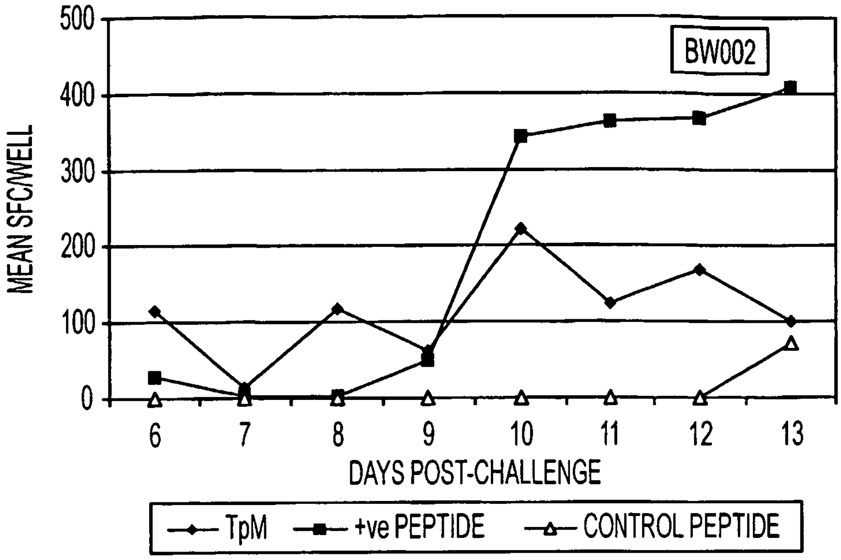


FIG. 5A

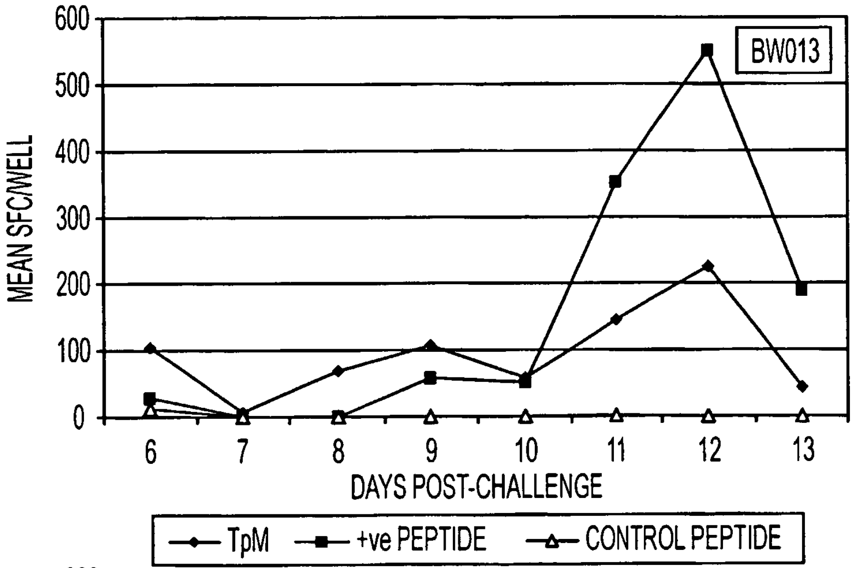


FIG. 5B

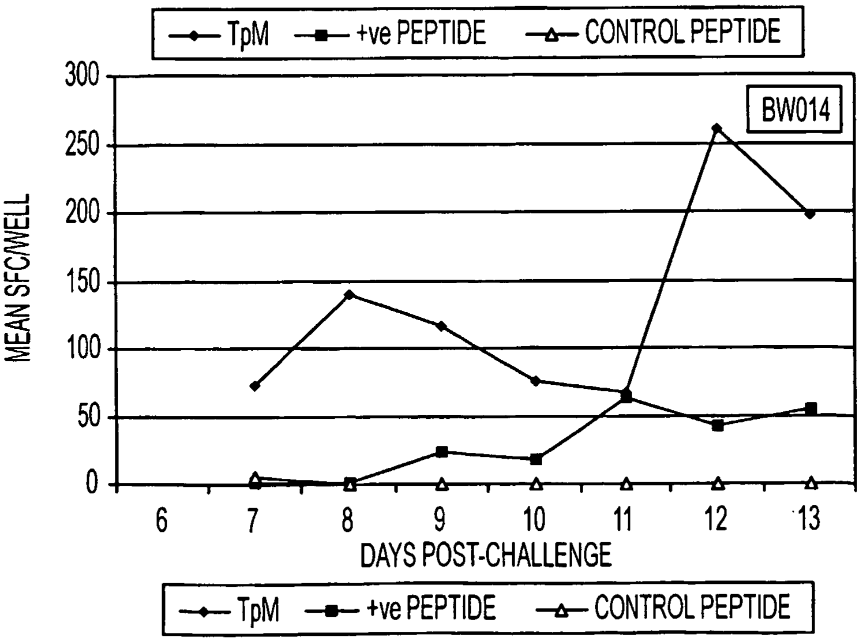


FIG. 5C

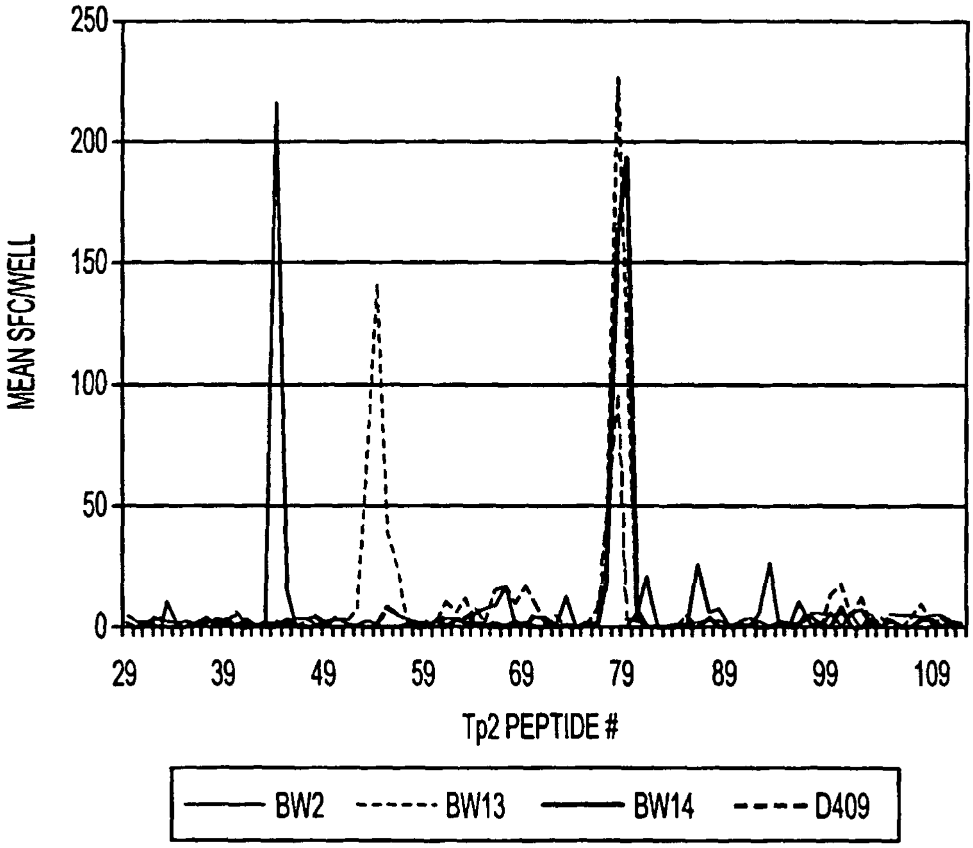
250
200
150
100
50
0
MEAN SFC/WELL
29
39
49
59
69
79
89
99
109
Tp2 PEPTIDE #
BW2
BW13
BW14
D409

FIG. 6

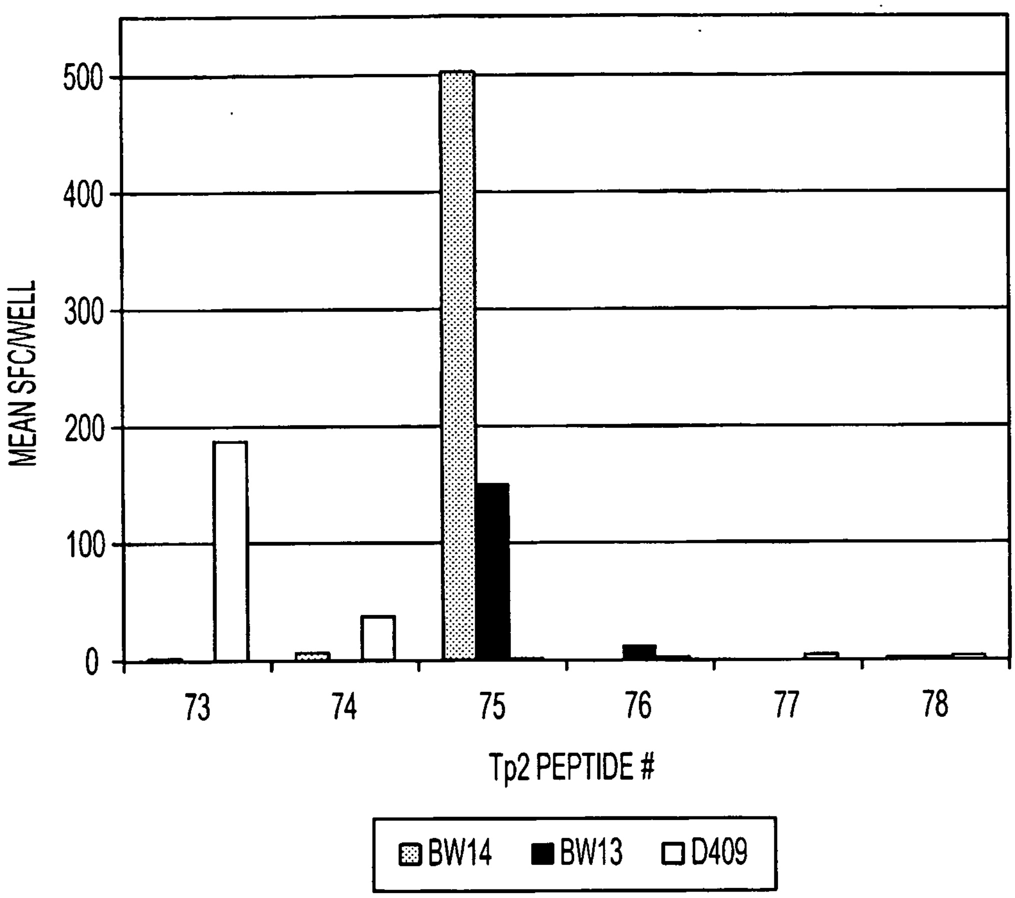
MEAN SFC/WELL
500
400
300
200
100
0
73
74
75
76
77
78
Tp2 PEPTIDE #
BW14
BW13
D409

FIG. 7

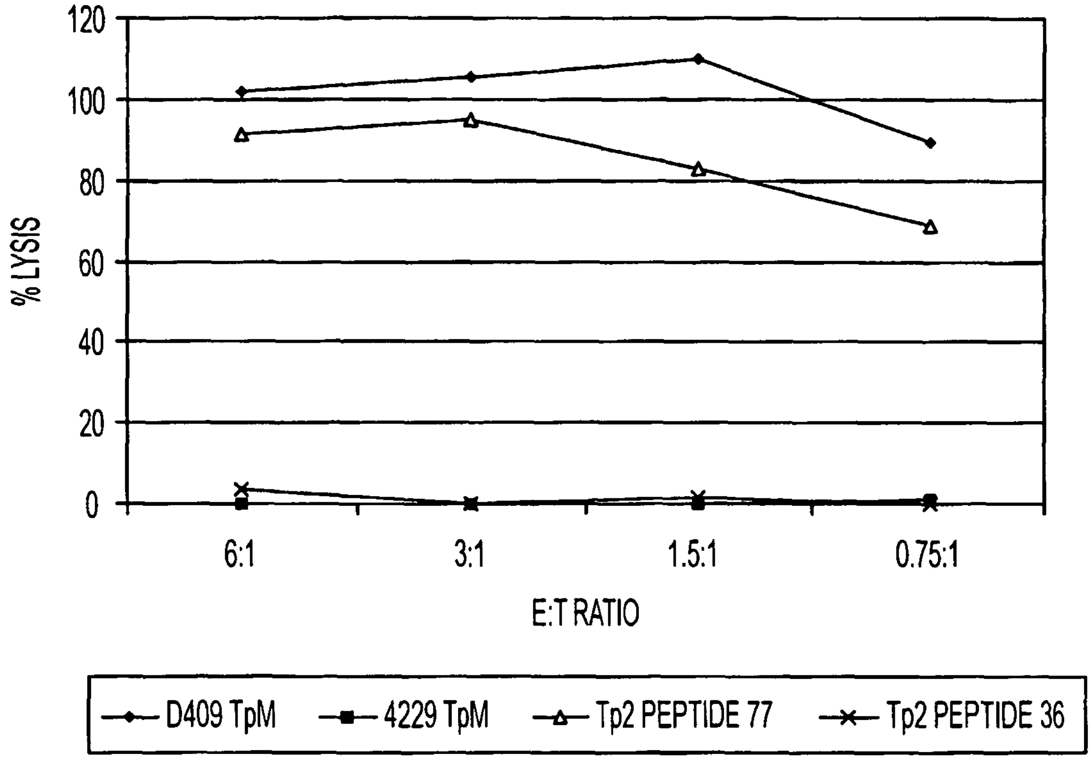
120
100
80
60
40
20
0
% LYSIS
6:1
3:1
1.5:1
0.75:1
E:T RATIO
D409 TpM
4229 TpM
Tp2 PEPTIDE 77
Tp2 PEPTIDE 36

FIG. 8

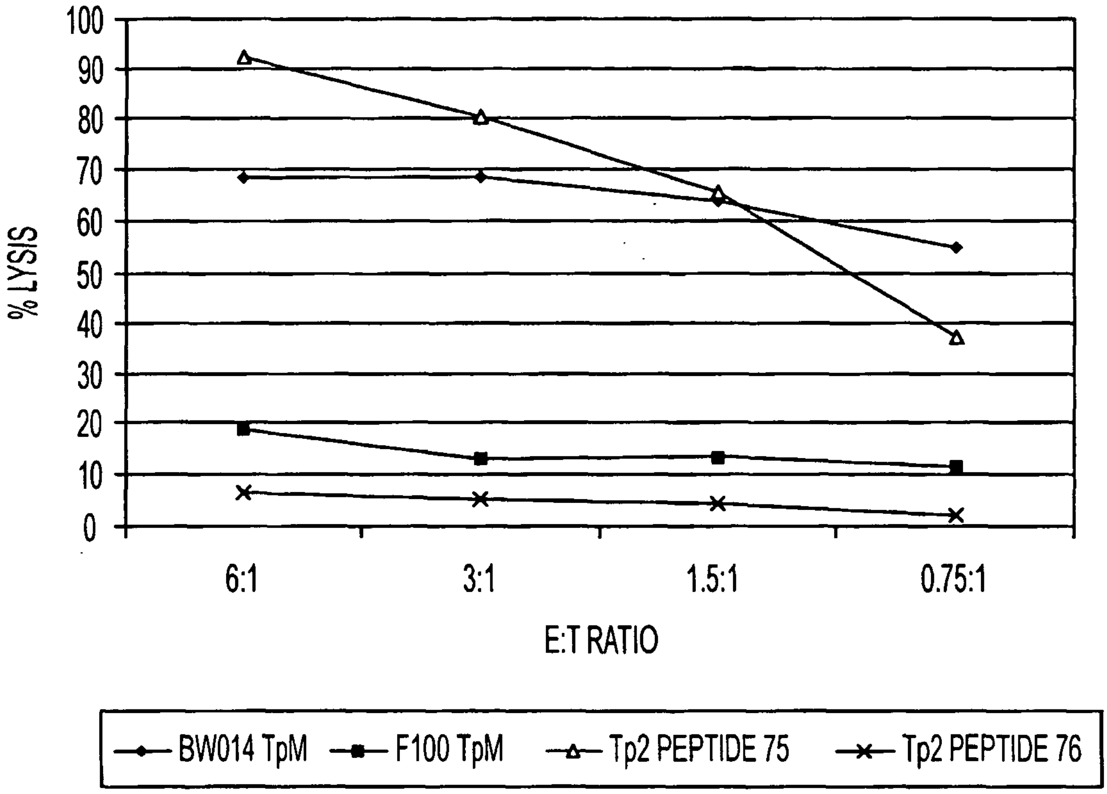
100
90
80
70
60
50
40
30
20
10
0
% LYSIS
6:1
3:1
1.5:1
0.75:1
E:T RATIO
BW014 TpM
F100 TpM
Tp2 PEPTIDE 75
Tp2 PEPTIDE 76

FIG. 9

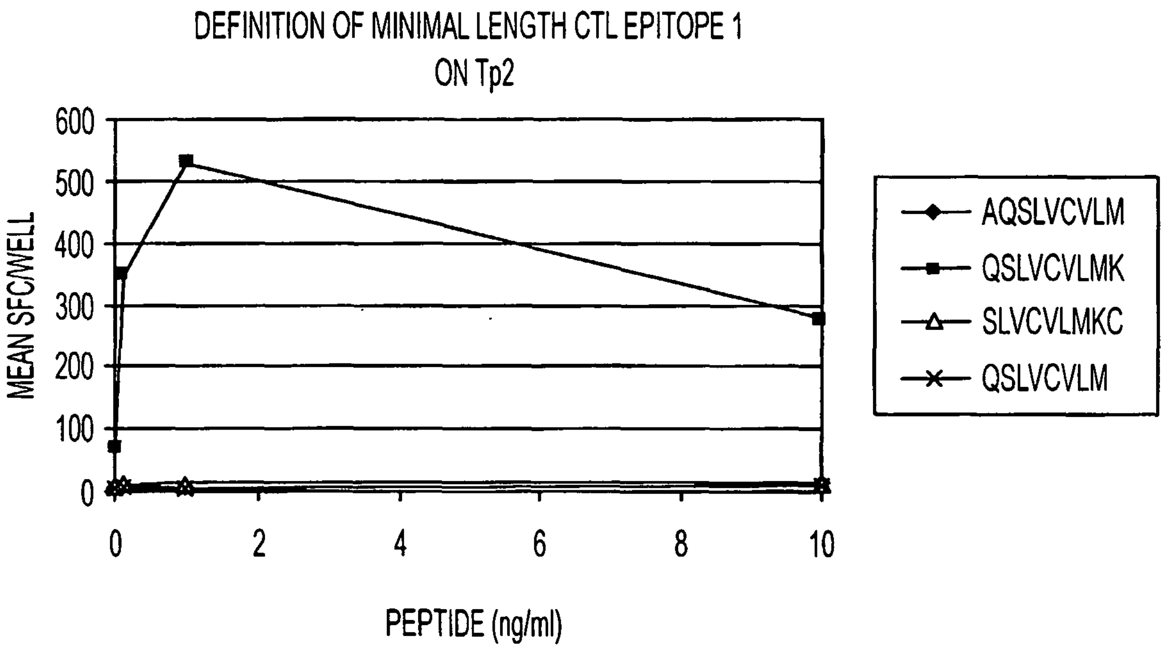
DEFINITION OF MINIMAL LENGTH CTL EPITOPE 1
ON Tp2
MEAN SFC/WELL
600
500
400
300
200
100
0
0
2
4
6
8
10
PEPTIDE (ng/ml)
AQSLVCVLM
QSLVCVLMK
SLVCVLMKC
QSLVCVLM

FIG. 10

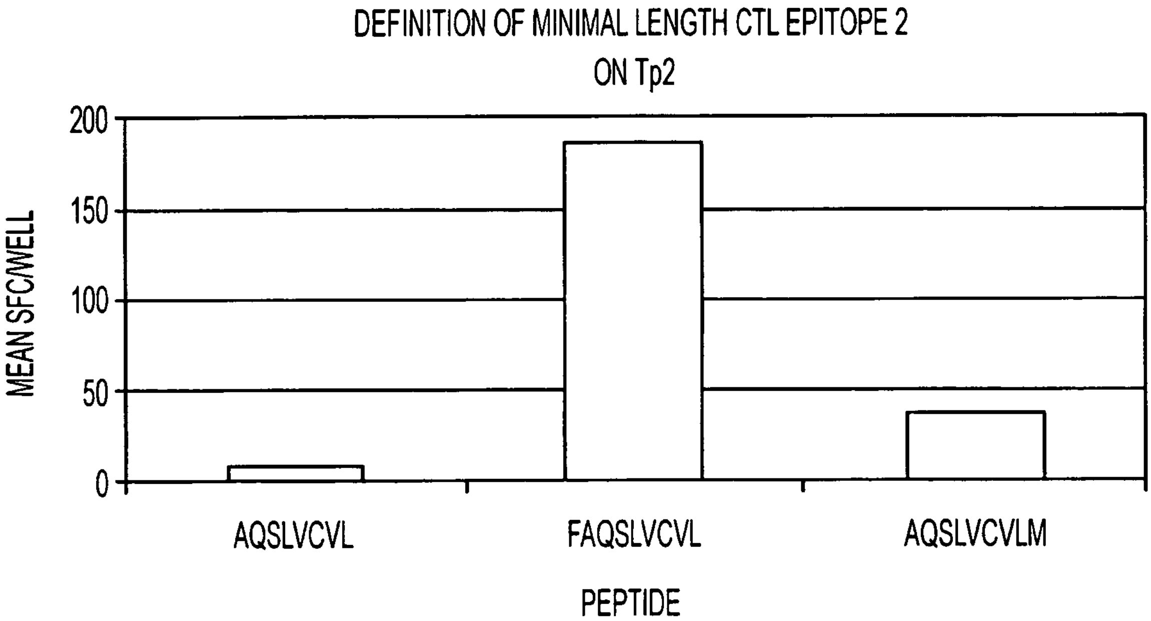
DEFINITION OF MINIMAL LENGTH CTL EPITOPE 2
ON Tp2
MEAN SFC/WELL
200
150
100
50
0
AQSLVCVL
FAQSLVCVL
AQSLVCVLM
PEPTIDE

FIG. 11

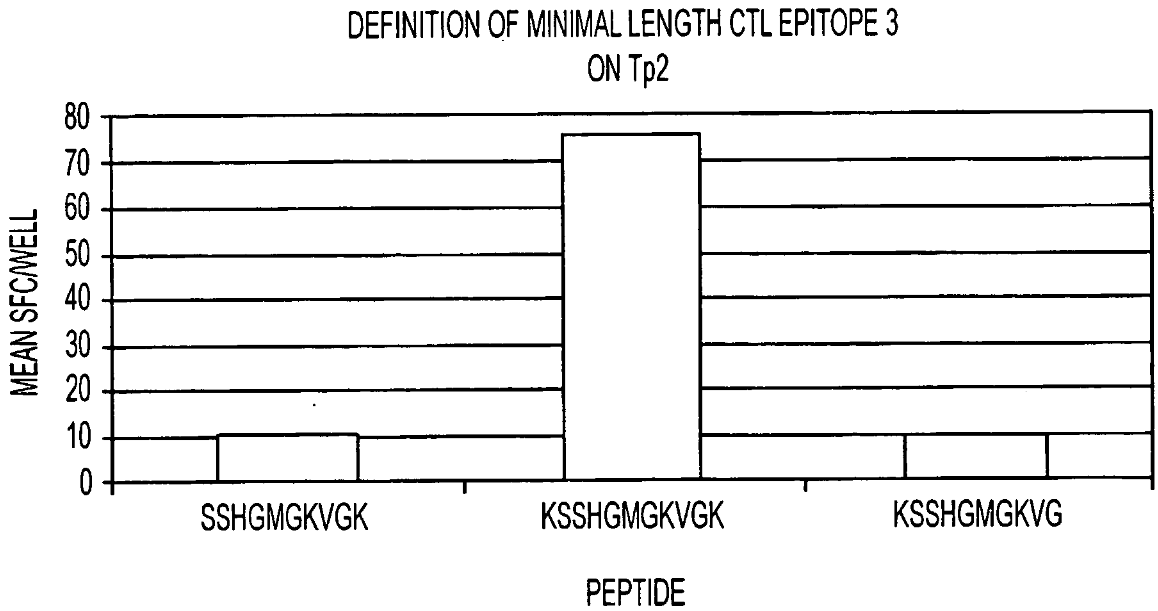
DEFINITION OF MINIMAL LENGTH CTL EPITOPE 3
ON Tp2
MEAN SFC/WELL
0
10
20
30
40
50
60
70
80
SSHGMGKVGK
KSSHGMGKVGK
KSSHGMGKVG
PEPTIDE

FIG. 12

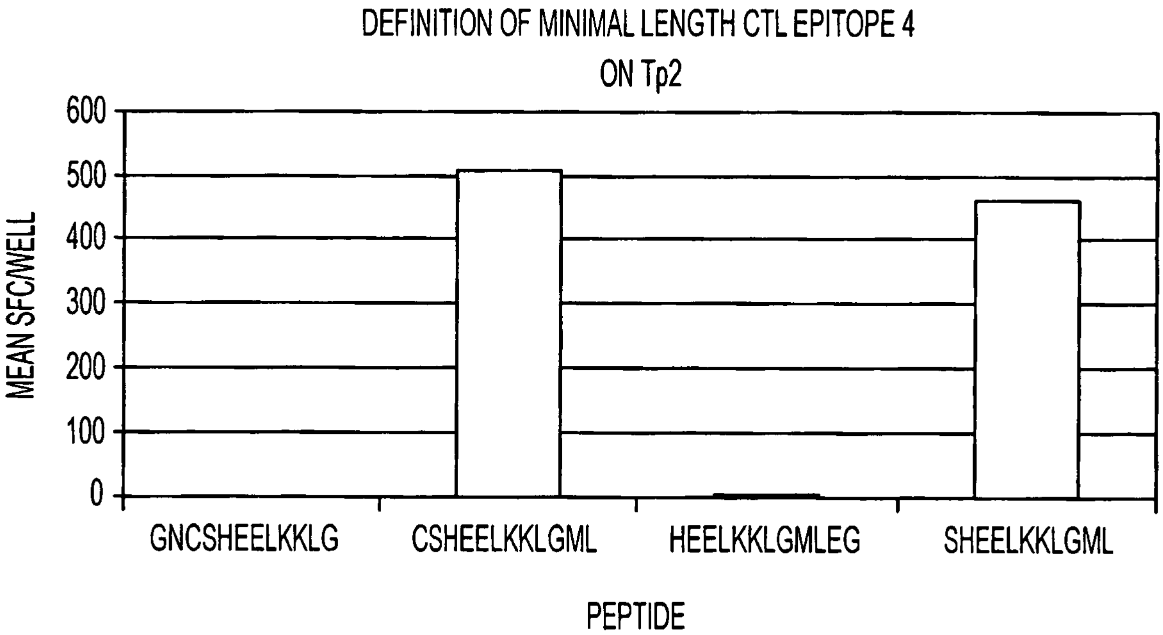
DEFINITION OF MINIMAL LENGTH CTL EPITOPE 4
ON Tp2
MEAN SFC/WELL
600
500
400
300
200
100
0
GNCSHEELKKLG
CSHEELKKLGML
HEELKKLGMLEG
SHEELKKLGML
PEPTIDE

FIG. 13

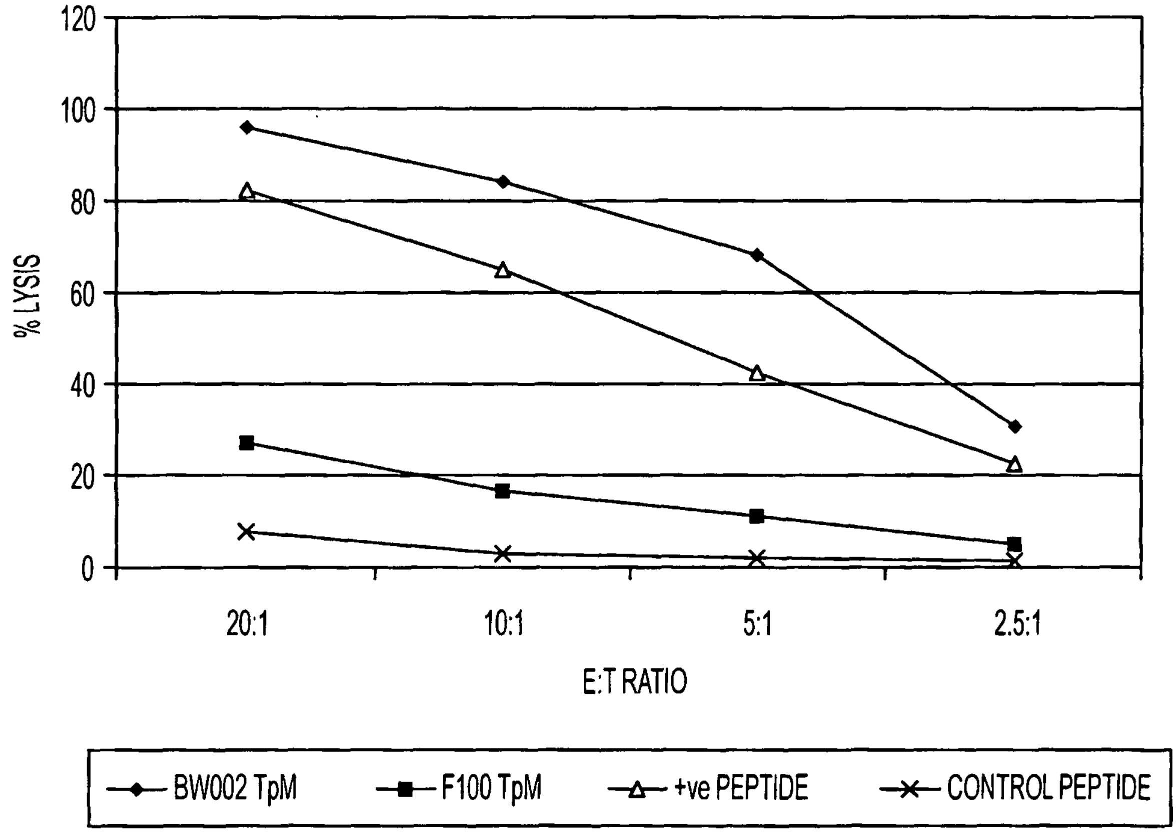
120
100
80
60
40
20
0
% LYSIS
20:1
10:1
5:1
2.5:1
E:T RATIO
BW002 TpM
F100 TpM
+ve PEPTIDE
CONTROL PEPTIDE

FIG. 14

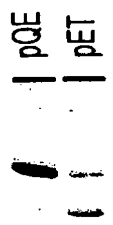


COOMASSIE

FIG. 15A

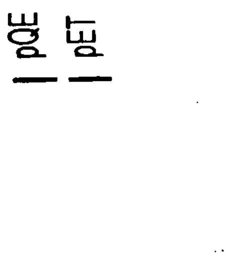


ANTI-HIS-TAG

FIG. 15B

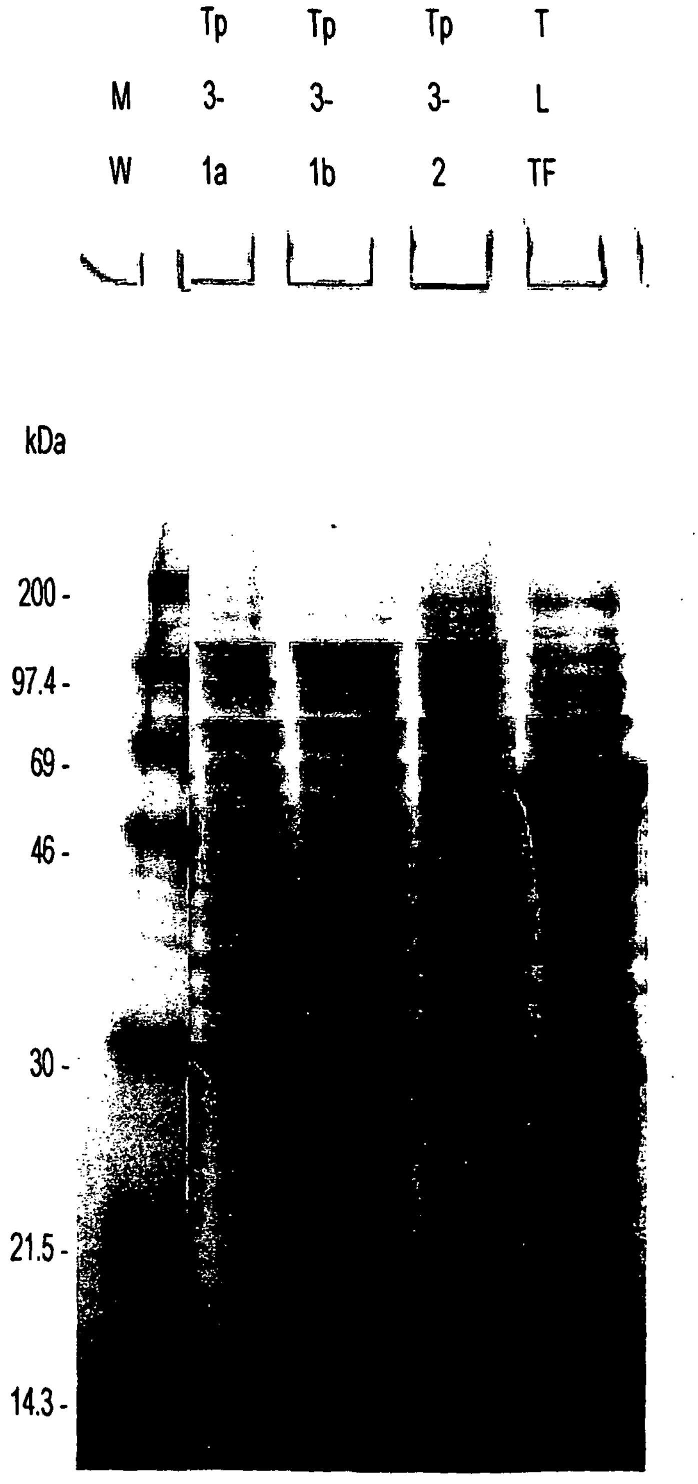


FIG. 16

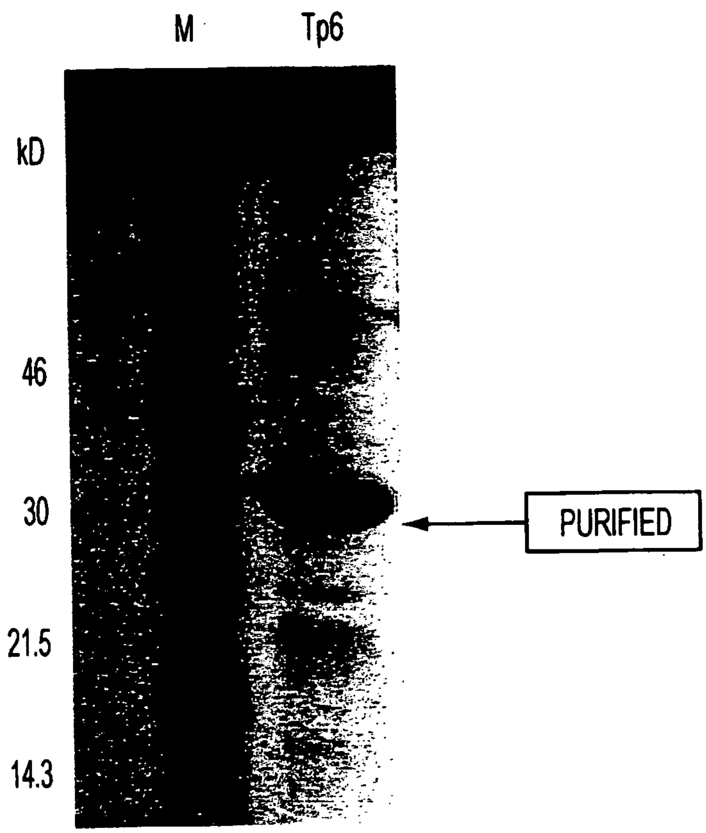


FIG. 17

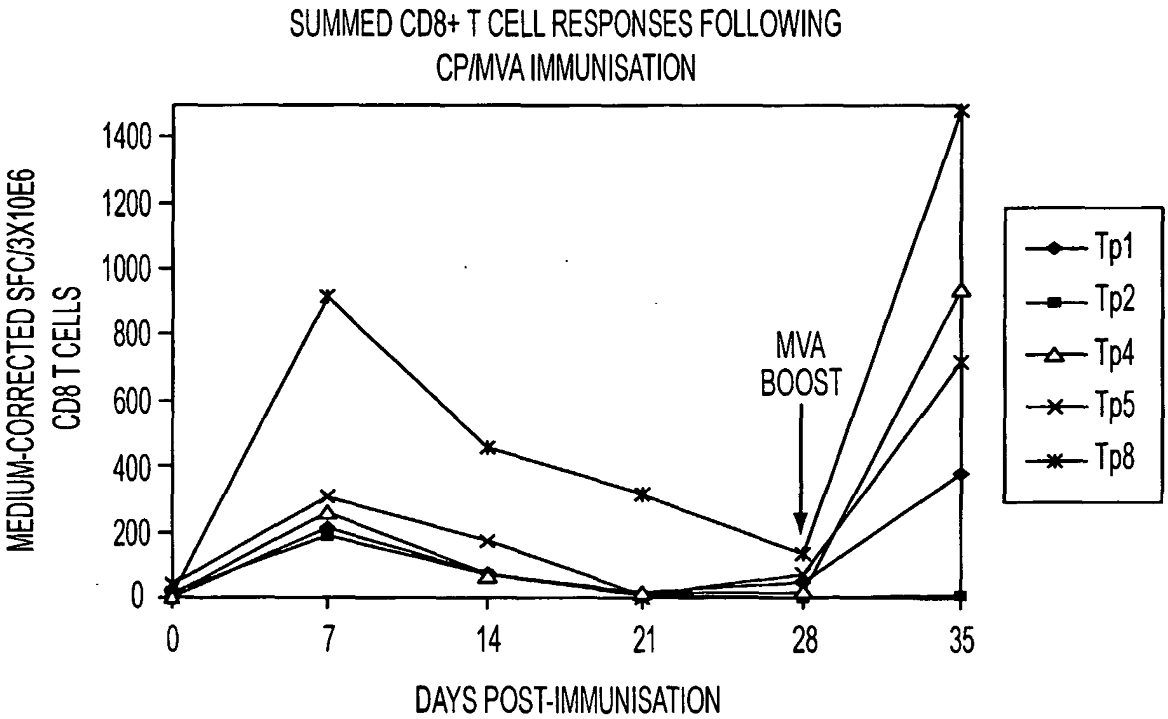
SUMMED CD8+ T CELL RESPONSES FOLLOWING
CP/MVA IMMUNISATION
MEDIUM-CORRECTED SFC/3X10E6
CD8 T CELLS
1400
1200
1000
800
600
400
200
0
0
7
14
21
28
35
DAYS POST-IMMUNISATION
MVA
BOOST
Tp1
Tp2
Tp4
Tp5
Tp8

FIG. 18

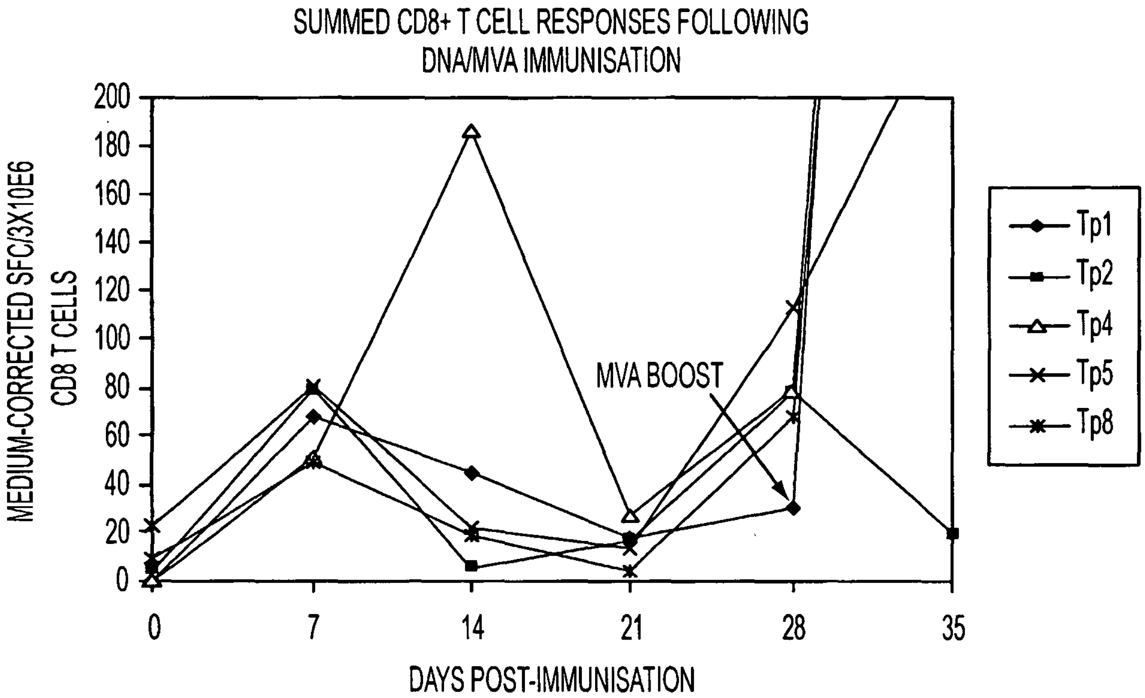
SUMMED CD8+ T CELL RESPONSES FOLLOWING
DNA/MVA IMMUNISATION
MEDIUM-CORRECTED SFC/3X10E6
CD8 T CELLS
200
180
160
140
120
100
80
60
40
20
0
0
7
14
21
28
35
DAYS POST-IMMUNISATION
MVA BOOST
Tp1
Tp2
Tp4
Tp5
Tp8

FIG. 19

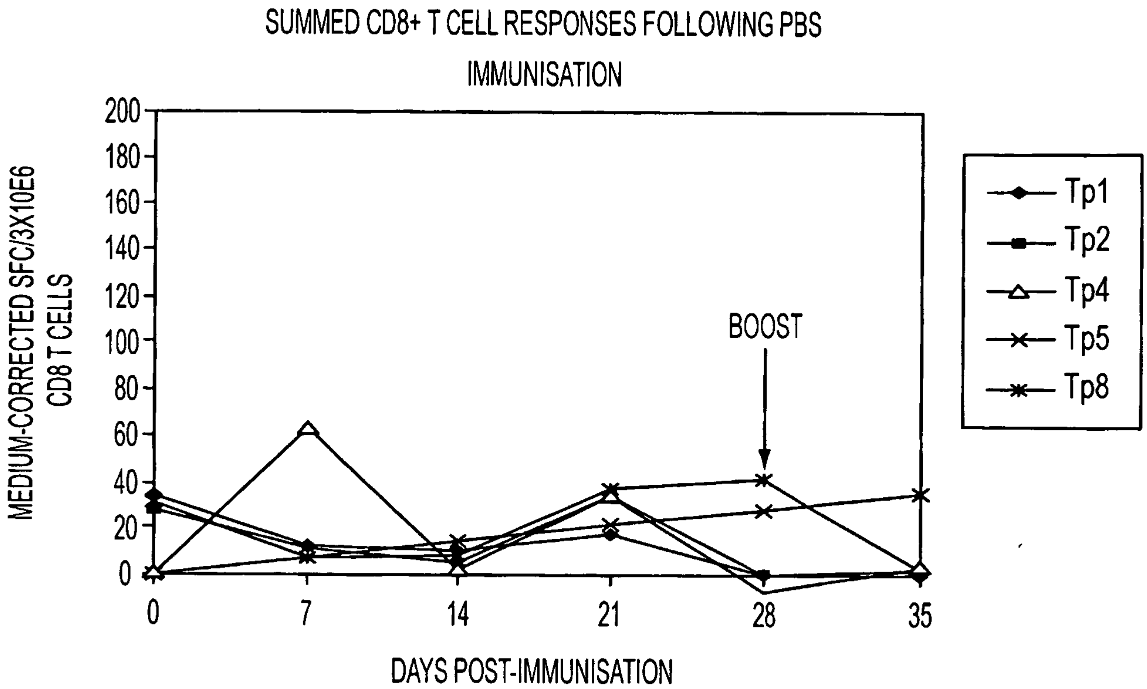
SUMMED CD8+ T CELL RESPONSES FOLLOWING PBS IMMUNISATION
MEDIUM-CORRECTED SFC/3X10E6 CD8 T CELLS
200
180
160
140
120
100
80
60
40
20
0
0
7
14
21
28
35
DAYS POST-IMMUNISATION
BOOST
Tp1
Tp2
Tp4
Tp5
Tp8

FIG. 20

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 5273744 A
- WO 9316094 A
- US 4683195 A
- US 4683202 A
- US 4800159 A
- US 5837832 A, Chee
- WO 9511995 A, Chee
- US 5807522 A, Brown
- WO 95251116 A, Baldeschweiler
- US 60504428 B


### Non-patent literature cited in the description


- **SHARMA et al.** *Vet. Parasito/.,* vol. 79, 135041
- **HOLDER et al.** *Nature,* 1981, vol. 294, 361
- **MAJARIAN et al.** *J. Immunol.,* 1984, vol. 132, 3131
- **LEW et al.** *Proc. Natl. Acad. Sci. USA,* 1989, vol. 86, 3768
- **MCKEEVER ; MORRISION.** *International Journal for Parasitology,* 1998, vol. 28, 693-706
- **GRIBSKOV ; DEVEREUS.** Sequence Analysis Primer. Stockton Press, 1991
- **SAMBROOK J. et al.** Molecular Cloning: A Laboratory Manual. Cold Spring Harbor Laboratory, 1989
- **AUSUBEL F. M. et al.** Current Protocols in Molecular Biology. John Wiley & Sons, 1989
- **GOEDDEL.** Gene Expression Technology, Methods and Enzymology. Academic Press, 1990, vol. 185
- **DUNACHIE et al.** *J Exp Biol,* 2003, vol. 206, 3771-9
- **KELLER, G. H. ; M. M. MANAK.** DNA Probes. Stockton Press, 1987, 169-170
- **SAMBROOK et al.** Molecular Cloning: A Laboratory Manual. Cold Spring Harbor Laboratory Press, 1989, vol. 1, 2, 3
- **BELTZ et al.** *Methods Enzymol,* 1983, vol. 100, 266-85
- **SAIKI et al.** *Science,* 1985, vol. 230, 1350-4
- **GOBINDA et al.** *PCR Methods Applic,* 1993, vol. 2, 318-22
- **TRIGLIA T. et al.** *Nucleic Acids Res,* 1988, vol. 16, 8186
- **SAMBROOK et al.** Molecular Cloning: A Laboratory Manual. Cold Spring Harbor Laboratory Press, 1989
- **SAMBROOK et al.** Molecular Cloning: A Laboratory Manual. Cold Spring Harbor Laboratory Press, 1989
- **SMITH et al.** *Gene,* 1988, vol. 67, 31-40
- **AMANN et al.** *Gene,* 1988, vol. 69, 301-315
- **STUDIER et al.** *Gene Expression Technology: Methods in Enzymology,* 1990, vol. 185, 60-89
- **GOTTESMAN, S.** Gene Expression Technology: Methods in Enzymology. Academic Press, 1990, vol. 185, 119-128
- **WADA et al.** *Nucleic Acids Res.,* 1992, vol. 20, 2111-2118
- **BALDARI et al.** *EMBO J.,* 1987, vol. 6, 229-234
- **KUJAN et al.** *Cell,* 1982, vol. 30, 933-943
- **SCHULTZ et al.** *Gene,* 1987, vol. 54, 113-123
- **SMITH et al.** *Mol. Cell Biol.,* 1983, vol. 3, 2156-2165
- **LUCKLOW et al.** *Virology,* 1989, vol. 170, 31-39
- **SEED, B.** *Nature,* 1987, vol. 329, 840
- **KAUFMAN et al.** *EMBO J.,* 1987, vol. 6, 187-195
- **SAMBROOK, J. ; FRITSH, E. F. ; MANIATIS, T.** Molecular Cloning: A Laboratory Manual. Cold Spring Harbor Laboratory Press, 1989
- **CARTER et al.** *Nucl. Acids Res.,* 1986, vol. 13, 4331
- **ZOLLER.** *Nucl. Acids Res.,* 1987, vol. 10, 6487
- **WELLS et al.** *Gene,* 1985, vol. 34, 315
- **WELLS et al.** *Philos. Trans. R. Soc. London SerA,* 1986, vol. 317, 415
- **CUNNINGHAM ; WELLS.** *Science,* 1989, vol. 244, 1081-1085
- **CREIGHTON.** The Proteins. W.H. Freeman & Co,
- **CHOTHIA.** *J. Mol. Biol,* 1976, vol. 150, 1
- **SALZBURG et al.** *NAR,* 1998, vol. 26, 544
- **DELCHER et al.** *NAR,* 1999, vol. 27, 4636
- **PERTEA, M. ; SALZBERG, S.L.** *Current Protocols in Bioinformatics,* 2002

- **S.L. SALZBERG ; M. PERTEA ; A.L. DELCHER ; M.J. GARDNER ; H. TETTELIN.** Interpolated Markov models for eukaryotic gene finding. *Genomics,* 1999, vol. 59, 24-31
- **J. E. ALLEN ; M. PERTEA ; S. L. SALZBERG.** *Genome Research,* 2004, vol. 14 (1
- **T. E. CREIGHTON.** Proteins-Structure and Molecular Properties. W. H. Freeman and Company, 1993
- **WOLD, F.** Posttranslational Covalent Modification of Proteins. Academic Press, 1983, 1-12
- **SEIFTER et al.** *Meth. Enzymol.,* 1990, vol. 182, 626-646
- **RATTAN et al.** *Ann. N.Y Acad. Sci.,* 1992, vol. 663, 48-62
- **ORLANDI R. et al.** *PNAS,* 1989, vol. 86, 3833-3837
- **HUSE W. D. et al.** *Science,* 1989, vol. 256, 1275-1281
- **WINTER G. ; MILSTEIN C.** *Nature,* 1991, vol. 349, 293-299
- **HARLOW.** Antibodies. Cold Spring Harbor Press, 1989
- Molecular Cloning: A Laboratory Manual. Cold Spring Harbor Laboratory Press, 1989
- Methods in Enzymology. Guide to Molecular Cloning Techniques. Academic Press, 1997
- **LOCKHART, D. J. et al.** *Nat. Biotech.,* 1996, vol. 14, 1675-1680
- **SCHENA, M. et al.** *Proc. Natl. Acad. Sci.,* 1996, vol. 93, 10614-10619
- **CHARD, T.** An Introduction to Radioimmunoassay and Related Techniques. Elsevier Science Publishers, 1986
- **BULLOCK, G. R. et al.** Techniques in Immunocytochemistry. Academic Press, 1982, vol. 1
- TECHNIQUES IN IMMUNOCYTOCHEMISTRY. 1983, vol. 2
- TECHNIQUES IN IMMUNOCYTOCHEMISTRY. 1985, vol. 3
- **TIJSSEN, P.** Practice and Theory of Enzyme Immunoassays: Laboratory Techniques in Biochemistry and Molecular Biology. Elsevier Science Publishers, 1985
- **RADLEY et al.** *Vet Rec,* 1975, vol. 96, 525-7
- **BYROM ; YUNKER.** *Cytotechnology,* 1990, vol. 4, 285-90
- **MWANGI et al.** *Vet Parasitol.,* 1998, vol. 79, 1-17
- **NIELSEN et al.** *Int J Neural Syst.,* 1997, vol. 8, 581-99

SEQUENCE LISTING

<110> INTERNATIONAL LIVESTOCK RESEARCH INSTITUTE THE INSTITUTE FOR GENOMIC RESEARCH

<120> EAST COAST FEVER VACCINE BASED ON CTL-SPECIFIC SCHIZONT ANTIGENS

<130> 41860-205199

<140>
<141>

<150> 60/504,428
<151> 2003-09-22

<160> 53

<170> PatentIn Ver. 3.2

<210> 1
<211> 174
<212> PRT
<213> Theileria parva

<220>
<223> Amino acid sequence of Tp2:

<400> 1

```
Met Lys Leu Ala Ala Arg Leu Ile Ser Leu Tyr Phe Ile Ile Tyr Ile
 1               5                  10                  15

Leu His Ser Pro Val Leu Gly Gly Asn Cys Ser His Glu Glu Leu Lys
            20                  25                  30

Lys Leu Gly Met Leu Glu Gly Asp Gly Phe Asp Arg Asp Ala Leu Phe
        35                  40                  45

Lys Ser Ser His Gly Met Gly Lys Val Gly Lys Arg Tyr Gly Leu Lys
    50                  55                  60

Thr Thr Pro Lys Val Asp Lys Val Leu Ala Asp Leu Glu Thr Leu Phe
65                  70                  75                  80

Gly Lys His Gly Leu Gly Gly Ile Ser Lys Asp Cys Leu Lys Cys Phe
                85                  90                  95

Ala Gln Ser Leu Val Cys Val Leu Met Lys Cys Arg Gly Ala Cys Leu
            100                 105                 110

Lys Gly Pro Cys Thr Asp Asp Cys Gln Asn Cys Phe Asp Arg Asn Cys
        115                 120                 125

Lys Ser Ala Leu Leu Glu Cys Ile Gly Lys Thr Ser Ile Pro Asn Pro
    130                 135                 140

Cys Lys Trp Lys Glu Asp Tyr Leu Lys Tyr Lys Phe Pro Glu Thr Asp
145                 150                 155                 160
```

Glu Asp Glu Ser Thr Lys Lys Gly Glu Ala Ser Gly Thr Ser
165 170

<210> 2
<211> 265
<212> PRT
<213> Theileria parva

<220>
<223> Amino acid sequence of Tp3

<400> 2

Met Lys Leu Asn Thr Ile Ala Ile Ala Phe Leu Tyr Ser Cys Phe Ser
1 5 10 15

Gln Phe Leu Lys Asn Val Ser Ala Leu Arg Arg Ser Ser Pro Asp Leu
20 25 30

Ser Pro Asp Gly Ser Phe Leu Gln Val Lys Ser Ala Ser Pro Gln Asp
35 40 45

Lys Gln Asp Val Ile Gln Ser Ser Ser Pro Lys Val Thr Val Pro Thr
50 55 60

Val Asp Pro Glu Gly Leu Lys Lys Ala Val Thr Ala Ala Val Leu Ser
65 70 75 80

Asn Gln Asn Gln Ala Leu Gln Asn Gly Ala Leu Asn Pro Ala Asp Phe
85 90 95

Thr Gln Ala Ala Ser Val Asn Ser Met Ser Asn Ala Val Ser Ala Met
100 105 110

Asn Asn Thr Val Gly Pro Val Lys Asn Pro Met Ala Thr Val Gly Thr
115 120 125

Met Asn Ser Phe Thr Gly Met Pro Gly Val Gln Asp Asn Phe Pro Gln
130 135 140

Thr Pro Pro Val Asn Val Gln Asp Thr Ser Thr Gln Glu Asn Ser Leu
145 150 155 160

Asp Asn Leu Asn Leu Leu Leu Asp Pro Ser Leu Val Lys Ile Ser Gln
165 170 175

Ala Asp Ser His Ile Lys Glu Ser Met Glu Lys Ala Val His Ser Leu
180 185 190

Lys Lys Val Leu Glu Gly Leu Thr Asn Leu Ala Thr Leu Ser Lys Ser
195 200 205

Arg Asp Thr Glu Pro Phe Asn Val Leu Gly Asp Asp Tyr Thr Met Arg
210 215 220

Asn Val Leu Asp Leu Met Asn Lys Glu Leu Arg Gln Val Glu Ser Leu
225 230 235 240

Gln Lys Val Val Phe Gln Phe Asn Ala Phe Ala Leu Ser Thr Phe Thr
245 250 255

Lys Ser Pro Asp Asp Asn Lys Lys Ser
260 265

<210> 3
<211> 277
<212> PRT
<213> Theileria parva

<220>
<223> Amino acid sequence of Tp6

<400> 3

Met Ala Gln Ile Pro Val Asp Lys Phe Ala Lys Leu Val Thr Gly Ala
1 5 10 15

Gly Ser Ala Leu Leu Leu Phe Gly Ser Gly Ala Trp Leu Val Asn Ser
20 25 30

Ser Leu Tyr Asp Val Gly Ala Gly His Arg Ala Val Val Tyr Asn Arg
35 40 45

Ile Thr Gly Ile Ser Glu Thr Thr His Gly Glu Gly Thr His Phe Ile
50 55 60

Ile Pro Trp Leu Glu Arg Pro Ile Ile Tyr Asp Val Arg Thr Arg Pro
65 70 75 80

Arg Thr Leu Met Ser Leu Thr Gly Ser Arg Asp Leu Gln Met Val Asn
85 90 95

Ile Thr Cys Arg Val Leu Ser Arg Pro Asp Glu Arg Arg Leu Arg Asp
100 105 110

Ile Tyr Arg His Leu Gly Lys Asp Tyr Asp Glu Arg Val Leu Pro Ser
115 120 125

Ile Ile Asn Glu Val Leu Lys Ser Ile Val Ala Gln Tyr Asn Ala Ser
130 135 140

Gln Leu Ile Thr Gln Arg Glu Arg Val Ser Lys Ala Val Arg Asp Gln
145 150 155 160

Leu Val Asn Arg Ala Arg Asp Phe Asn Ile Leu Leu Asp Asp Val Ser
165 170 175

Leu Thr His Leu Ser Phe Ser Pro Glu Tyr Glu Lys Ala Val Glu Ala
180 185 190

Lys Gln Val Ala Gln Gln Gln Ala Glu Arg Ser Lys Tyr Ile Val Leu
195 200 205

Lys Ala Gln Glu Glu Lys Lys Ser Thr Ile Ile Lys Ala Gln Gly Glu
210 215 220

Ser Glu Ala Ala Arg Leu Ile Gly Ser Ala Ile Lys Asp Asn Pro Ala
225 230 235 240

Phe Ile Thr Leu Arg Arg Ile Glu Thr Ala Lys Glu Val Ala Asn Ile
245 250 255

Leu Ser Lys Ser Gln Asn Lys Ile Met Leu Asn Ser Asn Thr Leu Leu
260 265 270

Leu Ser Thr Asp Lys
275

<210> 4
<211> 9
<212> PRT
<213> Theileria parva

<220>
<223> Amino acid sequence of Tp2 Epitope 1 (Tp2.4)

<400> 4

Gln Ser Leu Val Cys Val Leu Met Lys
1 5

<210> 5
<211> 9
<212> PRT
<213> Theileria parva

<220>
<223> Amino acid sequence of Tp2 Epitope 2 (Tp2.2)

<400> 5

Phe Ala Gln Ser Leu Val Cys Val Leu
1 5

<210> 6
<211> 11
<212> PRT
<213> Theileria parva

<220>
<223> Amino acid sequence of Tp2 Epitope 3 (Tp2.3)

<400> 6

Lys Ser Ser His Gly Met Gly Lys Val Gly Lys
1 5 10

<210> 7
<211> 11
<212> PRT

<213> Theileria parva

<220>
<223> Amino acid sequence of Tp2 Epitope 4 (Tp2.4)

<400> 7

```
Ser His Glu Glu Leu Lys Lys Leu Gly Met Leu
1               5                   10
```

<210> 8
<211> 12
<212> PRT
<213> Theileria parva

<220>
<223> Amino acid sequence of peptide of Tp2

<400> 8

```
Cys Ser His Glu Glu Leu Lys Lys Leu Gly Met Leu
1               5                   10
```

<210> 9
<211> 14
<212> PRT
<213> Theileria parva

<220>
<223> Amino acid sequence of peptide #43 of Tp2

<400> 9

```
Phe Lys Ser Ser His Gly Met Gly Lys Val Gly Lys Arg Tyr
1               5                   10
```

<210> 10
<211> 14
<212> PRT
<213> Theileria parva

<220>
<223> Amino acid sequence of peptide #53/54 of Tp2

<400> 10

```
Phe Lys Ser Ser His Gly Met Gly Lys Val Gly Lys Arg Tyr
1               5                   10
```

<210> 11
<211> 14
<212> PRT
<213> Theileria parva

<220>
<223> Amino acid sequence of peptide #78/79 of Tp2

<400> 11

Phe Ala Gln Ser Leu Val Cys Val Leu Met Lys Cys Arg Gly
1 5 10

<210> 12
<211> 14
<212> PRT
<213> Theileria parva

<220>
<223> Amino acid sequence of peptide #77/78 of Tp2

<400> 12

Lys Cys Phe Ala Gln Ser Leu Val Cys Val Leu Met Lys Cys
1 5 10

<210> 13
<211> 10
<212> PRT
<213> Theileria parva

<220>
<223> Amino acid sequence of peptide of Tp2

<400> 13

Ser Ser His Gly Met Gly Lys Val Gly Lys
1 5 10

<210> 14
<211> 12
<212> PRT
<213> Theileria parva

<220>
<223> Amino acid sequence of peptide #77 of Tp2

<400> 14

Lys Cys Phe Ala Gln Ser Leu Val Cys Val Leu Met
1 5 10

<210> 15
<211> 12
<212> PRT
<213> Theileria parva

<220>
<223> Amino acid sequence of peptide #36 used as an experimental control

<400> 15

Phe Ile Ile Tyr Ile Leu His Ser Pro Val Leu Gly
1 5 10

<210> 16
<211> 9
<212> PRT
<213> Theileria parva

<220>
<223> Amino acid sequence of synthesized peptide named #75, representing amino acid residues 97 through 105 of Tp2

<400> 16

Ala Gln Ser Leu Val Cys Val Leu Met
1 5

<210> 17
<211> 9
<212> PRT
<213> Theileria parva

<220>
<223> Amino acid sequence OF PEPTIDE #76 of Tp2

<400> 17

Ser Leu Val Cys Val Leu Met Lys Cys
1 5

<210> 18
<211> 8
<212> PRT
<213> Theileria parva

<220>
<223> Amino acid sequence representing amino acid residues numbers 98 through 105 of Tp2

<400> 18

Gln Ser Leu Val Cys Val Leu Met
1 5

<210> 19
<211> 9
<212> PRT
<213> Theileria parva

<220>
<223> Amino acid sequence representing amino acid residues numbers 99 through 107 of Tp2

<400> 19

Phe Gly Lys His Gly Leu Gly Gly Ile
1 5

<210> 20
<211> 10
<212> PRT
<213> Theileria parva

<220>
<223> Amino acid sequence for peptide fragment of A.A.#49-A.A.#58 of Tp2

<400> 20

Lys Ser Ser His Gly Met Gly Lys Val Gly
1 5 10

<210> 21
<211> 8
<212> PRT
<213> Theileria parva

<220>
<223> Amino acid sequence for peptide fragment of A.A.#97-A.A.#164 of Tp2

<400> 21

Ala Gln Ser Leu Val Cys Val Leu
1 5

<210> 22
<211> 12
<212> PRT
<213> Theileria parva

<220>
<223> Amino acid sequence for peptide fragment of A.A.#97-A.A.#104 of Tp2

<400> 22

Gly Asn Cys Ser His Glu Glu Leu Lys Lys Leu Gly
1 5 10

<210> 23
<211> 12
<212> PRT
<213> Theileria parva

<220>
<223> Amino acid sequence for peptide fragment of A.A.#28-A.A.#39 of Tp2

<400> 23

His Glu Glu Leu Lys Lys Leu Gly Met Leu Glu Gly
1 5 10

<210> 24
<211> 8
<212> PRT
<213> Theileria parva

<220>
<223> Amino acid sequence for peptide fragment of Tp1 representing amino acid residues #96 to amino acid #103

<400> 24

Phe Ala Gln Ser Leu Val Cys Val
1 5

<210> 25
<211> 525
<212> DNA
<213> Theileria parva

<220>
<223> Nucleotide sequence of Tp2

<400> 25

```
atgaaattgg ccgccagatt aattagcctt tactttatta tttacatttt acattcccca 60
gtgctgggag gtaattgtag tcatgaagaa ctaaaaaaat tgggaatgct agagggcgat 120
ggtttcgaca gggatgcatt gttcaaatca tcacatggta tgggaaaggt aggaaaaagg 180
tatggtctta aaactactcc aaaagtagat aaagtcttag cagatcttga aacactgttt 240
ggaaaacacg gtcttggtgg tattagtaaa gattgtctta aatgttttgc acaaagccta 300
gtgtgcgtat taatgaaatg tagaggagca tgtctcaaag gaccatgtac tgacgactgc 360
caaaattgct ttgatagaaa ctgtaaatct gcattgctgg aatgcattgg gaaaacaagt 420
attccaaatc catgtaaatg gaaagaagat tatctaaaat acaaatttcc tgaaacagat 480
gaggacgaat ctacgaaaaa aggagaagcc tccggcactt catag                525
```

<210> 26
<211> 798
<212> DNA
<213> Theileria parva

<220>
<223> Nucleotide sequence of Tp3

<400> 26

```
atgaaattaa atactatcgc aatagccttt ttgtattcct gtttctcaca gtttttaaaa 60
aatgtgtctg ctctgaggcg tagttctcca gatttgtcac cagatggttc ttttcttcaa 120
gtaaaatcag cttctcctca ggataaacaa gatgtaatcc aaagttcctc tcctaaggta 180
acagtgccta cggttgaccc tgaaggcctc aagaaggcgg ttactgcagc agttctatca 240
aaccaaaatc aagctctaca aaacggtgct cttaatccag cagatttcac tcaagctgcc 300
tctgttaatt ccatgagtaa tgctgttagt gccatgaaca atactgttgg tccagtaaaa 360
aatcccatgg ctactgttgg tactatgaac tcctttactg gaatgcctgg tgtacaggat 420
aattttcctc agacaccgcc tgttaatgtt caagacacct ctacccagga gaacagtctt 480
gacaacctaa atctcctctt agatccctcg ttagtaaaga tatctcaagc tgatagtcac 540
ataaaagaaa gcatggaaaa agctgtacac agccttaaaa aggtcttgga ggggctaacc 600
aaccttgcga ctctgtctaa aagtagggat actgaaccgt ttaatgttct gggggatgac 660
tatacgatgc gtaacgtttt ggacctcatg aataaggaac tcaggcaggt tgaatctctt 720
cagaaagttg tgttccaatt caacgccttt gcactttcca ccttcactaa gagtccagac 780
gataataaaa aatcctaa                                                798
```

<210> 27
<211> 834
<212> DNA
<213> Theileria parva

<220>
<223> Nucleotide sequence of Tp6

<400> 27

```
atggctcaga ttcctgttga taaattcgct aaattagtta ctggagccgg ctctgctctc 60
ttattattcg gttcaggtgc ctggcttgtc aattccagtt tatacgatgt tggagctggg 120
catagagctg ttgtatataa ccgtatcact ggaataagtg agactacaca tggagaagga 180
acgcacttca taattccctg gctagaacgt ccaataattt acgatgtgag gactcgtcct 240
aggactctga tgtctctcac cggaagccgt gacttgcaga tggttaacat cacctgccgt 300
gtgttgtctc gtcccgatga gcgcagactc agggatattt acaggcactt gggcaaagat 360
tacgacgagc gagtcctgcc ttcaataata aacgaggttc tgaagagtat tgtggcccag 420
tacaacgcct ctcagctcat tactcagaga gaaagagtta gcaaagcagt cagggaccag 480
ctggtgaaca gggccaggga ctttaatatt cttctcgatg atgtctcctt aacccactta 540
agcttcagtc ctgaatatga aaaggctgta gaggctaaac aagtagctca acagcaagct 600
gaacgcagta aatatatagt gttgaaggct caggaggaga agaaatcgac gataattaag 660
gctcagggag agtctgaggc tgcaaggctt attggaagtg caattaagga taaccctgcc 720
tttattacgc ttcggagaat tgaaaccgct aaggaagtgg ctaacattct ctccaaatcg 780
cagaataaaa tcatgctcaa tagtaatact ctcttactct caactgataa ataa       834
```

<210> 28
<211> 27
<212> DNA
<213> Theileria parva

<220>
<223> Nucleotide sequence Tp2 Epitope 1 (Tp2.1)

<400> 28
caaagcctag tgtgcgtatt aatgaaa 27

<210> 29
<211> 27
<212> DNA
<213> Theileria parva

<220>
<223> Nucleotide sequence of Tp2 Epitope 2 (Tp2.2)

<400> 29
tttgcacaaa gcctagtgtg cgtatta 27

<210> 30
<211> 33
<212> DNA
<213> Theileria parva

<220>
<223> Nucleotide sequence of Tp2 Epitope 3 (Tp2.3)

<400> 30
aaatcatcac atggtatggg aaaggtagga aaa 33

<210> 31
<211> 33
<212> DNA
<213> Theileria parva

<220>
<223> Nucleotide sequence of Tp2 Epitope 4 (Tp2.4)

<400> 31
agtcatgaag aactaaaaaa attgggaatg cta 33

<210> 32
<211> 22
<212> DNA
<213> Theileria parva

<220>
<223> Nucleotide sequence Tp2 forward primer for expression cloning

<400> 32
ggtaattgta gtcatgaaga ac 22

<210> 33
<211> 24
<212> DNA
<213> Theileria parva

<220>
<223> Nucleotide sequence Tp2 reverse primer for expression cloning

<400> 33
tttactaata ccaccaagac cgtg 24

<210> 34
<211> 37
<212> DNA
<213> Theileria parva

<220>
<223> Nucleotide sequence Tp2 forward primer for gene cloning

<400> 34
gccgccacca tgaaattggc cgccagatta attagcc 37

<210> 35
<211> 39
<212> DNA
<213> Theileria parva

<220>
<223> Nucleotide sequence Tp3 forward primer for gene cloning

<400> 35
gccgccacca tgaaattaaa tactatcgca atagcctttt 39

<210> 36
<211> 37
<212> DNA
<213> Theileria parva

<220>
<223> Nucleotide sequence Tp6 forward primer for gene cloning

<400> 36
gccgccacca tggctcagat tcctgttgat aaattcg 37

<210> 37
<211> 24
<212> DNA
<213> Theileria parva

<220>
<223> Nucleotide sequence Tp2 reverse primer for gene cloning

<400> 37
ctatgaagtg ccggaggctt ctcc 24

<210> 38
<211> 28
<212> DNA
<213> Theileria parva

<220>
<223> Nucleotide sequence Tp3 reverse primer for gene cloning

<400> 38
ttaggatttt ttattatcgt ctggactc 28

<210> 39
<211> 31
<212> DNA
<213> Theileria parva

<220>
<223> Nucleotide sequence Tp6 reverse primer for gene cloning

<400> 39
ttatttatca gttgagagta agagagtatt a 31

<210> 40
<211> 26
<212> DNA

<213> Theileria parva

<220>
<223> Nucleotide sequence Tp2 internal forward primer for gene cloning

<400> 40
tccatgtaaa tggaaagaag attatc 26

<210> 41
<211> 26
<212> DNA
<213> Theileria parva

<220>
<223> Nucleotide sequence Tp2 forward primer for gene cloning

<400> 41
ggaactcagg caggttgaat ctcttc 26

<210> 42
<211> 24
<212> DNA
<213> Theileria parva

<220>
<223> Nucleotide sequence Tp2 forward primer for gene cloning

<400> 42
ccgctaagga agtggctaac attc 24

<210> 43
<211> 23
<212> DNA
<213> Theileria parva

<220>
<223> Nucleotide sequence of vector-specific forward primer

<400> 43
acgccaggat tttcccagtc acg 23

<210> 44
<211> 21
<212> DNA
<213> Theileria parva

<220>
<223> Nucleotide sequence of vector-specific reverse primer

<400> 44
gagcggataa catcacacag g 21

<210> 45
<211> 33
<212> DNA
<213> Theileria parva

<220>

<223> Nucleotide sequence of Tp3-specific forward primer

<400> 45
cgcggatccg ccaccatgaa attaaatact atc 33

<210> 46
<211> 32
<212> DNA
<213> Theileria parva

<220>
<223> Nucleotide sequence of Tp3-specific reverse primer

<400> 46
cgcctcgagg gattttttat tatcgtctgg ac 32

<210> 47
<211> 33
<212> DNA
<213> Theileria parva

<220>
<223> Nucleotide sequence of Tp6-specific forward primer

<400> 47
cgcgctagcg ccgccaccat ggctcagatt cct 33

<210> 48
<211> 32
<212> DNA
<213> Theileria parva

<220>
<223> Nucleotide sequence of Tp6-specific reverse primer

<400> 48
gcgctcgagt ttatcagttg agagtaagag ag 32

<210> 49
<211> 33
<212> DNA
<213> Theileria parva

<220>
<223> Nucleotide sequence of specific forward primer for adding a tag (HIS) to gene sequences

<400> 49
cgcctcgagt cctacatacc atctgccgaa aag 33

<210> 50
<211> 35
<212> DNA
<213> Theileria parva

<220>
<223> Nucleotide sequence of specific reverse primer for adding a tag (HIS) to gene sequences

<400> 50

cgcaagcttt cagtctagtc ctagcaaagg gttag 35

<210> 51
<211> 33
<212> DNA
<213> Theileria parva

<220>
<223> Nucleotide sequence of specific forward primer for PCR amplification of Tp3 sequences

<400> 51
cgcggatccg ccaccatgaa attaaatact atc 33

<210> 52
<211> 35
<212> DNA
<213> Theileria parva

<220>
<223> Nucleotide sequence of specific reverse primer for PCR amplification of Tp3-pTargeT sequences

<400> 52
cgcaagcttt cagtctagtc ctagcaaagg gttag 35

<210> 53
<211> 33
<212> DNA
<213> Theileria parva

<220>
<223> Nucleotide sequence of specific forward primer for PCR amplification of Tp6 sequences

<400> 53
cgcgctagcg ccgccaccat ggctcagatt cct 33